# EUROPÄISCHE PATENTSCHRIFT

(11) **EP 1 893 589 B1**
(45) Veröffentlichungstag und Bekanntmachung des Hinweises auf die Patenterteilung: **14.09.2011**
(21) Anmeldenummer: 06761974.2
(22) Anmeldetag: 06.06.2006
(51) Int. Cl.: C07D 261/20, C07D 413/12, A61K 31/423, A61P 29/00

(54) **SUBSTITUIERTE N-BENZO [D] ISOXAZOL-3-YL-AMIN-DERIVATIVE ALS INHIBITOREN VON MGLUR5, SEROTONIN-(5-HT) UND NORADRENALIN REZEPTOREN UND DEREN VERWENDUNG ZUR HERSTELLUNG VON ARZNEIMITTELN**
SUBSTITUTED N-BENZO [D] ISOXAZOL-3-YL-AMINE DERIVATIVES AS INHIBITORS OF MGLUR5, SEROTONINE (5-HT) AND NORADRENALINE RECEPTORS, AND THE USE THEREOF FOR PRODUCING MEDICAMENTS
DERIVES DE N-BENZO [D] ISOXAZOL-3-YL-AMINE SUBSTITUES UTILISES COMME INHIBITEURS DES RECEPTEURS MGLUR5, DES RECEPTEURS DE LA SEROTONINE (5-HT) ET DE LA NORADRENALINE ET LEUR UTILISATION POUR LA PRODUCTION DE MEDICAMENTS

(30) Priorität: 06.06.2005 DE 102005026194
(43) Veröffentlichungstag der Anmeldung: 05.03.2008
(73) Patentinhaber: Grünenthal GmbH, 52078 Aachen (DE)
(72) Erfinder: MERLA, Beatrix, 52078 Aachen (DE); GERLACH, Matthias, 63636 Brachttal (DE); SUNDERMANN, Corinna, 52074 Aachen (DE); JAGUSCH, Utz-Peter, 52066 Aachen (DE); HENNIES, Hagen-Heinrich, 52152 Simmerath (DE); OBERBÖRSCH, Stefan, 52074 Aachen (DE); HAURAND, Michael, 52078 Aachen (DE); JOSTOCK, Ruth, 52223 Stolberg (DE); REICH, Melanie, 52072 Aachen (DE)
(74) Vertreter: Michalski, Stefan
(86) Internationale Anmeldenummer: PCT/EP2006/005356
(87) Internationale Veröffentlichungsnummer: WO 2006/131296

(56) Entgegenhaltungen:
- EP-A- 0 048 162
- EP-A- 0 428 437
- EP-A- 0 610 134
- WO-A-02/074388
- WO-A-2005/016915
- WO-A-2005/070891
- WO-A-2005/089753
- US-B1- 6 525 196
- VAN DER STELT, C.; VOORSPUIJ, A. J. ZWART; NAUTA, W. TH.: "Study of the tuberculostatic activity and synthesis of several derivatives of p-aminosalicylic acid" ARZNEIMITTEL-FORSCHUNG, Bd. 4, 1954, Seiten 544-548, XP009071330
- BERG, S. S.; PARNELL, E. W.: "Bisquaternary salts related to quinapyramine (antrycide). Replacement of the quinoline nucleus by other heterocycles" JOURNAL OF THE CHEMICAL SOCIETY, 1961, Seiten 5275-5284, XP009071331

## Beschreibung

Die vorliegende Erfindung betrifft substituierte N-Benzo[d]isoxazol-3-yl-amin-Derivate, Verfahren zu ihrer Herstellung, Arzneimittel enthaltend diese Verbindungen und die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung. Obstipation oder Toleranzentwicklung. Des weiteren sind sie bei neuropathischen Schmerzen, unter denen insbesondere Turnorpatienten leiden, häufig nicht ausreichend wirksam.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Behandlung von Schmerzen.

Eine weitere Aufgabe der vorliegenden Erfindung bestand darin, neue Verbindungen zur Verfügung zu stellen, die sich als pharmakologische Wirkstoffe in Arzneimitteln zur Behandlung von Störungen oder Krankheiten eignen, die zumindest teilweise durch mGluR5-Rezeptoren (mGluR5 = metabotroper Glutamatrezeptor 5) und/oder Serotonin-(5-HT)-Rezeptoren und/oder Noradrenalin-Rezeptoren vermittelt werden.

Überraschenderweise wurde nun gefunden, dass substituierte N-Benzo[d]isoxazol-3-yl-amin-Derivate der nachstehend angegebenen allgemeinen Formeln I und la eine ausgezeichnete Affinität zum mGluR5-Rezeptor, zum Serotonin-(5-HT)-Rezeptor und zum Noradrenalin-Rezeptor aufweisen und sich daher insbesondere zur Prophylaxe und/oder Behandlung von Störungen oder Krankheiten eignen, die zumindest teilweise durch mGluR5-Rezeptoren (mGluR5 = metabotroper Glutamatrezeptor 5) und/oder Serotonin-(5-HT)-Rezeptoren und/oder Noradrenalin-Rezeptoren vermittelt werden.

Ein Gegenstand der vorliegenden Erfindung ist daher ein Arzneimittel enthaltend wenigstens ein substituiertes N-Benzo[d]isoxazol-3-yl-amin-Derivat der allgemeinen Formel I, 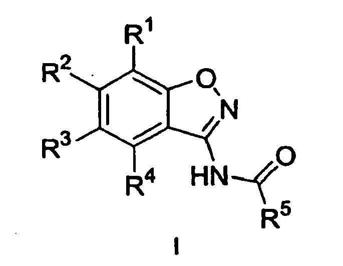 nämlich ein Arzneimittel enthaltend wenigstens eine Verbindung ausgewählt aus der Gruppe bestehend aus
[1] Thiophen-2-carbonsäure-benzo[d]isoxazol-3-ylamid,
[3] Naphthalen-2-carbonsäure-benzo[d]isoxazol-3-ylamid,
[4] Adamantan-2-carbonsäurebenzo[d]isoxazol-3-ylamid,
[5] Cyclohexancarbonsäure-benzo[d]isoxazol-3-ylamid,
[6] N-Benzo[d]isoxazol-3-yl-2,2-dimethyl-propionamid,
[7] N-(4-Methoxy-benzo[d]isoxazol-3-yl)-2,2-diphenyl-acetamid,
[8] Cyclopropancarbonsäure-(5-bromo-benzo[d]isoxazol-3-yl)-amid,
[9] 3,5-Dichloro-N-(6-fluoro-benzo[d]isoxazol-3-yl)-benzamid,
[10] N-(4-Amino-benzo[d]isoxazol-3-yl)-4-nitro-benzamid,
[11] Naphthalin-1-carbonsäure-(4-amino-benzo[d]isoxazol-3-yl)-amid,
[12] Benzo[b]thiophen-2-carbonsäure-(4-fluoro-benzo[d]isoxazol-3-yl)-amid,
[13] N-Benzo[d]isoxazol-3-yl-2-trifluoromethyl-benzamid,
[14] N-Benzo[d]isoxazol-3-yl-3,4-dichloro-benzamid,
[15] N-Benzo[d]isoxazol-3-yl-2,3-difluoro-benzamid,
[16] N-(4-Dimethylamino-benzo[d]isoxazol-3-yl)-3-fluoro-benzamid,
[17] Thiophen-2-carbonsäure-(6-fluoro-benzo[d]isoxazol-3-yl)-amid,
[18] N-(6-Fluoro-benzo[d]isoxazol-3-yl)-2-methyl-butyramid,
[19] N-(6-Chloro-benzo[d]isoxazol-3-yl)2-fluoro-3-trifluoromethyl-benzamid,
[20] N-(6-Chloro-benzo[d]isoxazol-3-yl)-3-methoxy-benzamid, _
[21] N-(6-Bromo-benzo[d]isoxazol-3-yl)-4-tert-butyl-benzamid,
[22] N-(6-Bromo-benzo[d]isoxazol-3-yl)-2-methoxy-benzamid,
[23] N-(6-Bromo-benzo[d]isoxazol-3-yl)-2-trifluoromethyl-benzamid,
[24] Adamantan-2-carbonsäure-(6-bromo-benzo[d]isoxazol-3-yl)amid,
[25] N-(6-Bromo-benzo[d]isoxazol-3-yl)-2-methyl-butyramid,
[26] N-(5-Fluoro-benzo[d]isoxazol-3-yl)-2-trifluommethyl-benzamid,
[27] 3,4-Dichloro-N-(5-fluoro-benzo[d]isoxazol-3-yl)-benzamid,
[28] N-(5-Fluoro-benzo[d]isoxazol-3-yl)-2-methyl-benzamid,
[29] N-(5-Bromo-benzo[d]isoxazol-3-yl)-2-fluoro-5-trifluoromethyl-benzamid,
[30] N-(5-Bromo-benzo[d]isoxazol-3-yl)-2,4-difluoro-benzamid,
[31] N-(5-Methyl-benzo[d]isoxazol-3-yl)-2-trifluoromethyl-benzamid,
[32] 3-Fluoro-N-(5-methyl-benzo[d]isoxazol-3-yl)-benzamid,
[33] N-(4-Methoxy-benzo[d]isoxazol-3-yl)-3,5-bis-trifluoromethyl-benzamid,
[34] 3,5-Difluoro-N-(4-methoxy-benzo[d]isoxazol-3-yl)benzamid,
[35] N-(4-Dimethylamino-benzo[d]isoxazol-3-yl)-3,5-bis-trifluoromethyl-benzamid,
[36] 2-Bromo-N-[4-(2,2,2-trifluoro-ethoxy)-benzo[d]isoxazol-3-yl]-benzamid,
[37] 3-Chloro-N-[4-(2,2,2-trifluoro-ethoxy)-benzo[d]isoxazol-3-yl]-benzamid,
[38] 4-tert-Butyl-N-[4-(2,2,2-trifluoro-ethoxy)-benzo[d]isoxazol-3-yl]-benzamid,
[39] 2-Methoxy-N-[4-(2',2,2-trifluoromethoxy)-benzo[d]isoxazol-3-yl]-benzamid,
[40] N-(6-Chloro-benzo[d]isoxazol-3-yl)-4-iodo-benzamid,
[41] Naphthalen-2-carbonsäure-(6-chloro-benzo[d]isoxazol-3-yl)-amid,
[42] N-(6-Chloro-benzo[d]isoxazol-3-yl)-3,4-difluoro-benzamid,
[43] N-(6-Bromo-benzo[d]isoxazol-3-yl)-4-fluoro-2-trifluoromethyl-benzamid,
[44] 2,4-Dichloro-N-(6-bromo-benzo[d]isoxazol-3-yl)-5-fluoro-benzamid,
[45] N-(6-Bromo-benzo[d]isoxazol-3-yl)-3-fluoro-4-trifluoromethyl-benzamid,
[46] N-(6-Bromo-benzo[d]isoxazol-3-yl)-3-fluoro-5-trifluoromethyl-benzamid,
[47] N-(6-Bromo-benzo[d]isoxazol-3-yl)-2,3,4-trifluoro-benzamid,
[48] N-(6-Bromo-benzo[d]isoxazol-3-yl)-4-propyl-benzamid,
[49] N-(6-Bromo-benzo[d]isoxazol-3-yl)-3,4-difluoro-benzamid,
[50] Furan-2-carbonsäure-(5-bromo-benzo[d]isoxazol-3-yl)-amid,
[51] 4-Fluoro-N-(7-fluoro-benzo[d]isoxazol-3-yl)-2-trifluoromethyl-benzamid,
[52] 2,4-Dichloro-5-fluoro-N-(7-fluoro-benzo[d]isoxazol-3-yl)-benzamid,
[53] 3-Fluoro-N-(7-fluoro-benzo[d]isoxazol-3-yl)-4-trifluoromethyl-benzamid,
[54] 2,3,4-Trifluoro-N-(7-fluoro-benzo[d]isoxazol-3-yl)benzamid,
[55] N-(7-Fluoro-benzo[d]isoxazol-3-yl)-4-iodo-benzamid,
[56] 2-Chloro-N-(7-fluoro-benzo[d]isoxazol-3-yl)-nicotinamid,
[57] Naphthalen-2-carbonsäure-(7-fluoro-benzo[d]isoxazol-3-yl)-amid,
[58] N-(7-Fluoro-benzo[d]isoxazol-3-yl)4-propyl-benzamid,
[59] 3,4-Difluoro-N-(7-fluoro-benzo[d]isoxazol-3-yl)benzamid,
[60] 2-Fluoro-N-(7-fluoro-benzo[d]isoxazol-3-yl)-3-trifluoromethyl-benzamid,
[61] N-(7-Fluoro-benzo[d]isoxazol-3-yl)-3-methoxy-benzamid,
[62] N-(7-Fluoro-benzo[d]isoxazol-3-yl)-2,2-diphenyl-acetamid,
[63] Furan-2-carbonsäure-(7-fluoro-benzo[d]isoxazol-3-yl)-amid,
[64] 2,4-Dichloro-N-(4-dimethylamino-benzo[d]isoxazol-3-yl)-5-fluoro-benzamid,
[65] N-(4-Dimethylamino-benzo[d]isoxazol-3-yl)2,3,4-trifluoro-benzamid,
[66] 2-Chloro-N-(4-dimethylamino-benzo[d]isoxazol-3-yl)-nicotinamid,
[67] Naphthalen-2-carbonsäure-(4-dimethylamino-benzo[d]isoxazol-3-yl)-amid,
[68] N-(4-Dimethylamino-benzo[d]isoxazol-3-yl)-4-propyl-benzamid,
[69] N-(4-Dimethylamino-benzo[d]isoxazol-3-yl)-3,4-difluoro-benzamid,
[70] N-(4-Dimethylamino-benzo[d]isoxazol-3-yl)-2-fluoro-3-trifluoromethyl-benzamid,
[71] N-(4-Dimethylamino-benzo[d]isoxazol-3-yl)-3-methoxy-benzamid,
[72] N-(4-Dimethylamino-benzo[d]isoxazol-3-yl)-2,2-dimethyl-propionamid,
[73] Cyclohexancarbonsäure-(4-dimethylamino-benzo[d]isoxazol-3-yl)amid,
[74] N-(4-Dimethylamino-benzo[d]isoxazol-3-yl)2,2-diphenyl-acetamid,
[75] Furan-2-carbonsäure-(4-dimethylamino-benzo[d]isoxazol-3-yl)-amid, .
[76] N-(4-Dimethylamino-benzo[d]isoxazol-3-yl)-2,2,3,3,4,4,4-heptafluoro-butyramid,
[77] 4-Fluoro-N-[4-(2,2,2-trifluoro-ethoxy)benzo[d]isoxazol-3-yl]-2-trifluoromethyl-benzamid,
[78] 2,4-Dichloro-5-fluoro-N-[4-(2,2,2-trifluoro-ethoxy)-benzo[d]isoxazol-3-yl]-benzamid,
[79] 3-Fluoro-N-[4-(2,2,2-trifluoro-ethoxy)-benzo[d]isoxazol-3-yl]-4-trifluoromethyl-benzamid,
[80] 2,3,4-Trifluoro-N-[4-(2,2,2-trifluoro-ethoxy)-benzo[d]isoxazol-3-yl]-benzamid,
[81] Naphthalen-2-carbonsäure-[4-(2,2,2-trifluoro-ethoxy)-benzo[d]isoxazol-3-yl]-amid,
[82] 3,4-Difluoro-N-[4-(2,2,2-trifluoro-ethoxy)-benzo[d]isoxazol-3-yl]-benzamid,
[83] 2-Fluoro-N-[4-(2,2,2-trifluoro-ethoxy)-benzo[d]isoxazol-3-yl]-3-trifluoromethyl-benzamid,
[85] 2-Ethyl-hexansäure-benzo[d]isoxazol-3-ylamid,
[86] N-Benzo[d]isoxazol-3-yl-2-methyl-butyramid,
[87] N-Benzo[d]isoxazol-3-yl-2,6-difluoro-benzamid,
[88] N-Benzo[d]isoxazol-3-yl-3-fluoro-4-täfluoromethyl-benzamid,
[89] N-Benzo[d]isoxazol-3-yl-2,3,6-trifluoro-benzamid,
[90] N-Benzo[d]isoxazol-3-yl-nicotinamid,
[91] 5-Methyl-isoxazol-3-carbonsäure-benzo[d]isoxazol-3-ylamid,
[92] Benzo[b]thiophen-3-carbonsäure-benzo[d]isoxazol-3-ylamid,
[93] 2-Chloro-N-(7-fluoro-benzo[d]isoxazol-3-yl)-benzamid,
[94] 4-tert-Butyl-N-(7-fluoro-benzo[d]isoxazol-3-yl)-benzamid,
[95] N-(7-Fluoro-benzo[d]isoxazol-3-yl)-benzamid,
[96] 4-Fluoro-N-(7-fluoro-benzo[d]isoxazol-3-yl)-benzamid,
[97] 2-Fluoro-N-(7-fluoro-benzo[d]isoxazol-3-yl)-benzamid,
[98] N-(7-Fluoro-benzo[d]isoxazol-3-yl)-2-methoxy-benzamid,
[99] N-(7-Fluoro-benzo[d]isoxazol-3-yl)-2-methyl-benzamid,
[100] 3,5-Difluoro-N-(6-fluoro-benzo[d]isoxazol-3-yl)-benzamid,
[101] Naphthalen-1-carbonsäure-(6-fluoro-benzo[d]isoxazol-3-yl)-amid,
[102] Adamantan-1-carbonsäure-(6-fluoro-benzo[d]isoxazol-3-yl)-amid,
[103] 2-Bromo-N-(5-fluoro-benzo[d]isoxazol-3-yl)-benzamid,
[104] 4-tert-Butyl-N-(5-fluoro-benzo[d]isoxazol-3-yl)-benzamid,
[105] 2,4-Difluoro-N-(5-fluoro-benzo[d]isoxazol-3-yl)-benzamid,
[106] Naphthalen-2-carbonsäure-(5-fluoro-benzo[d]isoxazol-3-yl)-amid,
[107] N-(5-Fluoro-benzo[d]isoxazol-3-yl)-4-propyl-benzamid,
[108] 2-Chloro-N-(6-chloro-benzo[d]isoxazol-3-yl)-benzamid,
[109] 4-tert-Butyl-N-(6-chloro-benzo[d]isoxazol-3-yl)-benzamid,
[110] N-(6-Chloro-benzo[d]isoxazol-3-yl)-4-fluoro-2-trifluoromethyl-benzamid,
[111] 2,4-Dichloro-N-(6-chloro-benzo[d]isoxazol-3-yl)-5-fluoro-benzamid,
[112] 2-Ethyl-hexansäure-(6-chloro-benzo[d]isoxazol-3-yl)-amid,
[113] N-(6-Chloro-benzo[d]isoxazol-3-yl)-2-methyl-butyramid,
[114] N-(6-Bromo-benzo[d]isoxazol-3-yl)-2-chloro-benzamid,
[115] N-(6-Bromo-benzo[d]isoxazol-3-yl)-3,4-dichloro-benzamid,
[116] Thiophen-2-carbonsäure-(6-bromo-benzo[d]isoxazol-3-yl)-amid,
[117] N-(6-Bromo-benzo[d]isoxazol-3-yl)-3,3-dimethyl-butyramid,
[118] N-(5-Methyl-benzo[d]isoxazol-3-yl)benzamid,
[119] 4-Bromo-N-(5-methyl-benzo[d]isoxazol-3-yl)-benzamid,
[120] 2-Chloro-N-(5-methyl-benzo[d]isoxazol-3-yl)-benzamid,
[121] 4-Fluoro-N-(5-methyl-benzo[d]isoxazol-3-yl)benzamid,
[122] 2-Fluoro-N-(5-methyl-benzo[d]isoxazol-3-yl)-benzamid,
[123] 3-Methyl-N-(5-methyl-benzo[d]isoxazol-3-yl)-benzamid,
[124] 4-tert-Butyl-N-(5-methyl-benzo[d]isoxazol-3-yl)-benzamid,
[125] 2-Methyl-N-(5-methyl-benzo[d]isoxazol-3-yl)-benzamid,
[126] 2,3-Difluoro-N-(5-methyl-benzo[d]isoxazol-3-yl)-benzamid,
[127] 2,4-Difluoro-N-(5-methyl-benzo[d]isoxazol-3-yl)-benzamid,
[128] 2-Chloro-N-(5-methyl-benzo[d]isoxazol-3-yl)nicotinamid,
[129] Cyclopropancarbonsäure-(5-fluoro-benzo[d]isoxazol-3-yl)-amid,
[130] N-(5-Fluoro-benzo[d]isoxazol-3-yl)-3,3-dimethyl-butyramid,
[131] 2,4-Difluoro-N-(5-fluoro-benzo[d]isoxazol-3-yl)-benzamid,
[132] 2,5-Dimethyl-furan-3-carbonsäure-(5-fluoro-benzo[d]isoxazol-3-yl)-amid,
[133] N-(5-Bromo-benzo[d]isoxazol-3-yl)-2,3-difluoro-benzamid,
[134] 2,5-Dimethyl-furan-3-carbonsäure-(5-bromo-benzo[d]isoxazol-3-yl)-amid,
[135] N-(5-Bromo-benzo[d]isoxazol-3-yl)-2-methyl-butyramid,
[136] N-(5-Bromo-benzo[d]isoxazol-3-yl)-2.6-difiuoro-benzamid,
[137] N-(5-Bromo-benzo[d]isoxazol-3-yl)-3,4-dimethoxy-benzamid,
[138] N-(5-Bromo-benzo[d]isoxazol-3-yl)-4-methyl-benzamid,
[139] N-(5-Bromo-benzo[d]isoxazol-3-yl)-2,6-dimethoxy-benzamid,
[140] 2-Bromo-N-(5-bromo-benzo[d]isoxazol-3-yl)-benzamid,
[141] N(-5-Bromo-benzo[d]isoxazol-3-yl)-5-fluoro-2-trifluommethyl-benzamid,
[142] N-(5-Bromo-benzo[d]isoxazol-3-yl)-3-methoxy-benzamid,
[143] 3-Methyl-N-(5-methyl-benzo[d]isoxazol-3-yl)-benzamid,
[144] 4-Cyano-N-(5-methyl-benzo[d]isoxazol-3-yl)-benzamid,
[145] N-(5-Methyl-benzo[d]isoxazol-3-yl)-2-phenyl-butyramid,
[146] 2-Methyl-pentansäure-(5-methyl-benzo[d]isoxazol-3-yl)-amid,
[147] N-(4-Fluoro-benzo[d]isoxazol-3-yl)benzamid,
[148] 4-Chloro-N-(4-fluoro-benzo[d]isoxazol-3-yl)-benzamid,
[149] 2-Fluoro-N-(4-fluoro-benzo[d]isoxazol-3-yl)-benzamid,
[150] Adamantan-1-carbonsäure-(4-fluoro-benzo[d]isoxazol-3-yl)-amid,
[151] Adamantan-1-carbonsäure-(4-chloro-benzo[d]isoxazol-3-yl)-amid,
[152] N-(4-Chloro-benzo[d]isoxazol-3-yl)-benzamid,
[153] N-(4-Chloro-benzo[d]isoxazol-3-yl)-3-methyl-benzamid,
[154] N-(4-Chloro-benzo[d]isoxazol-3-yl)-2-methyl-butyramid,
[155] N-(4-Chloro-benzo[d]isoxazol-3-yl)-3,3-dimethyl-butyramid,
[156] N-(4-Methoxy-benzo[d]isoxazol-3-yl)-2-methyl-benzamid,
[157] 2-Chloro-N-(4-methoxy-benzo[d]isoxazol-3-yl)-benzamid,
[158] N-(4-Methoxy-benzo[d]isoxazol-3-yl)-2-trifluoromethyl-benzamid,
[159] 2-Ethyl-hexansäure-(4-methoxy-benzo[d]isoxazol-3-yl)-amid,
[160] N-(4-Methoxy-benzo[d]isoxazol-3-yl)-2-methyl-butyramid,
[161] 2-Methyl-pentansäure-(4-methoxy-benzo[d]isoxazol-3-yl)-amid,
[162] Cyclopropancarbonsäure-[4-(2,2,2-trifluoro-ethoxy)-benzo[d]isoxazol-3-yl]-amid,
[163] 2-Methyl-N-[4-(2,2,2-trifluoro-ethoxy)-benzo[d]isoxazol-3-yl]-butyramid,
[164] 3,3-Dimethyl-N-[4-(2,2,2-trifluoro-ethoxy)-benzo[d]isoxazol-3-yl]-butyramid.
[165] Cyclohexancarbonsäure-[4-(2,2,2-trifluoro-ethoxy)-benzo[d]isoxazol-3-yl]-amid,
[166] 2,5-Dimethyl-furan-3-carbonsäure-[4-(2,2,2-trifluoro-ethoxy)-benzo[d]isoxazol-3-yl]-amid,
[167] N-[4-(4-Methyl-benzyloxy)-benzo[d]isoxazol-3-yl]-3-nitro-benzamid,
[168] N-[4-(4-Methyl-benzyloxy)-benzo[d]isoxazol-3-yl]-4-propyl-benzamid,
[169] N-(4-Dimethylamino-benzo[d]isoxazol-3-yl)-3-fluoro-5-trifluoromethyl-benzamid,
[170] 3,4-Dichloro-N-(4-dimethylamino-benzo[d]isoxazol-3-yl)-benzamid,
[171] N-(4-Dimethylamino-benzo[d]isoxazol-3-yl)-3-nitro-benzamid,
[172] Adamantan-1-carbonsäureg4-dimethylamino-benzo[d]isoxazol-3-ylfamid,
[173] Benzo[b]thiophen-2-carbonsäure-(4-dimethylamino-benzo[d]isoxazol-3-yl)-amid,
[174] N-(4-Dimethylamino-benzo[d]isoxazol-3-yl)-3,3-dimethyl-butyramid,
[176] N-(6-Amino-4,5,7-trifluoro-benzo[d]isoxazol-3-yl)-2,6-difluoro-benzamid
   und
[177] N-(6-Amino-4,5,7-trifluoro-benzo[d]isoxazol-3-yl)-2,4-difluoro-benzamid,
jeweils ggf. in Form eines seiner reinen Stereoisomeren, insbesondere Enantiomeren oder Diastereomeren oder Rotameren, seiner Racemate oder in Form einer Mischung von Stereoisomeren , insbesondere der Enantiomeren und/oder Diastereomeren und/oder Rotameren, in einem beliebigen Mischungsverhältnis, oder jeweils in Form entsprechender Salze, oder jeweils in Form entsprechender Solvate;
sowie ggf. einen oder mehrere physiologisch verträgliche Hilfsstoffe.

Das erfindungsgemäße Arzneimittel eignet sich insbesondere zur mGIuRS-Rezeptor-Regulation, vorzugsweise zur Inhibierung des mGluR5-Rezeptors, und/oder zur Noradrenalin-Rezeptor-Regulation, vorzugsweise zur Noradrenalin-Wiederaufnahme-Hemmung, und/oder zur Serotonin-(5-HT)-Rezeptor-Regulation, vorzugsweise zur Serotonin-Wiederaufnahme-Hemmung, und somit auch zur Prophylaxe und/oder Behandlung von Störungen und Krankheiten, die zumindest teilweise durch mGluR5-Rezeptoren und/oder Noradrenalin-Rezeptoren und/oder Serotonin-Rezeptoren vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; chronische paroxysmale Hemikranie; Depressionen; . Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Narkolepsie; Diarrhoe; Gastritis; Magengeschwür, Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit;
zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Regulation des kardiovaskulären Systems; zur Lokalanästhesie; zur Vigilanzsteigerung; zur Libidosteigerung; zur Diurese und/oder zur Antinatriurese.

Besonders bevorzugt eignet sich das erfindungsgemäße Arzneimittel zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Angstzuständen; Panikattacken; Alkohol- und/oder Drogen-, vorzugsweise Nikotin- oder Kokain-, und/oder Medikamentenmißbrauch: Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin-oder Kokain-, und/oder Medikamentenabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz.

Ein weiterer Gegenstand der vorliegenden Erfindung ist die Verwendung wenigstens eines erfindungsgemäßen substituierten N-Benzo[d]isoxazol-3-yl-amin-Derivats der vorstehend angegebenen allgemeinen Formel I, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur mGluR5-Rezeptor-Regulation, vorzugsweise zur Inhibierung des mGluR5-Rezeptors, und/oder zur Noradrenalin-Rezeptor-Regulation, vorzugsweise zur Noradrenalin-Wiederaufnahme-Hemmung, und/oder zur Serotonin-(5-HT)-Rezeptor-Regulation, vorzugsweise zur Serotonin-Wiederaufnahme-Hemmung.

Bevorzugt ist die Verwendung wenigstens eines erfindungsgemäßen substituierten N-Benzo[d]lsoxazol-3-yl-amin-Derivats der vorstehend angegebenen allgemeinen Formel 1, jeweils ggf. in Form eines seiner reinen Stereoisomeren, insbesondere Enantiomeren oder Diastereomeren oder Rotameren, seiner Racemate oder In Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechende Solvates, sowie ggf. eines oder mehrerer pharmazeutisch verträglicher Hilfsstoffe zur Herstellung eines Arzneimittels zur Prophylaxe und/oder Behandlung von Störungen und Krankheiten, die zumindest teilweise durch mGluR5-Rezeptoren und/oder Noradrenalin-Rezeptoren und/oder Serotonin-Rezeptoren vermittelt werden.

Besonders bevorzugt ist die Verwendung wenigstens eines erfindungsgemäßen substituierten N-Benzo[d]isoxazol-3-yl-amin-Derivats der vorstehend angegebenen allgemeinen Formel I, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; chronische paroxysmale Hemlkranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstönrngen; kognitiven Mangelzuständen (attention deficit syndrom, ADS); Epilepsie; Narkolepsie; Diarrhoe; Gastritis; Magengeschwür, Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin-oder Kokain-, und/oder Medikamentenabhängigkeit;
zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Regulation des kardiovaskulären Systems; zur Lokalanästhesie; zur Vigilanzsteigerung; zur Libidosteigerung; zur Diurese und/oder zur Antinatriurese.

Ganz besonders bevorzugt ist die Verwendung wenigstens eines erfindungsgemäßen substituierten N-Benzo[d]isoxazol-3-yl-amin-Derivats der vorstehend angegebenen allgemeinen Formel I, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Angstzuständen; Panikattacken; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol-und/oder Drogen-, vorzugsweise Nikotin- oder Kokain-, und/oder Medikamentenabhängigkeit.

Noch weiter bevorzugt ist die Verwendung wenigstens eines erfindungsgemäßen substituierten N-Benzo[d]isoxazol-3-yl-amin-Derivats der vorstehend angegebenen allgemeinen Formel 1, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in. Form von Pellets oder Granulaten, ggf. zu Tabletten verpreßt, in Kapseln abgefüllt oder in einer Flüssigkeit suspendiert, vorliegen und als solche auch verabreicht werden. Neben wenigstens einem substituierten N-Benzo[d]isoxazol-3-yl-amin-Derivat der vorstehend angegebenen allgemeinen Formel 1, ggf. in Form eines seiner reinen Stereoisomeren, insbesondere Enantiomeren oder Diastereomeren oder Rotameren, seines Racemates oder in Form von Mischungen der Stereoisomeren, insbesondere der Enantiomeren oder Diastereomeren oder Rotameren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen. Sprengmitteln, Gleibnitteln, Schmiermitteln, Aromen und Bindemitteln:

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen substituierten N-Benzo[d]isoxazol-3-yl-amin-Derivate der vorstehend angegebenen allgemeinen Formel I können in einem Depot, in gelöster Form oder in einem Pflaster, gegebenenfalls unter Zusatz von die Hautpenetration fördernden Mitteln, als geeignete perkutane Applikationszubereitungen vorliegen. Oral oder perkutan anwendbare Zubereitungsformen können die jeweilige erfindungsgemäßen substituierten N-Benzo[d]isoxazol-3-yl-amin-Derivate der vorstehend angegebenen allgemeinen Formel 1 auch verzögert freisetzen. Prinzipiell können dem erfindungsgemäßen Arzneimittel andere dem Fachmann bekannte weitere Wirkstoffe zugesetzt werden.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton. Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten N-Benzo[d]isoxazol-3-yl-amin-Derivate der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

Ein weiterer Gegenstand der vorliegenden Anmeldung sind substituierte N-Benzo[d]isoxazol-3-yl-amin-Derivate der allgemeinen Formel Ia, 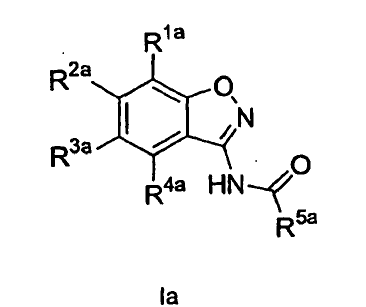 nämlich substituierte N-Benzo[d]isoxazol-3-yl-amin-Derivate der vorstehend angegebenen allgemeinen. Formel Ia ausgewählt aus der Gruppe bestehend aus
[1] Thiophen-2-carbonsäure-benzo[d]isoxazol-3-ylamid,
[3] Naphthalen-2-carbonsäure-benzo[d]isoxazol-3-ylamid ,
[4] Adamantan-2-carbonsäurebenzo[d]isoxazol-3-ylamid,
[5] Cyclohexancarbonsäure-benzo[d]isoxazol-3-ylamid,
[6] N-Benzo[d]isoxazol-3-yl-2,2-dimethyl-propionamid,
[7] N-(4-Methoxy-benzo[d]isoxazol-3-yl)-2,2-diphenyl-acetamid,
[8] Cyclopropancarbonsäure-(5-bromo-benzo[d]isoxazol-3-yl)-amid,
[9] 3,5-Dichloro-N-(6-fluoro-benzo[d]isoxazol-3-yl)-benzamid,
[10] N-(4-Amino-benzo[d]isoxazol-3-yl)-4-nitro-benzamid,
[11] Naphthalin-1-carbonsäure-(4-amino-benzo[d]isoxazol-3-yl)-amid,
[12] Benzo[b]thiophen-2-carbonsäure-(4-fluoro-benzo[d]isoxazol-3-yl)-amid,
[13] N-Benzo[d]isoxazol-3-yl-2-trifluoromethyl-benzamid,
[14] N-Benzo[d]isoxazol-3-yl-3,4-dichloro-benzamid,
[15] N-Benzo[d]isoxazol-3-yl-2,3-difluoro-benzamid,
[16] N-(4-Dimethylamino-benzo[d]isoxazol-3-yl)-3-fluoro-benzamid,
[17] Thiophen-2-carbonsäure-(6-fluoro-benzo[d]isoxazol-3-yl)-amid,
[18] N-(6-Fluoro-benzo[d]isoxazol-3-yl)-2-methyl-butyramid,
[19] N-(6-Chloro-benzo[d]isoxazol-3-yl)-2-fluoro-3-trifluoromethyl-benzamid,
[20] N-(6-Chloro-benzo[d]isoxazol-3-yl)-3-methoxy-benzamid,
[21] N-(6-Bromo-benzo[d]isoxazol-3-yl)-4-tert-butyl-benzamid,
[23] N-(6-Bromo-benzo[d]isoxazol-3-yl)-2-trifluoromethyl-benzamid,
[24] Adamantan-2-carbonsäure-(6-bromo-benzo[d]isoxazol-3-yl)-amid,
[25] N-(6-Bromo-benzo[d]isoxazol-3-yl)-2-methyl-butyramid,
[26] N-(5-Fluoro-benzo[d]isoxazol-3-yl)-2-trifluoromethyl-benzamid,
[27] 3,4-Dichloro-N-(5-fluoro-benzo[d]isoxazol-3-yl)-benzamid,
[29] N-(5-Bromo-benzo[d]isoxazol-3-yl)-2-fluoro-5-trifluoromethyl-benzamid,
[30] N-(5-Bromo-benzo[d]isoxazol-3-yl)-2,4-difluoro-benzamid,
[31] N-(5-Methyl-benzo[d]isoxazol-3-yl)-2-trifluoromethyl-benzamid,
[32] 3-Fluoro-N-(5-methyl-benzo[d]isoxazol-3-yl)-benzamid,
[33] N-(4-Methoxy-benzo[d]isoxazol-3-yl)-3,5-bis-trifluoromethyl-benzamid,
[34] 3,5-Difluoro-N-(4-'methoxy-benzo[d]isoxazol-3-yl)-benzamid,
[35] N-(4-Dimethylamino-benzo[d]isoxazol-3-yl)-3,5-bis-trifluoromethyl-benzamid,
[36] 2-Bromo-N-[4-(2,2,2-trifluoro-ethoxy)-benzo[d]isoxazol-3-yl]-benzamid,
[37] 3-Chloro-N-[4-(2,2,2-trifluoro-ethoxy)-benzo[d]isoxazol-3-yl]-benzamid,
[38] 4-tert-Butyl-N-[4-(2,2,2-trifluoro-ethoxy)-benzo[d]isoxazol-3-yl]-benzamid,
[39] 2-Methoxy-N-[4-(2,2,2-trifluoro-ethoxy)-benzo[d]isoxazol-3-yl]-benzamid,
[40] N-(6-Chloro-benzo[d]isoxazol-3-yl)-4-iodo-benzamid,
[41] Naphthalen-2-carbonsäure-(6-chloro-benzo[d]isoxazol-3-yl)amid,
[42] N-(6-Chloro-benzo[d]isoxazol-3-yl)-3,4-difluoro-benzamid,
[43] N-(6-Bromo-benzo[d]isoxazol-3-yl)-4-fluoro-2-trifluoromethyl-benzamid,
[44] 2,4-Dichloro-N-(6-bromo-benzo[d]isoxazol-3-yl)-5-fluoro-benzamid,
[45] N-(6-Bromo-benzo[d]isoxazol-3-yl)-3-fluoro-4-trifluommethyl-benzamid,
[46] N-(6-Bromo-benzo[d]isoxazol-3-yl)-3-fluoro-5-trifluoromethyl-benzamid,
[47] N-(6-Bromo-benzo[d]isoxazol-3-yl)-2,3,4-trifluoro-benzamid,
[48] N-(6-Bromo-benzo[d]isoxazol-3-yl)-4-propyl-benzamid,
[49] N-(6-Bromo-benzo[d]isoxazol-3-yl)-3,4-difluoro-benzamid,
[50] Furan-2-carbonsäure-(5-bromo-benzo[d]isoxazol-3-yl)-amid,
[51] 4-Fluoro-N-(7-fluoro-benzo[d]isoxazol-3-yl)-2-trifluoromethyl-benzamid,
[52] 2,4-Dichloro-5-fluoro-N-(7-fluoro-benzo[d]isoxazol-3-yl)-benzamid,
[53] 3-Fluoro-N-(7-fluoro-benzo[d]isoxazol-3-yl)-4-trifluoromethyt-benzamid,
[54] 2,3,4-Trifluoro-N-(7-fluoro-benzo[d]isoxazol-3-yl)-benzamid,
[55] N(7-Fluoro-benzo[d]isoxazol-3-yl)-4-iodo-benzamid
[56] 2-Chloro-N-(7-fluoro-benzo[d]isoxazol-3-yl)-nicotinamid,
[57] Naphthalen-2-carbonsäure-(7-fluoro-benzo[d]isoxazol-3-yl)-amid,
[58] N-(4-Fluoro-benzo[d]isoxazol-3-yl)-74-propyl-benzamid,
[59] 3,4-Difluoro-N(7-fluoro-benzo[d]isoxazol-3-yl)-bemzamid,
[60] 2-Fluoro-N-(7-fluoro-benzo[d]isoxazol-3-yl)-3-trifluoromethyl-benzamid,
[61] N-(7-Fluoro-benzo[d]isoxazol-3-yl)-3-methoxy-benzamid,
[62] N-(7-Fluoro-benzo[d]isoxazol-3-yl)-2,2-diphenyl-acetamid,
[63] Furan-2-carbonsäure-(7-fluoro-benzo[d]isoxazol-3-yl)amid, -benzamid,
[64] 2,4-Dichloro-N-(4-dimethylamino-benzo[d]isoxazol-3-yl)-5-fluoro-benzamid,
[65] N-(4-Dimethylamino-benzo[d]isoxazol-3-yl)-2,3,4-trifluoro-benzamid.
[66] 2-Chloro-N-(4-dimethylamino-benzo[d]isoxazol-3-yl)-nicotinamid,
[67] Naphthalen-2-carbonsäure-(4-dimethylamino-benzo[d]isoxazol-3-yl)-amid,
[68] N-(4-Dimethylamino-benzo[d]isoxazol-3-yl)-4-propyl-benzamld,
[69] N-(4-Dimethylamino-benzo[d]isoxazol-3-yl)-3,4-difluoro-benzamid,
[70] N-(4-Dimethylamino-benzo[d]isoxazol-3-yl)-2-fluoro-3-trifluoromethyl-benzamid,
[71] N-(4-Dimethylamino-benzo[d]isoxazol-3-yl)-3-methoxy-benzamid,
[72] N-(4-Dimethylamino-benzo[d]isoxazol-3-yl)-2,2-dimethyl-propionamid,
[73] Cyclohexancarbonsäure-(4-dimethylamino-benzo[d]isoxazol-3-yl)-amid, .
[74] N-(4-Dimethylamino-benzo[d]isoxazol-3-yl)-2,2-diphenyl-acetamid,
[75] Furan-2-carbonsäure-(4-dimethylamino-benzo[d]isoxazol-3-yl)-amid,
[76] N-(4-Dimethylamino-benzo[d]isoxazol-3-yl)-2,2,3,3,4,4,4-heptafluoro-butyramid,
[77] 4-Fluoro-N-[4-(2,2,2-trifluoro-ethoxy)-benzo[d]isoxazol-3-yl]-2-trifluoromethyl-benzamid,
[78] 2,4-Dichloro-5-fluoro-N-[4-(2 ,2 ,2-trifluoro-ethoxy)-benzo[d]isoxazol-3-yl]-benzamid,
[79] 3-Fluoro-N-[4-(2,2,2-trifluoro-ethoxy)-benzo[d]isoxazol-3-yl]-4-trifluoromethyl-benzamid,
[80] 2,3,4-Trifluoro-N-[4-(2,2,2-trifluoro-ethoxy)-benzo[d]isoxazol-3-yl]-benzamid.
[81] Naphthalen-2-carbonsäure-[4-(2,2,2-trifluoro-ethoxy)-benzo[d]isoxazol-3-yl]-amid,
[82] 3,4-Difluoro-N-[4-(2,2,2-trifluoro-ethoxy)-benzo[d]isoxazol-3-yl]-benzamid,
[83] 2-Fluoro-N-[4-(2,2,2-trifluoro-ethoxy)-benzo[d]isoxazol-3-yl]-3-trifluoromethyl-benzamid,
[85] 2-Ethyl-hexansäure-benzo[d]isoxazol-3-ylamid,
[86] N-Benzo[d]isoxazol-3-yl-2-methyl-butyramid,
[88] N-Benzo[d]isoxazol-3-yl-3-fluoro-4-trifluoromethyl-benzamid,
[89] N-Benzo[d]isoxazol-3-yl-2,3,6-trifluoro-benzamid,
[90] N-Benzo[d]isoxazol-3-yl-nicotinamid,
[91] 5-Methyl-isoxazol-3-carbonsäure-benzo[d]isoxazol-3-ylamid,
[92] Benzo[b]thiophen-3-carbonsäure-benzo[d]isoxazol-3-ylamid,
[94] 4-tert-Butyl-N-(7-fluoro-benzo[d]isoxazol-3-yl)-benzamid,
[96] 4-Fluoro-N-(7-fluoro-benzo[d]isoxazol-3-yl)-benzamid,
[100] 3,5-Difluoro-N-(6-fluoro-benzo[d]isoxazol-3-yl)-benzamid,
[101] Naphthalen-1-carbonsäure-(6-fluoro-benzo[d]isoxazol-3-yl)-amid,
[102] Adamantan-1-carbonsäure-(6-fluoro-benzo[d]isoxazol-3-yl)-amid,
[104] 4-tert-Butyl-N-(5-fluoro-benzo[d]isoxazol-3-yl)-benzamid,
[105] 2,4-Difluoro-Ng5-fluoro-benzo[d]isoxazol-3-ylfbenzamid,
[106] Naphthalen-2-carbonsäure-(5-fluoro-benzo[d]isoxazol-3-yl)-amid,
[107] N-(5-Fluoro-benzo[d]lsoxazol-3-yl)-4-propyl-benzamid,
[109] 4-tert-Butyl-N-(6-chloro-benzo[d]isoxazol-3-yl)-benzamid,
[110] N-(6-Chloro-benzo[d]isoxazol-3-yl)-4-fluoro-2-trifluoromethyl-benzamid,
[111] 2,4-Dichloro-N-(6-chloro-benzo[d]isoxazol-3-yl)-5-fluoro-benzamid,
[112] 2-Ethyl-hexansäure-(6-chloro-benzo[d]isoxazol-3-yl)-amid,
[113] N-(6-Chloro-benzo[d]isoxazol-3-yl)-2-methyl-butyramid,
[115] N-(6-Bromo-benzo[d]isoxazol-3-yl)-3,4-dichloro-benzamid,
[116] Thiophen-2-carbonsäure-(6-bromo-benzo[d]isoxazol-3-yl)-amid,
[117] N-(6-Bromo-benzo[d]isoxazol-3-yl)-3,3-dimethyl-butyramid,
[119] 4-Bromo-N-(5-methyl-benzo[d]isoxazol-3-yl)-benzamid,
[121] 4-Fluoro-N-(5-methyl-benzo[d]isoxazol-3-yl)-benzamid,
[123] 3-Methyl-N-5(methyl-benzo[d]isoxazol-3-yl)benzamid,
[124] 4-tert-Butyl-N-(5-methyl-benzo[d]isoxazol-3-yl)-benzamid,
[126] 2,3-Difluoro-N-(5-methyl-benzo[d]isoxazol-3-yl)-benzamid,
[127] 2,4-Difluoro-N-(5-methyl-benzo[d]isoxazol-3-yl)-benzamid,
[128] 2-Chloro-N-(5-methyl-benzo[d]isoxazol-3-yl)-nicotinamid,
[129] Cyclopropancarbonsäure-(5-fluoro-benzo[d]isoxazol-3-yl)-amid,
[130] N-(5-Fluoro-benzo[d]isoxazol-3-yl)3,3-dimethyl-butyramid,
[131] 2,4-Difluoro-N-(5-fluoro-benzo[d]isoxazol-3-yl)-benzamid,
[132] 2,5-Dimethyl-furan-3-carbonsäure-(5-fluoro-benzo[d]isoxazol-3-yl)-amid,
[133] N-(5-Bromo-benzo[d]isoxazol-3-yl)-2,3-difluoro-benzamid,
[134] 2,5-Dimethyl-furan-3-carbonsäure-(5-bromo-benzo[d]isoxazol.3.yl).amid,
[135] N-(5-Bromo-benzo[d]isoxazol-3-yl)-2-methyl-butyramid,
[137] N-(5-Bromo-benzo[d]isoxazol-3-yl)-3,4-dimethoxy-benzamid,
[138] N-(5-Bromo-benzo[d]isoxazol-3-yl)-4-methyl-benzamid,
[141] N-(5-Bromo-benzo[d]isoxazol-3-yl)-5-fluoro-2-trifluoromethyl-benzamid,
[142] N-(5-Bromo-benzo[d]isoxazol-3-yl)-3-methoxy-benzamid,
[143] 3-Methyl-N-(5-methyl-benzo[d]isoxazol-3-yl)-benzamid,
[144] 4-Cyano-N-(5-methyl-benzo[d]isoxazol-3-yl)-benzamid,
[145] N-(5-Methyl-benzo[d]isoxazol-3-yl)-2-phenyl-butyramid,
[146] 2-Methyl-pentansäure-(5-methyl-benzo[d]isoxazol-3-yl)-amid,
[148] 4-Chloro-N-(4-fluoro-benzo[d]isoxazol-3-yl)-benzamid,
[150] Adamantan-1-carbonsäure-(4-fluoro-benzo[d]isoxazol-3-yl)-amid,
[151] Adamantan-1-carbonsäure-(4-chloro-benzo[d]isoxazol-3-yl)-amid,
[153] N-(4-Chloro-benzo[d]isoxazol-3-yl)-3-methyl-benzamid,
[154] N-(4-Chloro-benzo[d]isoxazol-3-yl)-2-methyl-butyramid,
[155] N-(4-Chloro-benzo[d]isoxazol-3-yl)-3,3-dimethyl-butyramid,
[156] N-(4-Methoxy-benzo[d]isoxazol-3-yl)-2-methyf-benzamid,
[157] 2-Chloro-N-(4-methoxy-benzo[d]isoxazol-3-yl)-benzamid,
[158] N-(4-Methoxy-benzo[d]isoxazol-3-yl)-2-trifluoromethyl-benzamid,
[159] 2-Ethyl-hexansäure-(4-methoxy-benzo[d]isoxazol-3-yl)-amid,
[160] N-(4-Methoxy-benzo[d]isoxazol-3-yl)-2-methyl-butyramid,
[161] 2-Methyl-pentansäure-(4-methoxy-benzo[d]isoxazol-3-yl)-amid,
[162] Cyclopropancarbonsäure-[4-(2,2,2-trifluoro-ethoxy)benzo[d]isoxazol-3-yl]-amid,
[163] 2-Methyl-N-[4-(2,2,2-trifluoro-ethoxy)-benzo[d]isoxazol-3-yl]-butyramid,
[164] 3,3-Dimethyl-N-[4-(2,2,2-trifluoro-ethoxy)-benzo[d]isoxazol-3-yl]-butyramid,
[165] Cyclohexancarbonsäure-[4-(2,2,2-trifluoro-ethoxy)-benzo[d]isoxazol-3-yl]-amid,
[166] 2,5-Dimethyl-furan-3-carbonsäure-[4-(2,2,2-trifluoro-ethoxy)-benzo[d]Isoxazol-3-yl]-amid,
[167] N-[4-(4-Methyl-benzyloxy)-benzo[d]isoxazol-3-yl]-3-nitro-benzamid,
[168] N-[4-(4-Methyl-benzyloxy)-benzo[d]isoxazol-3-yl]-4-propyl-benzamid,
[169] N-(4-Dimethylamino-benzo[d]isoxazol-3-yl)-3-fluoro-5-trifluoromethyl-benzamid,
[170] 3,4-Dichloro-N-(4-dimethylamino-benzo[d]isoxazol-3-yl)-benzamid,
[171] N-(4-Dimethylamino-benzo[d]isoxazol-3-yl)-3-nitro-benzamid,
[172] Adamantan-1-carbonsäure-(4-dimethylamino-benzo[d]isoxazol-3-yl)-amid,
[173] Benzo[b]thiophen-2-carbonsäure-(4-dimethylamino-benzo[d]isoxazol-3-yl)-amid,
[174] N-(4-Dimethylamino-benzo[d]isoxazol-3-yl)-3,3-dimethyl-butyramid,
[176] N-(6-Amino-4,5,7-trifluorö-benzo[d]isoxazol-3-yl)-2,6-difluoro-benzamid
   und
[177] N-(6-Amino-4,5,7-trifluoro-benzo[d]isoxazol-3-yl)-2,4-difluoro-benzamid,
jeweils ggf. in Form eines ihrer reinen Stereoisomeren, insbesondere Enantiomeren oder Diastereomeren oder Rotameren, ihrer Racemate oder in Form einer Mischung von Stereoisomeren, Insbesondere der Enantiomeren und/oder Diastereomeren und/oder Rotameren, in einem beliebigen Mischungsverhältnis, oder jeweils in Form entsprechender Salze, oder jeweils in Form entsprechender Solvate.

Ein weiterer Gegenstand der vorliegenden Erfindung ist ein Verfahren zur Herstellung der vorstehend angegebenen Verbindungen der allgemeinen Formel la gemäß dem wenigstens eine Verbindung der allgemeinen Formel IIa, 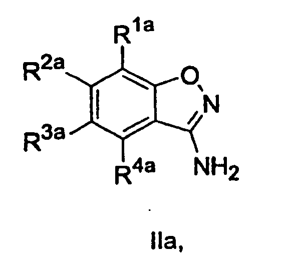 worin R^{1a} bis R^{4a} die Bedeutung wie durch die obigen Verbindungen definiert haben,
in einem Reaktionsmedium, ggf. in Gegenwart wenigstens einer Base, mit wenigstens einer Verbindung der allgemeinen Formel R^{5a}-C(=O)-X, worin R^{5a} die Bedeutung wie durch die obigen Verbindungen definiert hat und X für eine Abgangsgruppe, vorzugsweise einen Halogen-Rest steht, besonders bevorzugt für ein Chloratom steht,
oder in einem Reaktionsmedium, in Gegenwart wenigstens eines Kupplungsreagenzes, ggf. in Gegenwart wenigstens einer Base, mit wenigstens einer Verbindung der allgemeinen Formel R^{5a}-C(=O)-OH, worin R^{5a} die vorstehend genannte Bedeutung hat, zu einer obengenannten Verbindung umgesetzt wird und diese ggf. isoliert und/oder gereinigt wird.

Das erfindungsgemäße Verfahren zur Herstellung erfindungsgemäßer substituierter N-Benzo[d]isoxazol-3-yl-amin-Derivate der vorstehend angegebenen allgemeinen Formel la ist auch im folgenden Schema 1 angegeben. Das erfindungsgemäße Verfahren eignet sich ebenfalls zur Herstellung erfindungsgemäßer substituierter N-Benzo[d]isoxazol-3-yl-amin-Derivate der vorstehend angegebenen allgemeinen Formel I. 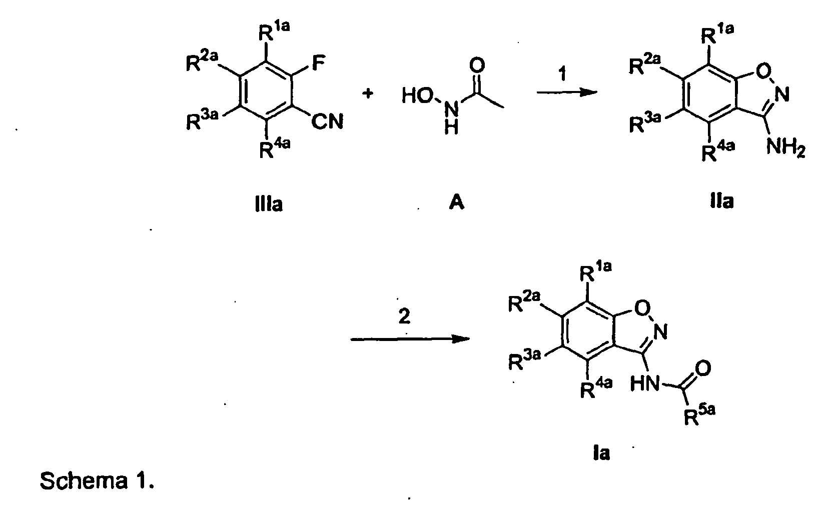 In Stufe 1 werden substituierte 2-Fluorbenzonitrile der allgemeinen Formel IIIa, worin R^{1a} bis R^{4a} die vorstehend genannte Bedeutung haben, in einem Reaktionsmedium, vorzugsweise ausgewählt aus der Gruppe bestehend aus Diethylether,

Tetrahydrofuran, Acetonitril, Dimethylsulfoxid, Dimethylformamid und Dichlormethan, in Gegenwart wenigstens einer Base, vorzugsweise in Gegenwart wenigstens eines Alkalimetallalkoholatsalzes, besonders bevorzugt in Gegenwart eines Alkalimetällalkoholatsalzes ausgewählt aus der Gruppe bestehend aus Kaliummethanolat, Natriummethanolat, Kalium-tert-butylat und Natrium-tert-butylat, mit Acetohydroxamsäure (A) vorzugsweise bei Temperaturen von 20°C bis 100 °C zu Verbindungen der allgemeinen Formel IIa umgesetzt.

In Stufe 2 werden Verbindungen der vorstehend angegebenen allgemeinen Formel IIa mit Carbonsäuren der allgemeinen Formel R^{5a}-C(=O)-OH, worin R^{5a} die vorstehend genannte Bedeutung hat, in einem Reaktionsmedium, vorzugsweise ausgewählt aus der Gruppe bestehend aus Diethylether, Tetrahydrofuran, Acetonitril, Dimethylformamid und Dichlormethan, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodümid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, Dimethylaminopyridin und Diisopropylethylamin vorzugsweise bei Temperaturen von -70°C bis 100°C zu Verbindungen der allgemeinen Formel la umgesetzt.

Alternativ werden Verbindungen der allgemeinen Formel IIa mit Carbonsäurederivaten bzw. Kohlensäurederivaten der allgemeinen Formel R^{5a}-C(=O)-X, wobei X für einen Halogen-Rest, vorzugsweise Chlor oder Brom, steht, in einem Reaktionsmedium, vorzugsweise ausgewählt aus der Gruppe bestehend aus Diethylether, Pyridin, Tetrahydrofuran, Acetonitril, Dimethylformamid und Dichlormethan, bevorzugt in Gegenwart wenigstens einer organischen oder anorganischen Base, beispielsweise Triethylamin, Dimethylaminopyridin, Pyridin, Diisopropylamin, Alkalimetallhydroxide, Alkalimetallcarbonate, Erdalkalimetallhydroxide und Erdalkalimethallcarbonate, besonders bevorzugt in Gegenwart einer organischen Base ausgewählt aus der Gruppe bestehend aus Triethylamin, Dimethylaminopyridin, Pyridin und Diisopropylamin, bei Temperaturen von vorzugsweise -70°C bis 100°C zu Verbindungen der allgemeinen Formel la umgesetzt.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die Verbindungen der vorstehend angegebenen Formeln IIIa sowie der allgemeinen Formeln R^{5a}-C(=O)-OH und R^{5a}-C(=O)-X sind jeweils am Markt käuflich erhältlich und/oder können nach den üblichen, dem Fachmann bekannten Verfahren hergestellt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre oder Argonatmosphäre, durchgeführt werden.

Die erfindungsgemäßen substituierten N-Benzo[d]isoxazol-3-ylamin-Derivate der vorstehend genannten allgemeinen Formeln I und la sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten N-Benzo[d]isoxazol-3-ylamin-Derivate der vorstehend genannten allgemeinen Formeln I und la sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden.

Die freien Basen der jeweiligen substituierten N-Benzo[d]isoxazol-3-ylamin-Derivate der vorstehend genannten allgemeinen Formeln I und la und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten N-Benzo[d]isoxazol-3-ylamin-Derivate der vorstehend genannten allgemeinen Formeln 1 und la und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-AlkylRest steht, genannt.

Die erfindungsgemäßen substituierten N-Benzo[d]isoxazol-3-ylamin-Derivate der vorstehend genannten allgemeinen Formeln I und la und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten N-Benzo[d]isoxazol-3-ylamin-Derivate der vorstehend genannten allgemeinen Formeln I und la nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Ein weiterer Gegenstand der vorliegenden Erfindung ist ein Arzneimittel enthaltend wenigstens eine erfindungsgemäße Verbindung der allgemeinen Formel la, ggf. in Form einer ihrer reinen Stereoisomeren, insbesondere Enantiomeren oder Diastereomeren oder Rotameren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, sowie ggf. einen oder mehrere physiologisch verträgliche Hilfsstoffe.

Das erfindungsgemäße Arzneimittel eignet sich insbesondere zur mGluR5-Rezeptor-Regulation, vorzugsweise zur Inhibierung des mGluR5-Rezeptors, und/oder zur Noradrenalin-Rezeptor-Regulation, vorzugsweise zur Noradrenalin-Wiederaufnahme-Hemmung, und/oder zur Serotonin-(5-HT)-Rezeptor-Regulation, vorzugsweise zur Serotonin-Wiederaufnahme-Hemmung, und somit auch zur Prophylaxe und/oder Behandlung von Störungen und Krankheiten, die zumindest teilweise durch mGluR5-Rezeptoren und/oder Noradrenalin-Rezeptoren und/oder Serotonin-Rezeptoren vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Narkolepsie; Diarrhoe; Gastritis; Magengeschwür; Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit;
zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Regulation des kardiovaskulären Systems; zur Lokalanästhesie; zur Vigilanzsteigerung; zur Libidosteigerung; zur Diurese und/oder zur Antinatriurese.

Besonders bevorzugt eignet sich das erfindungsgemäße Arzneimittel zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Angstzuständen; Panikattacken; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin-oder Kokain-, und/oder Medikamentenabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz.

Ein weiterer Gegenstand der vorliegenden Erfindung ist die Verwendung wenigstens eines erfindungsgemäßen substituierten N-Benzo[d]isoxazol-3-yl-amin-Derivats der vorstehend angegebenen allgemeinen Formel la, jeweils ggf. in form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur mGluR5-Rezeptor-Regulation, vorzugsweise zur Inhibierung des mGluR5-Rezeptors, und/oder zur Noradrenalin-Rezeptor-Regulation, vorzugsweise zur Noradrenalin-Wiederaufnahme-Hemmung, und/oder zur Serotonin-(5-HT)-Rezeptor-Regulation, vorzugsweise zur Serotonin-Wiederaufnahme-Hemmung.

Bevorzugt ist die Verwendung wenigstens eines erfindungsgemäßen substituierten N-Benzo[d]isoxazol-3-yl-amin-Derivats der vorstehend angegebenen allgemeinen Formel la, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen und Krankheiten, die zumindest teilweise durch mGluR5-Rezeptoren und/oder Noradrenalin-Rezeptoren und/oder Serotonin-Rezeptoren vermittelt werden.

Besonders bevorzugt ist die Verwendung wenigstens eines erfindungsgemäßen substituierten N-Benzo[d]isoxazol-3-yl-amin-Derivats der vorstehend angegebenen allgemeinen Formel la, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Narkolepsie; Diarrhoe; Gastritis; Magengeschwür; Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin-oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Regulation des kardiovaskulären Systems; zur Lokalanästhesie; zur Vigilanzsteigerung; zur Libidosteigerung; zur Diurese und/oder zur Antinatriurese.

Ganz besonders bevorzugt ist die Verwendung wenigstens eines erfindungsgemäßen substituierten N-Benzo[d]isoxazol-3-yl-amin-Derivats der vorstehend angegebenen allgemeinen Formel la, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Schmerz, vorzugsweise Schmerz ausgewählt aus der Gruppe bestehend aus akutem Schmerz, chronischem Schmerz, visceralem Schmerz und neuropathischem Schmerz; Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Angstzuständen; Panikattacken; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol-und/oder Drogen-, vorzugsweise Nikotin- oder Kokain-, und/oder Medikamentenabhängigkeit.

Noch weiter bevorzugt ist die Verwendung wenigstens eines erfindungsgemäßen substituierten N-Benzo[d]isoxazol-3-yl-amin-Derivats der vorstehend angegebenen allgemeinen Formel la, jeweils ggf. in Form eines seiner reinen Stereoisomeren, insbesondere Enantiomeren oder Diastereomeren oder Rotameren, seiner Racemate oder in Form einer Mischung von Stereoisomeren, insbesondere der Enantiomeren und/oder Diastereomeren und/oder Rotameren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden. Neben wenigstens einem substituierten N-Benzo[d]isoxazol-3-yl-amin-Derivat der vorstehend angegebenen allgemeinen Formel la, ggf. in Form eines seiner reinen Stereoisomeren, insbesondere Enantiomeren oder Diastereomeren oder Rotameren, seines Racemates oder in Form von Mischungen der Stereoisomeren, insbesondere der Enantiomeren oder Diastereomeren oder Rotameren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen substituierten N-Benzo[d]isoxazol-3-yl-amin-Derivate der vorstehend angegebenen allgemeinen Formel la können in einem Depot, in gelöster Form oder in einem Pflaster, gegebenenfalls unter Zusatz von die Hautpenetration fördernden Mitteln, als geeignete perkutane Applikationszubereitungen vorliegen. Oral oder perkutan anwendbare Zubereitungsformen können die jeweilige erfindungsgemäßen substituierten N-Benzo[d]isoxazol-3-yl-amin-Derivate der vorstehend angegebenen allgemeinen Formel la auch verzögert freisetzen. Prinzipiell können dem erfindungsgemäßen Arzneimittel andere dem Fachmann bekannte weitere Wirkstoffe zugesetzt werden.

Die Herstellung der erfindungsgemäßen Arzneimittel, erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten N-Benzo[d]isoxazol-3-yl-amin-Derivate der vorstehend angegebenen allgemeinen Formel la kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

### Pharmakologische Methoden

### I. Methode zur Bestimmung der Noradrenalin- und der 5HT-Uptake-Inhibierung:

Für in vitro Studien wurden Synaptosomen aus Rattenhimarealen frisch isoliert, wie in der Veröffentlichung,, The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wurde in eisgekühlter 0,32 M Sucrose (100 mg Gewebe / 1mL) in einem Glas-Homogenisierer mit Teflonstößel homogenisiert, indem fünf volle Auf- und Abschläge bei 840 Umdrehungen /Minute benutzt wurden. Das Homogenat wurde bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert wurden. Der jeweilige Uptake wurde in einer 96-well Mikrotiterplatte gemessen. Das Volumen betrug 250 µl und die Inkubation erfolgte bei Raumtemperatur (ca. 20-25 °C) unter O₂ Atmosphäre.

Die Inkubationszeit betrug 7,5 Minuten für [³H]-NA und 5 Minuten für [³H]-5-HT. Anschließend wurden die 96 Proben durch eine Unifilter GF/B^{®} Mikrotiterplatte (Packard) filtriert und mit 200 mL inkubierten Puffer mit Hilfe eines "Brabdel Cell-Harvester MPXRI-96T" gewaschen. Die Unifilter GF/B Platte wurde bei 55°C 1 h getrocknet. Im Anschluß wurde die Platte mit einem Back seal^{®} (Packard) verschlossen und 35 µl Szintilationsflüssigkeit pro Weil (Ultima Gold^{®}, Packard) versetzt. Nach dem Verschließen mit einem top seal^{®} (Packard) wurde, nach der Einstellung des Gleichgewichts (etwa 5 h), die Radioaktivität in einem,, Trilux 1450 Microbeta" (Wallac) bestimmt.

Folgende Kenndaten wurden für den NA-Transporter ermittelt:
NA-Uptake : Km = 0,32 ± 0,11 µM

Die Menge des bei der vorstehenden Bestimmung eingesetzten Proteins entsprach den aus der Literatur bekannten Werten, wie z.B. in "Protein measurement with the folin phenol reagent", Lowry et al., J. Biol. Chem., 193, 265-275, 1951 beschrieben. Eine detaillierte Methodenbeschreibung kann auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden. Die entsprechenden Literaturbeschreibungen werden hiermit als Referenz eingeführt und gelten als Teil der Offenbarung.

### II. Methode zur Bestimmung der Affinität zum mGluR5-Rezeptor

Schweinehimhomogenat wird durch Homogenisieren (Polytron PT 3000, Kinematica AG, 10.000 Umdrehungen pro Minute für 90 Sekunden) von Schweinehirnhälften ohne Medulla, Cerebellum und Pons in Puffer pH 8.0 (30mM Hepes, Sigma, Bestellnr. H3375 + 1 Tablette Complete auf 100ml, Roche Diagnostics, Bestellnr. 1836145) im Verhältnis 1:20 (Hirngewicht/Wolumen) und Differentialzentrifugation bei 900 x g und 40.000 x g hergestellt. In 250 µl Inkubationsansätzen in 96 Well Mikrotiterplatten werden jeweils 450 µg Protein aus Hirnhomogenat mit 5nM ³[H]-MPEP (Tocris, Bestellnr. R1212) (MPEP = 2-Methyl-6-(3-methoxyphenyl)-ethinylpyridin) und die zu untersuchenden Verbindungen (10 µM im Test) in Puffer (wie oben) bei Raumtemperatur für 60 min inkubiert.

Danach werden die Ansätze mit Hilfe eines Brandel Cell Harvesters (Brandel, TYP Robotic 9600) auf Unifilterplates mit Glasfaserfiltermatten (Perkin Elmer, Bestellnr. 6005177) filtriert und anschließend mit Puffer (wie oben) 3 mal mit je 250 µl pro Probe nach gewaschen. Die Filterplatten werden anschließend für 60 min bei 55 °C getrocknet. Anschließend werden pro Well 30 µL Ultima Gold^{™} Szintillator (Packard BioScience, Bestellnr.6013159) zugegeben und nach 3 Stunden werden die Proben am ß-Counter (Mikrobeta, Perkin Eimer) gemessen. Die unspezifische Bindung wird durch Zugabe von 10 µM MPEP (Tocris, Bestellnir.1212) bestimmt.

Im folgenden wird die Erfindung anhand von Beispielen erläutert. Diese Erläuterungen sind lediglich beispielhaft und schränken den allgemeinen Erfindungsgedanken nicht ein.

### Beispiele

Die Ausbeuten der hergestellten Verbindungen sind nicht optimiert.

Alle Temperaturen sind unkorrigiert.

### Abkürzungen

- aq.: wäßrig
- APCI: atmospheric pressure chemical ionisation
- DCM: Dichlormethan
- DMF: Dimethylformamid
- DMSO: Dimethylsulfoxid
- EtOAc: Ethylacetat
- ges.: gesättigt
- h: Stunden
- min: Minuten
- NMR: Kernresonanzspektroskopie
- RT: Raumtemperatur

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

### Allgemeine Vorschriften für die Herstellung beispielgemäßer substituierter N-Benzo[d]isoxazol-3-yl-amin-Derivate

Die erfindungsgemäßen substituierten *N-*Benzo[d]isoxazol-3-yl-amin-Derivate der allgemeinen Formel **Ia** lassen sich aus substituierten 2-Fluor-benzonitrilen der allgemeinen Formel **IIIa** über zwei Stufen wie in Schema 2 dargestellt erhalten.

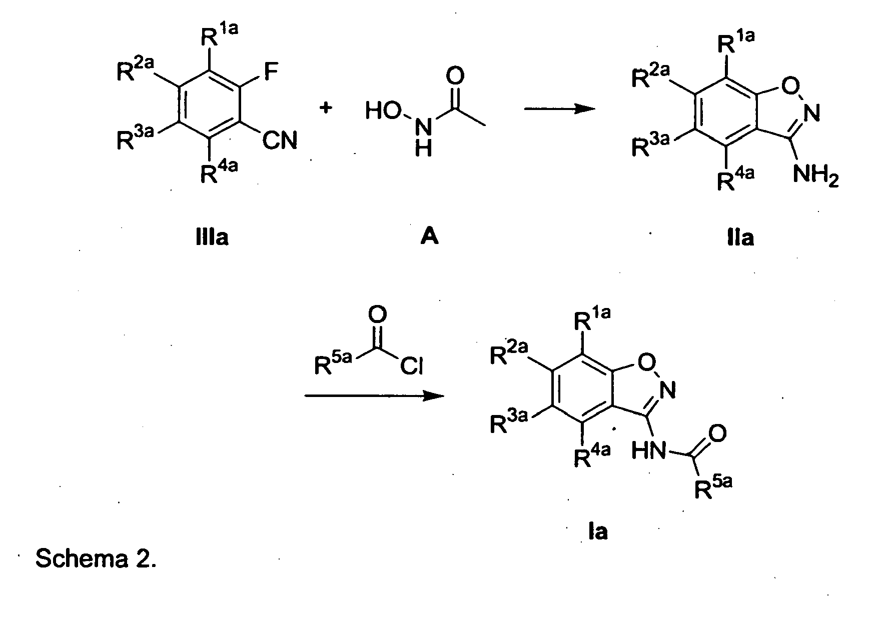

### 1. Allgemeine Vorschrift für die Herstellung substituierter Benzo[d]isoxazol-3-yl-amine der allgemeinen Formel IIa

Acetohydroxamsäure (**A**) (1.1 Äquivalente) wurde unter Inertgasatmosphäre in DMF (1.45 mL pro mmol der Verbindung der allgemeinen Formel **IIIa**) suspendiert. Es wurde Kalium-tert-butylat (1.1 Äquivalente) hinzu gegeben und die Reaktionsmischung wurde 30 min bei RT gerührt, mit der Verbindung der allgemeinen Formel **IIIa** (1 Äquivalent) versetzt und 1 h bei 50 °C gerührt. Nach Abkühlen wurde die Reaktionsmischung auf eine Mischung aus ges. aq. NaCl-Lösung (0.9 mL pro mmol **A**) und EtOAc (0.9 mL pro mmol **A**) gegeben und 30 min gerührt. Die Phasen wurden getrennt und die wäßrige Phase mehrfach mit EtOAc (jeweils 0.8 mL pro mmol **A**) extrahiert. Die vereinigten organischen Phasen wurden mehrfach mit ges. aq. NaCl-Lösung (jeweils 0.8 mL pro mmol **A**) gewaschen, über MgSO₄ getrocknet und das Lösungsmittel wurde im Vakuum entfernt.

Sofern es notwendig war zur Aufreinigung das entsprechende Hydrochlorid zu bilden, wurde der Rückstand jeweils in Methylethylketon (8.7 mL pro g Rückstand) aufgenommen. Nach Zugabe von Wasser (0.1 mL pro g Rückstand) wurde unter langsamen Rühren und Eiskühlung Trimethylchlorsilan (0.7 mL pro g Rückstand) hinzu getropft. Zum Kristallisieren wurde die Reaktionsmischung auf 4 °C gekühlt. Der ausgefallene Niederschlag wurde abfiltriert und im Exikkator mit Hilfe von Phosphorpentoxid als Trockenmittel getrocknet.

Auf diesem Weg wurden die folgenden Verbindungen der allgemeinen Formel **IIa** ggf. in Form des entsprechenden Hydrochlorids bzw. Dihydrochlorids erhalten.
[A1] Benzo[d]isoxazol-3-ylamin
[A2] 4-(4-Methyl-benzyloxy)-benzo[d]isoxazol-3-ylamin 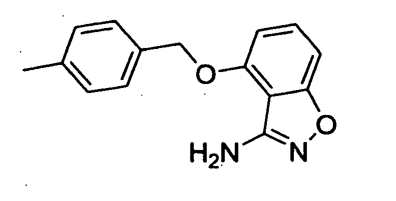 δ (DMSO, 300 MHz) = 2,32 (s, 3 H); 5,24 (s, 2 H); 6,74 (d, 1 H, J = 7,9 Hz); 6,94 (d, 1 H, J = 8,3 Hz); 7,20 (d, 2 H, J = 8,3 Hz); 7,38 (dd, 3 H, J = 10,9 Hz, J = 9,0 Hz).
[A3] 4-Methoxy-benzo[d]isoxazol-3-ylamin
[A4] 4-Chloro-benzo[d]isoxazol-3-ylamin Dihydrochlorid 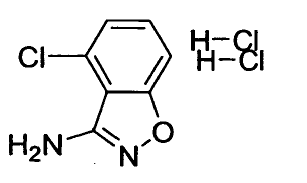 δ (DMSO, 300 MHz) = 6,09 (br.s, 4 H); 7,25 (dd, 1 H, J = 0,8 Hz, J = 7,5 Hz); 7,41 - 7,55 (m, 2 H).
[A5] 4-Fluoro-benzo[d]isoxazol-3-ylamin
[A6] Benzo[d]isoxazol-3,4-diamin Hydrochlorid 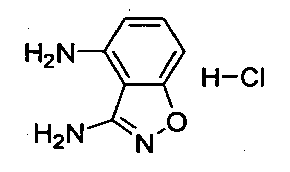 δ (DMSO, 300 MHz) = 6,39 (t, 1 H, J = 8,7 Hz); 6,62 (d, 1 H, J = 8,7 Hz); 7,27 (dd, 1 H, J = 6,8 Hz, J = 8,3 Hz); 8,10 (br.s, 3 H).
[A7] N⁴,N⁴-Dimethyl-benzo[d]isoxazol-3,4-diamin Hydrochlorid 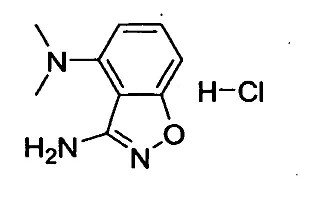 δ (DMSO, 300 MHz) = 3,04 (s, 3 H), 3,07 (s, 3 H); 6,70 (t, 1 H, J = 8,7 Hz); 6,77 (d, 1 H, J = 8,7 Hz); 7,15 (d, 1 H, J = 7,9 Hz); 7,33 (d, 1 H, J = 8,3 Hz); 7,41 - 7,58 (m, 1 H).
[A8] 4-(2,2,2-Trifluoro-ethoxy)-benzo[d]isoxazol-3-ylamin
   δ (DMSO, 300 MHz) = 4,91 - 5,11 (m, 2H); 5.80 (br.s, 2 H), 7,08 - 7,31 (m, 2 H); 7,73 - 7,85 (m, 1 H).
[A9] 5-Methyl-benzo[d]isoxazol-3-ylamin
[A10] 5-Bromo-benzo[d]isoxazol-3-ylamin
[A11] 5-Fluoro-benzo[d]isoxazol-3-ylamin Hydrochlorid 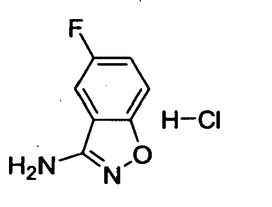 δ (DMSO, 300 MHz) = 7,39 (dt, 1 H, J = 2,2 Hz, J = 9,0 Hz); 7,49 (dd, 1 H, J = 4,2 Hz, J = 9,0 Hz); 7,78 (dd, 1 H, J = 2,2 Hz, J = 7,5 Hz).
[A12] 6-Fluoro-benzo[d]isoxazol-3-ylamin
[A13] 6-Chloro-benzo[d]isoxazol-3-ylamin Hydrochlorid 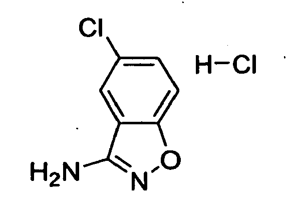 δ (DMSO, 300 MHz) = 7,29 (dd, 1 H, J = 1,5 Hz, J = 7,5 Hz); 7,59 (d, 1 H, J = 1,9 Hz); 7,93 (d, 1 H, J = 8,3 Hz); 8,73 (br.s, 1 H).
[A14] 6-Bromo-benzo[d]isoxazol-3-ylamin
[A15] 7-Fluoro-benzo[d]isoxazol-3-ylamin
   δ (DMSO, 300 MHz) = 6,53 (s, 2 H); 7,22 (dt, J = 4,2 Hz, J = 7,5 Hz); 7,37 (dd, 1 H, J = 10,9 Hz, J = 12,0 Hz); 7,66 (d, 1 H, J = 7,9 Hz).
[A16] 4,5,7-Trifluoro-benzo[d]isoxazol-3,6-diamin 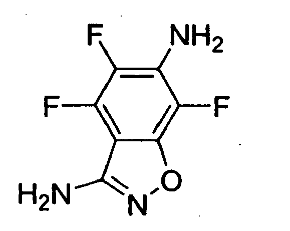 MS (APCI) = 204 [M⁺ + 1]; 184 [M⁺ + 1 - HF]

### 2. Allgemeine Vorschrift zur Umsetzung von substituierten Benzo[d]isoxazol-3-yl-aminen zu substituierten N-Benzo[d]isoxazol-3-yl-amin-Derivaten der allgemeinen Formel Ia

### a. Manuelle Synthese

Die substituierten Benzo[d]isoxazol-3-yl-aminen der allgemeine Formel **IIa** (2 Äquivalente) wurden in Pyridin (0.5 mL pro mmol Benzo[d]isoxazol-3-yl-amin der allgemeinen Formel **IIa**) gelöst. Das Reaktionsgemisch wurde auf - 15 °C abgekühlt, langsam mit dem entsprechenden Carbonsäurechlorid R^{5a}-C(=O)-Cl (1 Äquivalent) versetzt und über Nacht gerührt. Zur Aufarbeitung wurde die Reaktionsmischung auf Wasser geschüttet (8.5 mL pro mmol Benzo[d]isoxazol-3-yl-amin der allgemeinen. Formel **IIa**). Der entstandene Niederschlag wurde abgesaugt, mit Wasser gewaschen, im Vakuum getrocknet und aus Toluol umkristallisiert.

Die folgenden erfindungsgemäßen substituierten *N-*Benzo[d]isoxazol-3-yl-amin-Derivate wurden wie unter 2a. beschrieben hergestellt.

### Beispielverbindungen

1. Thiophen-2-carbonsäure-benzo[d]isoxazol-3-ylamid
   δ (DMSO, 600 MHz) = 7,26 (t,1 H, J = 4,5 Hz); 7,37 (t, 1 H, J = 7,6 Hz); 7,63 - 7,71 (m, 2 H); 7,93 (d, 1 H, J = 4,5 Hz); 8,10 (d, 1 H, J = 7,6 Hz); 8,21 (d, 1 H, J = 3,0 Hz), 11,53 (s, 1 H).
3. Naphthalen-2-carbonsäure-benzo[d]isoxazol-3-ylamid
   δ (DMSO, 600 MHz) = 7,39 (t, 1 H, J = 7,3 Hz); 7,61 - 7,73 (m, 4 H); 8,00 - 8,13 (m, 5 H); 8,76 (s, 1 H); 11,61 (s, 1 H).
4. Adamantan-2-carbonsäurebenzo[d]isoxazol-3-ylamid
   δ (DMSO, 600 MHz) = 1,71 - 1,75 (m, 6 H); 1,97 - 2,02 (m, 6 H); 2,04 - 2,07 (m, 3 H); 7,32 (dd, 1 H, J = 6,8 Hz, J = 7,5 Hz); 7,58 - 7,67 (m, 2 H); 7,84 (dd, 1 H, J = 6,8 Hz, J = 7,5 Hz); 10,38 (s, 1 H).
5. Cyclohexancarbonsäure-benzo[d]isoxazol-3-ylamid
   δ (DMSO, 600 MHz) = 1,22 - 1.51 (m, 3 H); 1,52 - 1,80 (m, 3 H); 1,81 - 1,95 (m, 2 H); 1,98 - 2,13 (m, 2 H); 2,45 - 2,64 (m, 1 H); 7,23 - 7, (m, 1 H); 7,46 - 7,65 (m, 2 H); 8,29 (d, 1H, J = 8,3 Hz); 9,12 (s, 1H).
6. N-Benzo[d]isoxazol-3-yl-2,2-dimethyl-propionamid
   δ (DMSO, 600 MHz) = 1,31 (s, 9 H); 7,33 (t, 1 H, 7,5 Hz); 7,59 - 7,65 (m, 2 H); 7,83 - 7,86 (m, 1 H); 10,47 (s, 1 H).

### b. Automatisierte Synthese

Es wurden zunächst folgende Stammlösungen erzeugt:
- Lösung I:: 0.1 M Lösung des Benzo[d]isoxazol-3-yl-amins der allgemeinen Formel IIa in Pyridin
- Lösung II:: 0.5 M Lösung des Carbonsäurechlorids der allgemeinen Formel R^{5a}-C(=O)-Cl in Pyridin
- Lösung III:: 0.1 M Lösung Triethylamin in Pyridin

Lösung I (1 mL) wurde in einem trockenen Gewindeglas mit Septumkappe bei RT vorgelegt und mit Lösung II (1 mL) sowie mit Lösung III (1 mL) versetzt. Das Reaktionsgemisch wurde 24 h bei RT im Trefferreaktor (Firma Zymark, Rüsselsheim, Deutschland) gerührt und in der Quench-Station (Firma Zymark, Rüsselsheim, Deutschland) bei RT mit 1 M Salzsäure-Lösung (2 mL) versetzt, so daß ein pH-Wert von 3 erreicht wurde. Nach 30 min wurde im Transferblock DCM (2 mL) hinzu pipettiert und die Reaktionsmischung 30 min im Spin-Reaktor durchmischt. Der Rührfisch wurde abfiltriert, und das Gefäß mit DCM (2 mL) ausgespült.
Die organische Phase wurde abgenommen und gesammelt. Die wäßrige Phase wurde mit DCM (1.5 mL) versetzt, im Vortexer behandelt und 15 min im Spin-Reaktor intensiv durchmischt. Nach Zentrifugieren wurde die organische Phasen abgetrennt und mit der ersten organischen Phase vereinigt. Die wäßrige Phase wird analog ein zweites Mal mit DCM extrahiert. Anschließend wurden die vereinigten organischen Phasen über eine Magnesiumsulfat-Kartusche getrocknet und das Lösungsmittel in einem Evaporatorsystem GeneVac HT Series 1(GeneVac, Wiesbaden, Deutschland) entfernt.

Die folgenden erfindungsgemäßen substituierten *N-*Benzo[d]isoxazol-3-yl-amin-Derivate wurden wie unter 2b. beschrieben hergestellt.

### Beispielverbindungen

7. N-(4-Methoxy-benzo[d]isoxazol-3-yl)-2,2-diphenyl-acetamid
   MS (APCI) = 359 [M⁺ + 1]
8. Cyclopropancarbonsäure-(5-bromo-benzo[d]isoxazol-3-yl)-amid
   MS (APCI) = 281 [M (⁷⁹Br)⁺ + 1]; 283 [M (⁸¹Br)⁺ + 1], 213.
9. 3,5-Dichloro-N-(6-fluoro-benzo[d]isoxazol-3-yl)-benzamid
   MS (APCI) = 325 [M(³⁵Cl³⁵Cl)⁺ + 1]; 327 [M(³⁵Cl³⁷Cl)⁺ + 1]; 329 [M(³⁷Cl³⁷Cl)⁺ + 1]
10. N-(4-Amino-benzo[d]isoxazol-3-yl)-4-nitro-benzamid
   MS (APCI) = 299 [M⁺ + 1], 150
11. Naphthalin-1-carbonsäure-(4-amino-benzo[d]isoxazol-3-yl)-amid
   MS (APCI) = 304 [M⁺ + 1], 155.
12. Benzo[b]thiophen-2-carbonsäure-(4-fluoro-benzo[d]isoxazol-3-yl)-amid
   MS (APCI) =313 [M⁺ + 1], 161.
13. N-Benzo[d]isoxazol-3-yl-2-trifluoromethyl-benzamid
   MS (APCI) = 307 [M⁺ + 1]; 287.
14. N-Benzo[d]isoxazol-3-yl-3,4-dichloro-benzamid
   MS (APCI) = 307 [M(³⁵Cl³⁵Cl)⁺ + 1]; 309 [M(³⁵Cl³⁷Cl)⁺ + 1]; 311 [M(³⁷Cl³⁷Cl)⁺ + 1]
15. N-Benzo[d]isoxazol-3-yl-2,3-difluoro-benzamid
   MS (APCI) = 275 [M⁺ + 1]
16. N-(4-Dimethylamino-benzo[d]isoxazol-3-yl)-3-fluoro-benzamid
   MS (APCI) = 300 [M⁺ + 1]
17. Thiophen-2-carbonsäure-(6-fluoro-benzo[d]isoxazol-3-yl)-amid
   MS (APCI) = 263 [M⁺ 1], 153.
18. N-(6-Fluoro-benzo[d]isoxazol-3-yl)-2-methyl-butyramid
   MS (APCI) =237 [M⁺ + 1], 153.
19. N-(6-Chloro-benzo[d]isoxazol-3-yl)-2-fluoro-3-trifluoromethyl-benzamid
   MS (APCI) = 359 [M (³⁵Cl)⁺ + 1]; 361[M (³⁷Cl)⁺ + 1].
20. N-(6-Chloro-benzo[d]isoxazol-3-yl)-3-methoxy-benzamid
   MS (APCI) = 303 [M (³⁵Cl)⁺ + 1]; 305 [M (³⁷Cl)⁺ + 1], 135.
21. N-(6-Bromo-benzo[d]isoxazol-3-yl)-4-tert-butyl-benzamid
   MS (APCI) = 373 [M (⁷⁹Br)⁺ + 1]; 375 [M (⁸¹Br)⁺ + 1].
22. N-(6-Bromo-benzo[d]isoxazol-3-yl)-2-methoxy-benzamid
   MS (APCI) = 347 [M (⁷⁹Br)⁺ + 1]; 349 [M (⁸¹Br)⁺ + 1].
23. N-(6-Bromo-benzo[d]isoxazol-3-yl)-2-trifluoromethyl-benzamid
   MS (APCI) = 385 [M (⁷⁹Br)⁺ + 1]; 387 [M (⁸¹Brt)⁺ + 1].
24. Adamantan-2-carbonsäure-(6-bromo-benzo[d]isoxazol-3-yl)-amid
   MS (APCI) = 375 [M (⁷⁹Br)⁺ + 1]; 377 [M (⁸¹Br)⁺ + 1]
25. N-(6-Bromo-benzo[d]isoxazol-3-yl)-2-methyl-butyramid
   MS (APCI) = 297 [M (⁷⁹Br)⁺ + 1]; 299 [M (⁸¹Br)⁺ + 1], 213.
26. N-(5-Fluoro-benzo[d]isoxazol-3-yl)-2-trifluoromethyl-benzamid
   MS (APCI) =325 [M⁺ + 1]; 305.
27. 3,4-Dichloro-N-(5-fluoro-benzo[d]isoxazol-3-yl)-benzamid
   MS (APCI) = 325 [M(³⁵Cl³⁵Cl)⁺ + 1]; 327 [M(³⁵Cl³⁷Cl)⁺ + 1]; 329 [M(³⁷Cl³⁷Cl)⁺ + 1]
28. N-(5-Fluoro-benzo[d]isoxazol-3-yl)-2-methyl-benzamid
   MS (APCI) = 271 [M⁺ + 1]; 119.
29. N-(5-Bromo-benzo[d]isoxazol-3-yl)-2-fluoro-5-trifluoromethyl-benzamid
   MS (APCI) = 403 [M (⁷⁹Br)⁺ + 1];405 [M (⁸¹Br)⁺ + 1], 363, 191.
30. N-(5-Bromo-benzo[d]isoxazol-3-yl)-2,4-difluoro-benzamid
   MS (APCI) = 353 [M (⁷⁹Br)⁺ + 1]; 355 [M (⁸¹Br)⁺ + 1], 312, 216, 141.
31. N-(5-Methyl-benzo[d]isoxazol-3-yl)-2-trifluoromethyl-benzamid
   MS (APCI) = 321 [M⁺ + 1], 301, 173.
32. 3-Fluoro-N-(5-methyl-benzo[d]isoxazol-3-yl)-benzamid
   MS (APCI) = 271 [M⁺ + 1].
33. N-(4-Methoxy-benzo[d]isoxazol-3-yl)-3,5-bis-trifluoromethyl-benzamid
   MS (APCI) = 297 [M⁺ + 1]
34. 3,5-Difluoro-N-(4-methoxy-benzo[d]isoxazol-3-yl)-benzamid
   MS (APCI) =271 [M⁺ + 1]
35. N-(4-Dimethylamino-benzo[d]isoxazol-3-yl)-3,5-bis-trifluoromethyl-benzamid
   MS (APCI) = 418 [M⁺ + 1].
36. 2-Bromo-N-[4-(2,2,2-trifluoro-ethoxy)-benzo[d]isoxazol-3-yl]-benzamid
   MS (APCI) = 415 [M (⁷⁹Br)⁺ + 1]; 417 [M (⁸¹Br)⁺ + 1]
37. 3-Chloro-N-[4-(2,2,2-trifluoro-ethoxy)-benzo[d]isoxazol-3-yl]-benzamid
   MS (APCI) = 371 [M (³⁵Cl)⁺ + 1]; 373 [M (³⁷Cl)⁺ + 1], 139.
38. 4-tert-Butyl-N-[4-(2,2,2-trifluoro-ethoxy)-benzo[d]isoxazol-3-yl]-benzamid
   MS (APCI) = 393 [M⁺ + 1], 161.
39. 2-Methoxy-N-[4-(2,2,2-trifluoro-ethoxy)-benzo[d]isoxazol-3-yl]-benzamid
   MS (APCI) = 367 [M⁺ + 1], 134.
40. N-(6-Chloro-benzo[d]isoxazol-3-yl)-4-iodo-benzamid
   MS (APCI) = 399 [M (³⁵Cl)⁺ + 1]; 401 [M (³⁷Cl)⁺ + 1]
41. Naphthalen-2-carbonsäure-(6-chloro-benzo[d]isoxazol-3-yl)-amid
   MS (APCI) = 323 [M⁺ + 1]
42. N-(6-Chloro-benzo[d]isoxazol-3-yl)-3,4-difluoro-benzamid
   MS (APCI) = 363 [M (³⁵Cl)⁺ + 1]; 365 [M (³⁷Cl)⁺ + 1]
43. N-(6-Bromo-benzo[d]isoxazol-3-yl)-4-fluoro-2-trifluoromethyl-benzamid
   MS (APCI) = 403 [M (⁷⁹Br)⁺ + 1]; 405 [M (⁸¹Br)⁺ + 1]
44. 2,4-Dichloro-N-(6-bromo-benzo[d]isoxazol-3-yl)-5-fluoro-benzamid
   MS (APCI) = 403 [M(³⁵Cl³⁵Cl⁷⁹Br)⁺ + 1]; 405 [M(³⁵Cl³⁷Cl⁷⁹Br)⁺ + 1], [Me(³⁵Cl³⁵Cl⁸¹Br)+ + 1], 407 [M(³⁷Cl³⁷Cl⁷⁹Br)⁺ + 1], [M(³⁷Cl³⁵Cl⁸¹Br)⁺ + 1], 409 [M(³⁷Cl³⁷Cl⁸¹Br)⁺ + 1]
45. N-(6-Bromo-benzo[d]isoxazol-3-yl)-3-fluoro-4-trifluoromethyl-benzamid
   MS (APCI) = 403 [M (⁷⁹Br)⁺ + 1]; 405 [M (⁸¹Br)⁺ + 1]
46. N-(6-Bromo-benzo[d]isoxazol-3-yl)-3-fluoro-5-trifluoromethyl-benzamid
   MS (APCI) = 403 [M (⁷⁹Br)⁺ + 1]; 405 [M (⁸¹Br)⁺ + 1]
47. N-(6-Bromo-benzo[d]isoxazol-3-yl)-2,3,4-trifluoro-benzamid
   MS (APCI) = 371 [M (⁷⁹Br)⁺ + 1]; 373 [M (⁸¹Br)⁺ + 1]
48. N-(6-Bromo-benzo[d]isoxazol-3-yl)-4-propyl-benzamid
   MS (APCI) = 359 [M (⁷⁹Br)⁺ + 1]; 361 [M (⁸¹Br)⁺ + 1]
49. N-(6-Bromo-benzo[d]isoxazol-3-yl)-3,4-difluoro-benzamid
   MS (APCI) = 353 [M (⁷⁹Br)⁺ + 1]; 355 [M (⁸¹Br)⁺ + 1]
50. Furan-2-carbonsäure-(5-bromo-benzo[d]isoxazol-3-yl)-amid
   MS (APCI) = 307 [M (⁷⁹Br)⁺ + 1]; 309 [M (⁸¹Br)⁺ + 1]
51. 4-Fluoro-N-(7-fluoro-benzo[d]isoxazol-3-yl)-2-trifluoromethyl-benzamid
   MS (APCI) = 343 [M⁺ + 1]
52. 2,4-Dichloro-5-fluoro-N-(7-fluoro-benzo[d]isoxazol-3-yl)-benzamid
   MS (APCI) = 343 [M(³⁵Cl³⁵Cl)⁺ + 1]; 345 M(³⁵Cl³⁷Cl)⁺ + 1]; 347 M(³⁷Cl³⁷Cl)⁺ + 1]
53. 3-Fluoro-N-(7-fluoro-benzo[d]isoxazol-3-yl)-4-trifluoromethyl-benzamid
   MS (APCI) = 343 [M⁺ + 1]
54. 2,3,4-Trifluoro-N-(7-fluoro-benzo[d]isoxazol-3-yl)-benzamid
   MS (APCI) = 311 [M⁺ + 1]
55. N-(7-Fluoro-benzo[d]isoxazol-3-yl)-4-iodo-benzamid
   MS (APCI) = 383 [M⁺ + 1]
56. 2-Chloro-N-(7-fluoro-benzo[d]isoxazol-3-yl)-nicotinamid
   MS (APCI) = 292 [M (³⁵Cl)⁺ + 1]; 294. [M (³⁷Cl)⁺ + 1]; 256 [M⁺ - HCl]
57. Naphthalen-2-carbonsäure-(7-fluoro-benzo[d]isoxazol-3-yl)-amid
   MS (APCI) = 307 [M⁺ + 1]
58. N-(74-Fluoro-benzo[d]isoxazol-3-yl)-4-propyl-benzamid
   MS (APCI) = 298 [M⁺ + 1]
59. 3,4-Difluoro-N-(7-fluoro-benzo[d]isoxazol-3-yl)-benzamid
   MS (APCI) = 293 [M⁺ + 1]
60. 2-Fluoro-N-(7-fluoro-benzo[d]isoxazol-3-yl)-3-trifluoromethyl-benzamid
   MS (APCI) = 343 [M⁺ + 1]
61. N-(7-Fluoro-benzo[d]isoxazol-3-yl)-3-methoxy-benzamid
   MS (APCI) = 287 [M⁺ + 1], 135 [M⁺ - Benzisoxazol]
62. N-(7-Fluoro-benzo[d]isoxazol-3-yl)-2,2-diphenyl-acetamid
   MS (APCI) = 343 [M⁺ + 1], 167 [CHPh₂⁺]
63. Furan-2-carbonsäure-(7-fluoro-benzo[d]isoxazol-3-yl)-amid
   MS (APCI) = 247 [M⁺ + 1]
64. 2,4-Dichloro-N-(4-dimethylamino-benzo[d]isoxazol-3-yl)-5-fluoro-benzamid
   MS (APCI) = 276 [M(³⁵Cl³⁵Cl)⁺ + 1]; 278 M(³⁵Cl³⁷Cl)⁺ + 1]: 280 M(³⁷Cl³⁷Cl)⁺ + 1]. 191 [M(³⁵Cl³⁵Cl)⁺ - Benzisoxazol]
65. N-(4-Dimethylamino-benzo[d]isoxazol-3-yl)-2,3,4-trifluoro-benzamid
   MS (APCI) = 247 [M⁺ + 1], 159 [M⁺ - Benzisoxazol]
66. 2-Chloro-N-(4-dimethylamino-benzo[d]isoxazol-3-yl)-nicotinamid
   MS (APCI) = 317 [M(³⁵Cl³⁵Cl)⁺ +1]; 319 [M(³⁵Cl³⁷Cl)⁺ + 1], 321 [M(³⁷Cl³⁷Cl)⁺ + 1], 281 [M(³⁵Cl³⁵Cl)⁺ - HCl],
67. Naphthalen-2-carbonsäure-(4-dimethylamino-benzo[d]isoxazol-3-yl)-amid
   MS (APCI ) = 332 [M⁺ + 1], 155 [M⁺ - Benzisoxazol]
68. N-(4-Dimethylamino-benzo[d]isoxazol-3-yl)-4-propyl-benzamid
   MS (APCI) = 324 [M⁺ + 1], 147 [M⁺ - Benzisoxazol]
69. N-(4-Dimethylamino-benzo[d]isoxazol-3-yl)-3,4-difluoro-benzamid
   MS (APCI) = 324 [M⁺ + 1], 147 [M⁺ - Benzisoxazol]
70. N-(4-Dimethylamino-benzo[d]isoxazol-3-yl)-2-fluoro-3-trifluoromethyl-benzamid
   MS (APCI) = 368 [M⁺ + 1], 191 [M⁺ - Benzisoxazol]
71. N-(4-Dimethylamino-benzo[d]isoxazol-3-yl)-3-methoxy-benzamid
   MS (APCI) = 312 [M⁺ + 1], 135 [M⁺ - Benzisoxazol]
72. N-(4-Dimethylamino-benzo[d]isoxazol-3-yl)-2,2-dimethyl-propionamid
   MS (APCI) = 262 [M⁺ + 1]
73. Cyclohexancarbonsäure-(4-dimethylamino-benzo[d]isoxazol-3-yl)-amid
   MS (APCI) = 288 [M⁺ + 1], 178 [Benzisoxazol +1]
74. N-(4-Dimethylamino-benzo[d]isoxazol-3-yl)-2,2-diphenyl-acetamid
   MS (APCI) = 372 [M⁺ + 1], 178 [Benzisoxazol +1]
75. Furan-2-carbonsäure-(4-dimethylamino-benzo[d]isoxazol-3-yl)-amid
   MS (APCI) = 372 [M⁺ + 1]
76. N-(4-Dimethylamino-benzo[d]isoxazol-3-yl)-2,2,3,3,4,4,4-heptafluoro-butyramid MS (APCI) = 372 [M⁺ + 1]
77. 4-Fluoro-N-[4-(2,2,2-trifluoro-ethoxy)-benzo[d]isoxazol-3-yl]-2-trifluoromethyl-benzamid
   MS (APCI) = 423 [M⁺ + 1], 191 [M⁺ - Benzisoxazol]
78. 2,4-Dichloro-5-fluoro-N-[4-(2,2,2-trifluoro-ethoxy)-benzo[d]isoxazol-3-yl]-benzamid
   MS (APCI) = 423 [M(³⁵Cl³⁵Cl)⁺ + 1]; 425 [M(³⁵Cl³⁷Cl)⁺ + 1], 427 [M(³⁷Cl³⁷Cl)⁺ + 1]
79. 3-Fluoro-N-[4-(2,2,2-trifluoro-ethoxy)-benzo[d]isoxazol-3-yl]-4-trifluoromethyl-benzamid
   MS (APCI) = 423 [M⁺ + 1], 191 [M⁺ - Benzisoxazol]
80. 2,3,4-Trifluoro-N-[4-(2,2,2-trifluoro-ethoxy)-benzo[d]isoxazol-3-yl]-benzamid
   MS (APCI) = 391 [M⁺ + 1], 159 [M⁺ - Benzisoxazol]
81. Naphthalen-2-carbonsäure-[4-(2,2,2-trifluoro-ethoxy)-benzo[d]isoxazol-3-yl]-amid
   MS (APCI) = 387 [M⁺ + 1], 155 [M⁺ - Benzisoxazol]
82. 3,4-Difluoro-N-[4-(2,2,2-trifluoro-ethoxy)-benzo[d]isoxazol-3-yl]-benzamid
   MS (APCI) = 373 [M⁺ + 1], 141 [M⁺ - Benzisoxazol]
83. 2-Fluoro-N-[4-(2,2,2-trifluoro-ethoxy)-benzo[d]isoxazol-3-yl]-3-trifluoromethyl-benzamid
   MS (APCI) = 373 [M⁺ + 1], 141 [M⁺ - Benzisoxazol]
85. 2-Ethyl-hexansäure-benzo[d]isoxazol-3-ylamid
86. N-Benzo[d]isoxazol-3-yl-2-methyl-butyramid
87. N-Benzo[d]isoxazol-3-yl-2,6-difluoro-benzamid
88. N-Benzo[d]isoxazol-3-yl-3-fluoro-4-trifluoromethyl-benzamid
89. N-Benzo[d]isoxazol-3-yl-2,3,6-trifluoro-benzamid
90. N-Benzo[d]isoxazol-3-yl-nicotinamid
91. 5-Methyl-isoxazol-3-carbonsäure-benzo[d]isoxazol-3-ylamid
92. Benzo[b]thiophen-3-carbonsäure-benzo[d]isoxazol-3-ylamid
93. 2-Chloro-N-(7-fluoro-benzo[d]isoxazol-3-yl)-benzamid
94. 4-tert-Butyl-N-(7-fluoro-benzo[d]isoxazol-3-yl)-benzamid
95. N-(7-Fluoro-benzo[d]isoxazol-3-yl)-benzamid
96. 4-Fluoro-N-(7-fluoro-benzo[d]isoxazol-3-yl)-benzamid
97. 2-Fluoro-N-(7-fluoro-benzo[d]isoxazol-3-yl)-benzamid
98. N-(7-Fluoro-benzo[d]isoxazol-3-yl)-2-methoxy-benzamid
99. N-(7-Fluoro-benzo[d]isoxazol-3-yl)-2-methyl-benzamid
100. 3,5-Difluoro-N-(6-fluoro-benzo[d]isoxazol-3-yl)-benzamid
101. Naphthalen-1-carbonsäure-(6-fluoro-benzo[d]isoxazol-3-yl)-amid
102. Adamantan-1-carbonsäure-(6-fluoro-benzo[d]isoxazol-3-yl)-amid
103. 2-Bromo-N-(5-fluoro-benzo[d]isoxazol-3-yl)-benzamid
104. 4-tert-Butyl-N-(5-fluoro-benzo[d]isoxazol-3-yl)-benzamid
105. 2,4-Difluoro-N-(5-fluoro-benzo[d]isoxazol-3-yl)-benzamid
106. Naphthalen-2-carbonsäure-(5-fluoro-benzo[d]isoxazol-3-yl)-amid
107. N-(5-Fluoro-benzo[d]isoxazol-3-yl)-4-propyl-benzamid
108. 2-Chloro-N-(6-chloro-benzo[d]isoxazol-3-yl)-benzamid
109. 4-tert-Butyl-N-(6-chloro-benzo[d]isoxazol-3-yl)-benzamid
110. N-(6-Chloro-benzo[d]isoxazol-3-yl)-4-fluoro-2-trifluoromethyl-benzamid
111. 2,4-Dichloro-N-(6-chloro-benzo[d]isoxazol-3-yl)-5-fluoro-benzamid
112. 2-Ethyl-hexansäure-(6-chloro-benzo[d]isoxazol-3-yl)-amid
113. N-(6-Chloro-benzo[d]isoxazol-3-yl)-2-methyl-butyramid
114. N-(6-Bromo-benzo[d]isoxazol-3-yl)-2-chloro-benzamid
115. N-(6-Bromo-benzo[d]isoxazol-3-yl)-3,4-dichloro-benzamid
116. Thiophen-2-carbonsäure-(6-bromo-benzo[d]isoxazol-3-yl)-amid
117. N-(6-Bromo-benzo[d]isoxazol-3-yl)-3,3-dimethyl-butyramid
118. N-(5-Methyl-benzo[d]isoxazol-3-yl)-benzamid
119. 4-Bromo-N-(5-methyl-benzo[d]isoxazol-3-yl)-benzamid
120. 2-Chloro-N-(5-methyl-benzo[d]isoxazol-3-yl)-benzamid
121. 4-Fluoro-N-(5-methyl-benzo[d]isoxazol-3-yl)-benzamid
122. 2-Fluoro-N-(5-methyl-benzo[d]isoxazol-3-yl)-benzamid
123. 3-Methyl-N-(5-methyl-benzo[d]isoxazol-3-yl)-benzamid
124. 4-tert-Butyl-N-(5-methyl-benzo[d]isoxazol-3-yl)-benzamid
125. 2-Methyl-N-(5-methyl-benzo[d]isoxazol-3-yl)-benzamid
126. 2,3-Difluoro-N-(5-methyl-benzo[d]isoxazol-3-yl)-benzamid
127. 2,4-Difluoro-N-(5-methyl-benzo[d]isoxazol-3-yl)-benzamid
128. 2-Chloro-N-(5-methyl-benzo[d]isoxazol-3-yl)-nicotinamid
129. Cyclopropancarbonsäure-(5-fluoro-benzo[d]isoxazol-3-yl)-amid
130. N-(5-Fluoro-benzo[d]isoxazol-3-yl)-3,3-dimethyl-butyramid
131. 2,4-Difluoro-N-(5-fluoro-benzo[d]isoxazol-3-yl)-benzamid
132. 2,5-Dimethyl-furan-3-carbonsäure-(5-fluoro-benzo[d]isoxazol-3-yl)-amid
133. N-(5-Bromo-benzo[d]isoxazol-3-yl)-2,3-difluoro-benzamid
134. 2,5-Dimethyl-furan-3-carbonsäure-(5-bromo-benzo[d]isoxazol-3-yl)-amid
135. N-(5-Bromo-benzo[d]isoxazol-3-yl)-2-methyl-butyramid
136. N-(5-Bromo-benzo[d]isoxazol-3-yl)-2,6-difluoro-benzamid
137. N-(5-Bromo-benzo[d]isoxazol-3-yl)-3,4-dimethoxy-benzamid
138. N-(5-Bromo-benzo[d]isoxazol-3-yl)-4-methyl-benzamid
139. N-(5-Bromo-benzo[d]isoxazol-3-yl)-2,6-dimethoxy-benzamid
140. 2-Bromo-N-(5-bromo-benzo[d]isoxazol-3-yl)-benzamid
141. N-(5-Bromo-benzo[d]isoxazol-3-yl)-5-fluoro-2-trifluoromethyl-benzamid
142. N-(5-Bromo-benzo[d]isoxazol-3-yl)-3-methoxy-benzamid
143. 3-Methyl-N-(5-methyl-benzo[d]isoxazol-3-yl)-benzamid
144. 4-Cyano-N-(5-methyl-benzo[d]isoxazol-3-yl)-benzamid
145. N-(5-Methyl-benzo[d]isoxazol-3-yl)-2-phenyl-butyramid
146. 2-Methyl-pentansäure-(5-methyl-benzo[d]isoxazol-3-yl)-amid
147. N-(4-Fluoro-benzo[d]isoxazol-3-yl)-benzamid
148. 4-Chloro-N-(4-fluoro-benzo[d]isoxazol-3-yl)-benzamid
149. 2-Fluoro-N-(4-fluoro-benzo[d]isoxazol-3-yl)-benzamid
150. Adamantan-1-carbonsäure-(4-fluoro-benzo[d]isoxazol-3-yl)-amid
151. Adamantan-1-carbonsäure-(4-chloro-benzo[d]isoxazol-3-yl)-amid
152. N-(4-Chloro-benzo[d]isoxazol-3-yl)-benzamid
153. N-(4-Chloro-benzo[d]isoxazol-3-yl)-3-methyl-benzamid
154. N-(4-Chloro-benzo[d]isoxazol-3-yl)-2-methyl-butyramid
155. N-(4-Chloro-benzo[d]isoxazol-3-yl)-3,3-dimethyl-butyramid
156. N-(4-Methoxy-benzo[d]isoxazol-3-yl)-2-methyl-benzamid
157. 2-Chloro-N-(4-methoxy-benzo[d]isoxazol-3-yl)-benzamid
158. N-(4-Methoxy-benzo[d]isoxazol-3-yl)-2-trifluoromethyl-benzamid
159. 2-Ethyl-hexansäure-(4-methoxy-benzo[d]isoxazol-3-yl)-amid
160. N-(4-Methoxy-benzo[d]isoxazol-3-yl)-2-methyl-butyramid
161. 2-Methyl-pentansäure-(4-methoxy-benzo[d]isoxazol-3-yl)-amid
162. Cyclopropancarbonsäure-[4-(2,2,2-trifluoro-ethoxy)-benzo[d]isoxazol-3-yl]-amid
163. 2-Methyl-N-[4-(2,2,2-trifluoro-ethoxy)-benzo[d]isoxazol-3-yl]-butyramid
164. 3,3-Dimethyl-N-[4-(2,2,2-trifluoro-ethoxy)-benzo[d]isoxazol-3-yl]-butyramid
165. Cyclohexancarbonsäure-[4-(2,2,2-trifluoro-ethoxy)-benzo[d]isoxazol-3-yl]-amid
166. 2,5-Dimethyl-furan-3-carbonsäure-[4-(2,2,2-trifluoro-ethoxy)-benzo[d]isoxazol-3-yl]-amid
167. N-[4-(4-Methyl-benzyloxy)-benzo[d]isoxazol-3-yl]-3-nitro-benzamid
168. N-[4-(4-Methyl-benzyloxy)-benzo[d]isoxazol-3-yl]-4-propyl-benzamid
169. N-(4-Dimethylamino-benzo[d]isoxazol-3-yl)-3-fluoro-5-trifluoromethyl-benzamid
170. 3,4-Dichloro-N-(4-dimethylamino-benzo[d]isoxazol-3-yl)-benzamid
171. N-(4-Dimethylamino-benzo[d]isoxazol-3-yl)-3-nitro-benzamid
172. Adamantan-1-carbonsäure-(4-dimethylamino-benzo[d]isoxazol-3-yl)-amid
173. Benzo[b]thiophen-2-carbonsäure-(4-dimethylamino-benzo[d]isoxazol-3-yl)-amid
174. N-(4-Dimethylamino-benzo[d]isoxazol-3-yl)-3,3-dimethyl-butyramid
176. N-(6-Amino-4,5,7-trifluoro-benzo[d]isoxazol-3-yl)-2,6-difluoro-benzamid
177. N-(6-Amino-4,5,7-trifluoro-benzo[d]isoxazol-3-yl)-2,4-difluoro-benzamid

### Pharmakologische Daten:

Die Noradrenalin-Wiederaufnahmehemmung (NA-Uptake Inhibierung) und die Serotonin-Wiederaufnahmehemmung (5-HT-Uptake Inhibierung) der erfindungsgemäßen substituierten N-Benzo[d]isoxazol-3-yl- amin-Derivate wurde wie vorstehend beschrieben bestimmt.

Die erfindungsgemäßen substituierten N-Benzo[d]isoxazol-3-yl-amin-Derivate weisen eine ausgezeichnete Affinität zum Noradrenalin-Rezeptor und zum 5-HT-Rezeptor auf.

### NA-Uptake Inhibierung

| **Verbindung gemäß Beispiel** | **R¹** | **R²** | **R³** | **R⁴** | **R⁵** | **NA-Uptake Inhibierung [%] Konz. 10 µM** |
|---|---|---|---|---|---|---|
| **7** | H | H | H | OCH₃ | CH(Phenyl)₂ | 63 |
| **8** | H | H | Br | H | Cyclopropyl | 62 |
| **9** | H | F | H | H | 3,5-Cl₂-Phenyl | 67 |
| **10** | H | H | H | NH₂ | 4-NO₂-Phenyl | 66 |
| **11** | H | H | H | NH₂ | 1-Naphthyl | 71 |
| **12** | H | H | H | F | 2-Benzo[b]thiophen | 60 |
| **40** | H | Cl | H | H | 4-I-Ph | 61 |
| **41** | H | Cl | H | H | 2-Naphthyl | 61 |
| **42** | H | Cl | H | H | 3,4-F₂Ph | 64 |
| **19** | H | Cl | H | H | 3-CF₃-2-F-Ph | 63 |
| **43** | H | Br | H | H | 2-CF₃-4-FPh | 66 |
| **44** | H | Br | H | H | 2,4-Cl₂-5-FPh | 61 |
| **45** | H | Br | H | H | 4-CF₃-3-F-Ph | 64 |
| **46** | H | Br | H | H | 5-CF₃-3-F-Ph | 65 |
| **47** | H | Br | H | H | 2,3,4-F₃Ph | 66 |
| **48** | H | Br | H | H | 4-*n*-Pr-Ph | 61 |
| **49** | H | Br | H | H | 3,4-F₂-Ph | 65 |
| **50** | H | H | Br | H | 2-Furyl | 66 |
| **51** | F | H | H | H | 2-CF3-4-F-Ph | 71 |
| **52** | F | H | H | H | 2,4-Cl₂-5-FPh | 70 |
| **53** | F | H | H | H | 4-CF₃-3-F-Ph | 69 |
| **54** | F | H | H | H | 2,3,4-F₃Ph | 63 |
| **55** | F | H | H | H | 4-I-Ph | 69 |
| **56** | F | H | H | H | 2-Cl-3-Pyridinyl | 72 |
| **57** | F | H | H | H | 2-Naphthyl | 67 |
| **58** | F | H | H | H | 4-*n-*Pr-Ph | 64 |
| **59** | F | H | H | H | 3,4-F₂-Ph | 67 |
| **60** | F | H | H | H | 3-CF₃-2-F-Ph | 66 |
| **61** | F | H | H | H | 3-OMe-Ph | 66 |
| **62** | F | H | H | H | CHPh₂ | 63 |
| **63** | F | H | H | H | 2-Furyl | 64 |
| **64** | H | H | H | NMe₂ | 2,4-Cl₂-5-F-Ph | 63 |
| **65** | H | H | H | NMe₂ | 2,3,4-F₃-Ph | 68 |
| **66** | H | H | H | NMe₂ | 2-Cl-3-Pyridinyl | 64 |
| **67** | H | H | H | NMe₂ | 2-Naphthyl | 73 |
| **68** | H | H | H | NMe₂ | 4-*n-*Pr-Ph | 73 |
| **69** | H | H | H | NMe₂ | 3,4-F₂-Ph | 67 |
| **70** | H | H | H | NMe₂ | 3-CF₃-2-F-Ph | 66 |
| **71** | H | H | H | NMe₂ | 3-OMe-Ph | 65 |
| **72** | H | H | H | NMe₂ | C(CH₃)₃ | 66 |
| **73** | H | H | H | NMe₂ | Cyclohexyl | 70 |
| **74** | H | H | H | NMe₂ | CHPh₂ | 70 |
| **75** | H | H | H | NMe₂ | 2-Furyl | 64 |
| **76** | H | H | H | NMe₂ | (CF₂)₂CF₃ | 67 |
| **77** | H | H | H | OCH₂CF₃ | 2-CF₃-4-F-Ph | 70 |
| **78** | H | H | H | OCH₂CF₃ | 2,4-Cl₂-5-FPh | 66 |
| **79** | H | H | H | OCH₂CF₃ | 4-CF₃-3-F-Ph | 62 |
| **80** | H | H | H | OCH₂CF₃ | 2,3,4-F₃Ph | 66 |
| **81** | H | H | H | OCH₂CF₃ | 2-Naphthyl | 65 |
| **82** | H | H | H | OCH₂CF₃ | 3,4-F₂-Ph | 68 |
| **83** | H | H | H | OCH₂CF₃ | 3-CF₃-2-F-Ph | 65 |

### 5-HT-Uptake Inhibierung

| **Verbindung gemäß Beispiel** | **R¹** | **R²** | **R³** | **R⁴** | **R⁵** | **5 HT-Uptake Inhibierung [%] Konz. 10 µM** |
|---|---|---|---|---|---|---|
| **13** | H | H | H | H | 2-CF₃-Phenyl | 84 |
| **14** | H | H | H | H | 3,4-Cl₂-Phenyl | 69 |
| **15** | H | H | H | H | 2.,3-F₂-Phenyl | 86 |
| **16** | H | H | H | N(CH₃)₂ | 3-F-Phenyl | 72 |
| **17** | H | F | H | H | 2-Thiophen | 77 |
| **18** | H | F | H | H | 1-Methylpropyl | 70 |
| **19** | H | Cl | H | H | 2-F-3-CF₃-Phenyl | 77 |
| **20** | H | Cl | H | H | 3-OCH₃-Phenyl | 77 |
| **21** | H | Br | H | H | 4-(tert.-Butyl)-Phenyl | 81 |
| **22** | H | Br | H | H | 2-OCH₃-Phenyl | 79 |
| **23** | H | Br | H | H | 2-CF₃-Phenyl | 86 |
| **24** | H | Br | H | H | Adamantyl | 79 |
| **25** | H | Br | H | H | 1-Methylpropyl | 86 |
| **26** | H | H | F | H | 2-CF₃-Phenyl | 82 |
| **27** | H | H | F | H | 3,4-Cl₂-Phenyl | 89 |
| **28** | H | H | F | H | 2-CH₃-Phenyl | 91 |
| **29** | H | H | Br | H | 2-F-5-CF₃-Phenyl | 83 |
| **30** | H | H | Br | H | 2,4-F₂-Phenyl | 79 |
| **31** | H | H | CH3 | H | 2-CF₃-Phenyl | 69 |
| **32** | H | H | CH₃ | H | 3-F-Phenyl | 89 |
| **33** | H | H | H | OCH₃ | 3,5-(CF₃)₂-Phenyl | 85 |
| **34** | H | H | H | OCH₃ | 3,5-F₂-Phenyl | 78 |
| **35** | H | H | H | N(CH₃)₂ | 3,5-(CF₃)₂-Phenyl | 86 |
| **36** | H | H | H | OCH₂CF₃ | 2-Br-Phenyl | 84 |
| **37** | H | H | H | OCH₂CF₃ | 3-Cl-Phenyl | 90 |
| **38** | H | H | H | OCH₂CF₃ | 4-(tert.-Butyl)-Phenyl | 77 |
| **39** | H | H | H | OCH₂CF₃ | 2-OCH₃-Phenyl | 82 |

Die Affinität der erfindungsgemäßen substituierten N-Benzo[d]isoxazol-3-yl-amin-Derivate der allgemeinen Formel I für den mGluR5-Rezeptor wurde wie vorstehend beschrieben bestimmt.

Die erfindungsgemäßen substituierten N-Benzo[d]isoxazol-3-yl-amin-Derivate zeigen eine ausgezeichnete Affinität für den mGluR5-Rezeptor

### mGluR5-Rezeptor Inhibierung

| **Bsp.** | **R¹** | **R²** | **R³** | **R⁴** | **R⁵** | **mGluR5-Rezeptor Hemmung der ³[H]-MPEP-Bindung in % (10 µM)** |
|---|---|---|---|---|---|---|
| **85** | H | H | H | H | 3-Heptyl | 63,19 |
| **86** | H | H | H | H | 2-Butyl | 39,59 |
| **87** | H | H | H | H | 2,6-F₂-Phenyl | 37,01 |
| **88** | H | H | H | H | 3-F-4-CF₃-Phenyl | 30,03 |
| **89** | H | H | H | H | 2,3,6-F₃-Phenyl | 51,10 |
| **90** | H | H | H | H | 3-Pyridyl | 50,28 |
| **91** | H | H | H | H | 3-(5-Methyl)-isoxazolyl | 35,00 |
| **92** | H | H | H | H | 2-Benzo[b]thiophenyl | 55,98 |
| **93** | F | H | H | H | 2-Cl-Phenyl | 35,39 |
| **94** | F | H | H | H | 4-(tert.-Butyl)-Phenyl | 30,79 |
| **95** | F | H | H | H | Phenyl | 45,82 |
| **96** | F | H | H | H | 4-F-Phenyl | 30,47 |
| **98** | F | H | H | H | 2-OCH₃-Phenyl | 32,01 |
| **99** | F | H | H | H | 2-CH₃-Phenyl | 43,05 |
| **100** | H | F | H | H | 3,5-F₂-Phenyl | 33,33 |
| **101** | H | F | H | H | 1-Naphthyl | 48,99 |
| **102** | H | F | H | H | Adamantyl | 52,11 |
| **103** | H | H | F | H | 2-Br-Phenyl | 41,17 |
| **104** | H | H | F | H | 4-(tert.-Butyl)-Phenyl | 44,61 |
| **28** | H | H | F | H | 2-CH₃-Phenyl | 35,00 |
| **105** | H | H | F | H | 2,4-F₂-Phenyl | 53.77 |
| **106** | H | H | F | H | 2-Naphthyl | 33,72 |
| **107** | H | H | F | H | 4-Propyl-Phenyl | 42,12 |
| **108** | H | Cl | H | H | 2-Cl-Phenyl | 34,73 |
| **109** | H | Cl | H | H | 4-(tert.-Butyl)-Phenyl | 35,20 |
| **110** | H | Cl | H | H | 4-F-2-CF₃-Phenyl | 35,96 |
| **111** | H | Cl | H | H | 2,4-Cl₂-5-F-Phenyl | 43,05 |
| **112** | H | Cl | H | H | 3-Heptyl | 35,12 |
| **113** | H | Cl | H | H | 2-Butyl | 49,16 |
| **49** | H | Br | H | H | 3,4-F₂-Phenyl | 36,90 |
| **114** | H | Br | H | H | 2-Cl-Phenyl | 37,51 |
| **115** | H | Br | H | H | 3,4-Cl₂-Phenyl | 35,84 |
| **116** | H | Br | H | H | 2-Thiophenyl | 37,71 |
| **117** | H | Br | H | H | 2,2-Dimethylpropyl | 30,78 |
| **118** | H | H | CH₃ | H | Phenyl | 69,01 |
| **119** | H | H | CH₃ | H | 4-Br-Phenyl | 37,90 |
| **120** | H | H | CH₃ | H | 2-Cl-Phenyl | 47,86 |
| **121** | H | H | CH₃ | H | 4-F-Phenyl | 32,22 |
| **122** | H | H | CH₃ | H | 2-F-Phenyl | 41,03 |
| **123** | H | H | CH₃ | H | 3-CH₃-Phenyl | 89,70 |
| **124** | H | H | CH₃ | H | 4-(tert.-Butyl)-Phenyl | 43,51 |
| **125** | H | H | CH₃ | H | 2-CH₃-Phenyl | 40,62 |
| **126** | H | H | CH₃ | H | 2,3-F₂-Phenyl | 39,49 |
| **127** | H | H | CH₃ | H | 2,4-F₂-Phenyl | 43,22 |
| **128** | H | H | CH₃ | H | 3-(2-Cl)-Pyridyl | 33,12 |
| **129** | H | H | F | H | Cyclopropyl | 38,82 |
| **130** | H | H | F | H | 2,2-Dimethylpropyl | 31,64 |
| **131** | H | H | F | H | 2,4-F₂-Phenyl | 42,52 |
| **132** | H | H | F | H | 3-(2,5-Dimethyl)-furanyl | 36,88 |
| **30** | H | H | Br | H | 2,4-F₂-Phenyl | 45,22 |
| **133** | H | H | Br | H | 2,3-F₂-Phenyl | 34,56 |
| **134** | H | H | Br | H | 3-(2,5-Dimethyl)-furanyl | 43,11 |
| **135** | H | H | Br | H | 2-Butyl | 40,00 |
| **136** | H | H | Br | H | 2,6-F₂-Phenyl | 40,76 |
| **137** | H | H | Br | H | 3,4-(OCH₃)₂-Phenyl | 31,25 |
| **138** | H | H | Br | H | 4-CH₃-Phenyl | 31,96 |
| **139** | H | H | Br | H | 2,6-(OCH₃)₂-Phenyl | 41,55 |
| **140** | H | H | Br | H | 2-Br-Phenyl | 38,42 |
| **141** | H | H | Br | H | 5-F-2-CF₃-Phenyl | 33,08 |
| **142** | H | H | Br | H | 3-OCH₃-Phenyl | 37,84 |
| **32** | H | H | CH₃ | H | 3-F-Phenyl | 40,06 |
| **143** | H | H | CH₃ | H | 3-CH₃-Phenyl | 44,66 |
| **144** | H | H | CH₃ | H | 4-CN-Phenyl | 48,79 |
| **145** | H | H | CH₃ | H | 1-Phenyl-propyl | 43,51 |
| **146** | H | H | CH₃ | H | 2-Pentyl | 33,97 |
| **147** | H | H | H | F | Phenyl | 31,35 |
| **148** | H | H | H | F | 4-Cl-Phenyl | 79,45 |
| **149** | H | H | H | F | 2-F-Phenyl | 32,45 |
| **150** | H | H | H | F | Adamantyl | 53,18 |
| **151** | H | H | H | Cl | Adamantyl | 74,05 |
| **152** | H | H | H | Cl | Phenyl | 31,62 |
| **153** | H | H | H | Cl | 3-CH₃-Phenyl | 63,76 |
| **154** | H | H | H | Cl | 2-Butyl | 30,02 |
| **155** | H | H | H | Cl | 2,2-Dimethylpropyl | 52,37 |
| **156** | H | H | H | OCH₃ | 2-CH₃-Phenyl | 33,76 |
| **157** | H | H | H | OCH₃ | 2-Cl-Phenyl | 32,39 |
| **158** | H | H | H | OCH₃ | 2-CF₃-Phenyl | 36,13 |
| **159** | H | H | H | OCH₃ | 3-Heptyl | 43,24 |
| **160** | H | H | H | OCH₃ | 2-Butyl | 53,97 |
| **161** | H | H | H | OCH₃ | 2-Pentyl | 38,49 |
| **39** | H | H | H | OCH₂CF₃ | 2-OCH₃-Phenyl | 30,50 |
| **36** | H | H | H | OCH₂CF₃ | 2-Br-Phenyl | 30,31 |
| **77** | H | H | H | OCH₂CF₃ | 4-F-2-CF₃-Phenyl | 37,98 |
| **82** | H | H | H | OCH₂CF₃ | 3,4-F₂-Phenyl | 41,99 |
| **162** | H | H | H | OCH₂CF₃ | Cyclopropyl | 30,99 |
| **163** | H | H | H | OCH2CF₃ | 2-Butyl | 35,96 |
| **164** | H | H | H | OCH₂CF₃ | 2,2-Dimethylpropyl | 33,37 |
| **165** | H | H | H | OCH₂CF₃ | Cyclohexyl | 38,04 |
| **166** | H | H | H | OCH₂CF₃ | 3-(2,5-Dimethyl)-furanyl | 32,39 |
| **167** | H | H | H | OCH₂-(4-CH₃-Phenyl) | 3-NO₂-Phenyl | 39,74 |
| **168** | H | H | H | OCH₂-(4-CH₃-Phenyl) | 4-Propyl-Phenyl | 32,20 |
| **169** | H | H | H | N(CH₃)₂ | 3-F-5-CF₃-Phenyl | 36,77 |
| **170** | H | H | H | N(CH₃)₂ | 3,4-Cl₂-Phenyl | 37,97 |
| **171** | H | H | H | N(CH₃)₂ | 3-NO₂-Phenyl | 42,31 |
| **73** | H | H | H | N(CH₃)₂ | Cyclohexyl | 30,05 |
| **172** | H | H | H | N(CH₃)₂ | Adamantyl | 51,40 |
| **173** | H | H | H | N(CH₃)₂ | 2-Benzo[b]thiophenyl | 43,55 |
| **174** | H | H | H | N(CH₃)₂ | 2,2-Dimethylpropyl | 30,66 |
| **176** | F | NH₂ | F | F | 2,6-F₂-Phenyl | 36,07 |
| **177** | F | NH₂ | F | F | 2,4-F₂-Phenyl | 30,80 |

## Patentansprüche

1. Arzneimittel enthaltend wenigstens eine Verbindung ausgewählt aus der Gruppe bestehend aus
[1] Thiophen-2-carbonsäure-benzo[d]isoxazol-3-ylamid,
*[3] Naphthalen-2-carbonsäure-benzo[d]isoxazol-3-ylamid ,*
[4] Adamantan-2-carbonsäurebenzo[d]isoxazol-3-ylamid,
[5] Cyclohexancarbonsäure-benzo[d]isoxazol-3-ylamid,
[6] N-Benzo[d]isoxazol-3-yl-2,2-dimethyl-propionamid,
[7] N-(4-Methoxy-benzo[d]isoxazol-3-yl)-2,2-diphenyl-acetamid,
[8] Cyclopropancarbonsäure-(5-bromo-benzo[d]isoxazol-3-yl)-amid,
[9] 3,5-Dichloro-N-(6-fluoro-benzo[d]isoxazol-3-yl)-benzamid,
[10] N-(4-Amino-benzo[d]isoxazol-3-yl)-4-nitro-benzamid,
[11] Naphthalin-1-carbonsäure-(4-amino-benzo[d]isoxazol-3-yl)-amid,
[12] Benzo[b]thiophen-2-carbonsäure-(4-fluoro-benzo[d]isoxazol-3-yl)-amid,
[13] N-Benzo[d]isoxazol-3-yl-2-trifluoromethyl-benzamid,
[14] N-Benzo[d]isoxazol-3-yl-3,4-dichloro-benzamid,
[15] N-Benzo[d]isoxazol-3-yl-2,3-difluoro-benzamid,
[16] N-(4-Dimethylamino-benzo[d]isoxazol-3-yl)-3-fluoro-benzamid,
[17] Thiophen-2-carbonsäure-(6-fluoro-benzo[d]isoxazol-3-yl)-amid,
[18] N-(6-Fluoro-benzo[d]isoxazol-3-yl)-2-methyl-butyramid,
[19] N-(6-Chloro-benzo[d]isoxazol-3-yl)-2-fluoro-3-trifluoromethyl-benzamid,
[20] N-(6-Chloro-benzo[d]isoxazol-3-yl)-3-methoxy-benzamid,
[21] N-(6-Bromo-benzo[d]isoxazol-3-yl)-4-tert-butyl-benzamid,
[22] N-(6-Bromo-benzo[d]isoxazol-3-yl)-2-methoxy-benzamid,
[23] N-(6-Bromo-benzo[d]isoxazol-3-yl)-2-trifluoromethyl-benzamid,
[24] Adamantan-2-carbonsäure-(6-bromo-benzo[d]isoxazol-3-yl)-amid,
[25] N-(6-Bromo-benzo[d]isoxazol-3-yl)-2-methyl-butyramid,
[26] N-(5-Fluoro-benzo[d]isoxazol-3-yl)-2-trifluoromethyl-benzamid,
[27] 3,4-Dichloro-N-(5-fluoro-benzo[d]isoxazol-3-yl)-benzamid,
[28] N-(5-Fluoro-benzo[d]isoxazol-3-yl)-2-methyl-benzamid,
[29] N-(5-Bromo-benzo[d]isoxazol-3-yl)-2-fluoro-5-trifluommethyl-benzamid,
[30] N-(5-Bromo-benzo[d]isoxazol-3-yl)-2,4-difluoro-benzamid,
[31] N-(5-Methyl-benzo[d]isoxazol-3-yl)-2-trifluoromethyl-benzamid,
[32] 3-Fluoro-N-(5-methyl-benzo[d]isoxazol-3-yl)benzamid,
[33] N-(4-Methoxy-benzo[d]isoxazol-3-yl)-3,5-bis-trifluoromethyl-benzamid,
[34] 3,5-Difluoro-N-(4-methoxy-benzo[d]isoxazol-3-yl)-benzamid,
[35] N-(4-Dimethylamino-benzo[d]isoxazol-3-yl)-3,5-bis-trifluoromethyl-benzamid,
[36] 2-Bromo-N-[4-(2,2,2-trifluoro-ethoxy)-benzo[d]isoxazol-3-yl]-benzamid,
[37] 3-Chloro-N-[4-(2,2,2-trifluoro-ethoxy)-benzo[d]isoxazol-3-yl]-benzamid,
[38] 4-tert-Butyl-N-[4-(4,4,4-trifluoro-ethoxy)-benzo[d]isoxazol-3-yl]-benzamid,
[39] 2-Methoxy-N-[4-(2,2,2-trifluoro-ethoxy)-benzo[d]isoxazol-3-yl]-benzamid,
[40] N-(6-Chloro-benzo[d]isoxazol-3-yl)-4-iodo-benzamid,
[41] Naphthalen-2-carbonsäure-(6-chloro-benzo[d]isoxazol-3-yl)-amid,
[42] N-(6-Chloro-benzo[d]isoxazol-3-yl)-3,4-difluoro-benzamid,
[43] N-(6-Bromo-benzo[d]isoxazol-3-yl)-4-fluoro-2-trifluoromethyl-benzamid;
[44] 2,4-Dichloro-N-(6-bromo-benzo[d]isoxazol-3-yl)-5-fluoro-benzamid,
[45] N-(6-Bromo-benzo[d]isoxazol-3-yl)-3-fluoro-4-trifluoromethyl-benzamid,
[46] N-(6-Bromo-benzo[d]isoxazol-3-yl)-3-fluoro-5-trifluoromethyl-benzamid,
[47] N-(6-Bromo-benzo[d]isoxazol-3-yl)-2,3,4-trifluoro-benzamid,
[48] N-(6-Bromo-benzo[d]isoxazol-3-yl)-4-propyl-benzamid ,
[49] N-(6-Bromo-benzo[d]isoxazol-3-yl)-3,4-difluoro-benzamid,
[50] Furan-2-carbonsäure-(5-bromo-benzo[d]isoxazol-3-yl)-amid,
[51] 4-Fluoro-N-(7-fluoro-benzo[d]isoxazol-3-yl)-2-trifluoromethyl-benzamid,
[52] 2,4-Dichloro-5-fluoro-N-(7-fluoro-benzo[d]isoxazol-3-yl)-benzamid,
[53] 3-Fluoro-N-(7-fluoro-benzo[d]isoxazol-3-yl)-4-trifluoromethyl-benzamid,
[54] 2,3,4-Trifluoro-N-(7-fluor-benzo[d]isoxazol-3-yl)-benzamid,
[55] N-(7-Fluoro-benzo[d]isoxazol-3-yl)-4-iodo-benzamid,
[56] 2-Chloro-N-(7-fluoro-benzo[d]isoxazol-3-yl)-nicotinamid,
[57] Naphthalen-2-carbonsäure-(7-fluoro-benzo[d]isoxazol-3-yl)-amid,
[58] N-(7-Fluoro-benzo[d]isoxazol-3-yl)-4-propyl-benzamid,
[59] 3,4-Difluoro-N-(7-fluoro-benzo[d]isoxazol-3-yl)-benzamid,
[60] 2-Fluoro-N-(7-fluoro-benzo[d]isoxazol-3-yl)-3-trifluoromethyl-benzamid,
[61] N-(7-Fluoro-benzo[d]isoxazol-3-yl)-3-methoxy-benzamid,
[62] N-(7-Fluoro-benzo[d]isoxazol-3-yl)-2,2-diphenyl-acetamid,
[63] Furan-2-carbonsäure-(7-fluoro-benzo[d]isoxazol-3-yl)amid,
[64] 2,4-Dichloro-N-(4-dimethylamino-benzo[d]isoxazol-3-yl)-5-fluoro-benzamid,
[65] N-(4-Dimethylamino-benzo[d]isoxazol-3-yl)-2,3,4-trifluoro-benzamid,
[66] 2-Chloro-N-(4-dimethylamino-benzo[d]isoxazol-3-yl)-nicotinamid,
[67] Naphthalen-2-carbonsäure-(4-dimethylamino-benzo[d]isoxazol-3-yl)-amid,
[68] N-(4-Dimethylamino-benzo[d]isoxazol-3-yl)-4-propyl-benzamid,
[69] N-(4-Dimethylamino-benzo[d]isoxazol-3-yl)-3,4-difluoro-benzamid,
[70] N-(4-Dimethylamino-benzo[d]isoxazol-3-yl)-2-fluoro-3-trifluoromethyl-benzamid,
[71] N-(4-Dimethylamino-benzo[d]isoxazol-3-yl)-3-methoxy-benzamid,
[72] N-(4-Dimethylamino-benzo[d]isoxazol-3-yl)-2,2-dimethyl-propionamid,
[73] Cyclohexancarbonsäure-(4-dimethylamino-benzo[d]isoxazol-3-yl)-amid,
[74] .N-(4-Dimethylamino-benzo[d]isoxazol-3-yl)-2,2-diphenyl-acetamid,
[75] Furan-2-carbonsäure-(4-dimethylamino-benzo[d]isoxazol-3-yl)-amid,
[76] N-(4-Dimethylamino-benzo[d]isoxazol-3-yl)-2,2,3,3,4,4,4-heptafluoro-butyramid,
[77] 4-Fluoro-N-[4-(2,2,2-trifluoro-ethoxy)-benzo[d]isoxazol-3-yl]-2-trifluoromethyl-benzamid,
[78] 2,4-Dichloro-5-fluoro-N-[4-(2,2,2-trifluoro-ethoxy)-benzo[d]isoxazol-3-yl]-benzamid,
[79] 3-Fluoro-N-[4-(2,2,2-trifluoro-ethoxy)-benzo[d]isoxazol-3-yl]-4-trifluoromethyl-benzamid,
[80] 2,3,4-Trifluoro-N-[4-(2,2,2-trifluoro-ethoxy)-benzo[d]isoxazol-3-yl]-benzamid,
[81] Naphthalen-2-carbonsäure-[4-(2,2,2-trifluoro-ethoxy)-benzo[d]isoxazol-3-yl]-amid,
[82] 3,4-Difluoro-N-[4-(2,2,2-trifluoro-ethoxy)-benzo[d]isoxazol-3-yl]-benzamid,
[83] 2-Fluoro-N-[4-(2,2,2-trifluoro-ethoxy)-benzo[d]isoxazol-3-yl]-3-trifluoromethyl-benzamid,
[85] 2-Ethyl-hexansäure-benzo[d]isoxazol-3-ylamid,
[86] N-Benzo[d]isoxazol-3-yl-2-methyl-butyramid,
[87] N-Benzo[d]isoxazol-3-yl-2,6-difluoro-benzamid,
[88] N-Benzo[d]isoxazol-3-yl-3-fluoro-4-trifluoromethyl-benzamid,
[89] N-Benzo[d]isoxazol-3-yl-2,3,6-trifluoro-benzamid,
[90] N-Benzo[d]isoxazol-3-yl-nicotinamid,
[91] 5-Methyl-isoxazol-3-carbonsäure-benzo[d]isoxazol-3-ylamid,
[92] Benzo[b]thiophen-3-carbonsäure-benzo[d]isoxazol-3-ylamid,
[93] 2-Chloro-N-(7-fluoro-benzo[d]isoxazol-3-yl)-benzamid,
[94] 4-tert-Butyl-N-(7-fluoro-benzo[d]isoxazol-3-yl)-benzamid,
[95] N-(7-Fluoro-benzo[d]isoxazol-3-yl)-benzamid,
[96] 4-Fluoro-N-(7-fluoro-benzo[d]isoxazol-3-yl)-benzamid,
[97] 2-Fluoro-N-(7-fluoro-benzo[d]isoxazol-3-yl)-benzamid,
[98] N-(7-Fluoro-benzo[d]isoxazol-3-yl)-2-methoxy-benzamid,
[99] N-(7-Fluoro-benzo[d]isoxazol-3-yl)-2-methyl-benzamid.
[100] 3,5-Difluoro-N-(6-fluoro-benzo[d]isoxazol-3-yl)-benzamid,
[101] Naphthalen-1-carbonsäure-(6-fluoro-benzo[d]isoxazol-3-yl)-amid,
[102] Adamantan-1-carbonsäure-(6-fluoro-benzo[d]isoxazol-3-yl)-amid,
[103] 2-Bromo-N-(5-fluoro-benzo[d]isoxazol-3-yl)-benzamid,
[104] 4-tet-N-Butyl-N-(5-fluoro-benzo[d]isoxazol-3-yl)-benzamid,
[105] 2,4-Difluoro-N-(5-fluoro-benzo[d]isoxazol-3-yl)-benzamid,
[106] Naphthalen-2-carbonsäure-(5-fluoro-benzo[d]isoxazol-3-yl)-amid,
[107] N-(5-Fluoro-benzo[d]isoxazol-3-yl)-4-propyl-benzamid,
[108] 2-Chloro-N-(6-chloro-benzo[d]isoxazol-3-yl)-benzamid,
[109] 4-tert-Butyl-N-(6-chloro-benzo[d]isoxazol-3-yl)-benzamid,
[110] N-(6-Chloro-benzo[d]isoxazol-3-yl)-4-fluoro-2-trifluoromethyl-benzamid,
[111] 2,4-Dichloro-N-(6-chloro-benzo[d]isoxazol-3-yl)-5-fluoro-benzamid,
[112] 2-Ethyl-hexansäure-(6-chloro-benzo[d]isoxazol-3-yl)-amid,
[113] N-(6-Chloro-benzo[d]isoxazol-3-yl)-2-methyl-butyramid,
[114] N-(6-Bromo-benzo[d]isoxazol-3-yl)-2-chloro-benzamid,
[115] N-(6-Bromo-benzo[d]isoxazol-3-yl)-3,4-dichloro-benzamid,
[116] Thiophen-2-carbonsäure-(6-bromo-benzo[d]isoxazol-3-yl)-amid,
[117] N-(6-Bromo-benzo[d]isoxazol-3-yl)-3,3-dimethyl-butyramid,
[118] N-(5-Methyl-benzo[d]isoxazol-3-yl)-benzamid,
[119] 4-Bromo-N-(5-methyl-benzo[d]isoxazol-3-yl)-benzamid,
[120] 2-Chloro-N-(5-methyl-benzo[d]isoxazol-3-yl)-benzamid,
[121] 4-Fluoro-N-(5-methyl-benzo[d]isoxazol-3-yl)-benzamid,
[122] 2-Fluoro-N-(5-methyl-benzo[d]isoxazol-3-yl)-benzamid,
[123] 3-Methyl-N-(5-methyl-benzo[d]isoxazol-3-yl)-benzamid,
[124] 4-tert-Butyf-N-(5-methyl-benzo[d]isoxazol-3-yl)-benzamid,
[125] 2-Methyl-N-(5-methyl-benzo[d]isoxazol-3-yl)-benzamid,
[126] 2,3-Difluoro-N-(5-methyl-benzo[d]isoxazol-3-yl)-benzamid,
[127] 2,4-Difluoro-N-(5-methyl-benzo[d]isoxazol-3-yl)-benzamid,
[128] 2-Chloro-N-(5-methyl-benzo[d]isoxazol-3-yl)-nicotinamid,
[129] Cyclopropancarbonsäure-(5-fluoro-benzo[d]isoxazol-3-yl)-amid,
[130] N-(5-Fluoro-benzo[d]isoxazol-3-yl)-3,3-dimethyl-butyramid,
[131] 2,4-Difluoro-N-(5-fluoro-benzo[d]isoxazol-3-yl)-benzamid,
[132] 2,5-Dimethyl-furan-3-carbonsäure-(5-fluoro-benzo[d]isoxazol-3-yl)-amid,
[133] N-(5-Bromo-benzo[d]isoxazol-3-yl)-2,3-difluoro-benzamid,
[134] 2,5-Dimethyl-furan-3-carbonsäure-(5-bromo-benzo[d]isoxazol-3-yl)-amid,
[135] N-(5-Bromo-benzo[d]isoxazol-3-yl)-2-methyl-butyramid,
[136] N-(5-Bromo-benzo[d]isoxazol-3-yl)-2,6-difluoro-benzamid,
[137] N-(5-Bromo-benzo[d]isoxazol-3-yl)-3,4-dimethoxy-benzamid,
[138] N-(5-Bromo-benzo[d]isoxazol-3-yl)-4-methyl-benzamid,
[139] N-(5-Bromo-benzo[d]isoxazol-3-yl)-2,6-dimethoxy-benzamid,
[140] 2-Bromo-N-(5-bromo-benzo[d]isoxazol-3-yl)-benzamid,
[141] N-(5-Bromo-benzo[d]isoxazol-3-yl)-5-fluoro-2-trifluoromethyl-benzamid,
[142] N-(5-Bromo-benzo[d]isoxazol-3-yl)-3-methoxy-benzamid,
[143] 3-Methyl-N-(5-methyl-benzo[d]isoxazol-3-yl)-benzamid,
[144] 4-Cyano-N-(5-methyl-benzo[d]isoxazol-3-yl)-benzamid,
[145] N-(5-Methyl-benzo[d]isoxazol-3-yl)-2-phenyl-butyramid,
[146] 2-Methyl-pentansäure-(5-methyl-benzo[d]isoxazol-3-yl)-amid,
[147] N-(4-Fluoro-benzo[d]isoxazol-3-yl)-benzamid,
[148] 4-Chloro-N-(4-fluoro-benzo[d]isoxazol-3-yl)-benzamid,
[149] 2-Fluoro-N-(4-fluoro-benzo[d]isoxazol-3-yl)-benzamid,
[150] Adamantan-1-carbonsäure-(4-fluoro-benzo[d]isoxazol-3-yl)-amid,
[151] Adamantan-1-carbonsäure-(4-chloro-benzo[d]isoxazol-3-yl)-amid,
[152] N-(4-Chloro-benzo[d]isoxazol-3-yl)-benzamid,
[153] N-(4-Chloro-benzo[d]isoxazol-3-yl)-3-methyl-benzamid,
[154] N-(4-Chloro-benzo[d]isoxazol-3-yl)-2-methyl-butyramid,
[155] N-(4-Chloro-benzo[d]isoxazol-3-yl)-3,3-dimethyl-butyramid,
[156] N-(4-Methoxy-benzo[d]isoxazol-3-yl)-2-methyl-benzamid,
[157] 2-Chloro-N-(4-methoxy-benzo[d]isoxazol-3-yl)-benzamid,
[158] N-(4-Methoxy-benzo[d]isoxazol-3-yl)-2-trifluoromethyl-benzamid,
[159] 2-Ethyl-hexansäure-(4-methoxy-benzo[d]isoxazol-3-yl)-amid,
[160] N-(4-Methoxy-benzo[d]isoxazol-3-yl)-2-methyl-butyramid,
[161] 2-Methyl-pentansäure-(4-methoxy-benzo[d]isoxazol-3-yl)-amid,
[162] Cyclopropancarbonsäure-[4-(2,2,2-trifluoro-ethoxy)-benzo[d]isoxazol-3-yl]-amid;
[163] 2-Methyl-N-[4-(2,2,2-trifluoro-ethoxy)-benzo[d]isoxazol-3-yl]-butyramid,
[164] 3,3-Dimethyl-N-[4-(2,2,2-trifluoro-ethoxy)-benzo[d]isoxazol-3-yl]-butyramid,
[165] Cyclohexancarbonsäure-[4-(2,2,2-trifluoro-ethoxy)-benzo[d]isoxazol-3-yl]-amid,
[166] 2,5-Dimethyl-furan-3-carbonsäure-[4-(2,2,2-trifluoro-ethoxy)-benzo[d]isoxazol-3-yl]-amid,
[167] N-[4-(4-Methyl-benzyloxy)-benzo[d]isoxazol-3-yl]-3-nitro-benzamid,
[168] N-[4-(4-Methyl-benzyloxy)-benzo[d]isoxazol-3-yl]-4-propyl-benzamid,
[169] N-(4-Dimethylamino-benzo[d]isoxazol-3-yl)-3-fluoro-5-trifluoromethyl-benzamid,
[170] 3,4-Dichloro-N-(4-dimethylamino-benzo[d]isoxazol-3-yl)-benzamid,
[171] N-(4-Dimethylamino-benzo[d]isoxazol-3-yl)-3-nitro-benzamid,
[172] Adamantan-1-carbonsäure-(4-dimethylamino-benzo[d]isoxazol-3-yl)-amid,
[173] Benzo[b]thiophen-2-carbonsäure-(4-dimethy)amino-benzo[d]isoxazol-3-yl)-amid.
[174] N-(4-Dimethylamino-benzo[d]isoxazol-3-yl)-3,3-dimethyl-butyramid.
[176] N-(6-Amino-4,5,7-trifluoro-benzo[d]isoxazol-3-yl)-2,6-difluoro-benzamid
und
[177] N-(6-Amino-4,5,7-trifluoro-benzo[d]isoxazol-3-yl)-2,4-difluoro-benzamid.
jeweils ggf. in Form eines ihrer reinen Stereoisomeren, insbesondere Enantiomeren oder Diastereomeren oder Rotameren, ihrer Racemate oder in. Form einer Mischung von Stereoisomeren, insbesondere der Enantiomeren und/oder Diastereomeren und/oder Rotameren, in einem beliebigen Mischungsverhältnis, oder jeweils in Form entsprechender Salze, oder jeweils in Form entsprechender Solvate;
sowie ggf. einen oder mehrere physiologisch verträgliche Hilfsstoffe.

2. Arzneimittel gemäß Anspruch 1 zur Prophylaxe und/oder Behandlung von Schmerz, vorzugsweise von Schmerz ausgewählt aus der Gruppe bestehend aus akutem Schmerz, chronischem Schmerz, visceralem Schmerz und neuropathischem Schmerz.

3. Arzneimittel gemäß Anspruch 1 zur
Prophylaxe und/oder Behandlung von einer oder mehrerer Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie;Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Narkolepsie; Diarrhoe; Gastritis; Magengeschwür; Pruritus; Angstzuständen; Panikattacken; Schizophrenie; cerebralen Ischämien; Muskelspasmen; Krämpfen; Magen-Ösaphagus-Reflux-Syndrom; Alkohol-und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme: zur Modulation der Bewegungsaktivität, zur Regulation des kardiovaskulären Systemes; zur Lokalanästhesie; zur Vigilanzsteigerung; zur Libidosteigerung; zur Diurese und/oder zur Antinatriurese.

4. Verwendung wenigstens einer Verbindung gemäß Anspruch 1 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, visceralem Schmerz und neurophatischem Schmerz.

5. Verwendung wenigstens einer Verbindung gemäß Anspruch 1 zur Herstellung eines
Arzneimittels zur Prophylaxe und/oder Behandlung von einer oder mehrerer Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Narkolepsie; Diarrhoe; Gastritis; Magengeschwür; Pruritus; Angstzuständen; Panikattacken; Schizophrenie; cerebralen lschämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Regulation des kariovaskulären Systems; zur Lokalanästhesie; zur Vigilanzsteigerung; zur Libidosteigerung; zur Diurese und/oder zur Antinatriurese.

6. Verbindung, ausgewählt aus der Gruppe bestehend aus
[1] Thiophen-2-carbonsäure-benzo[d]isoxazol-3-ylamid,
[3] Naphthalen-2-carbonsäure-benzo[d]isoxazol-3-ylamid ,
[4] Adamantan-2-carbonsäurebenzo[d]isoxazol-3-ylamid,
[5] Cyclohexancarbonsäure-benzo[d]isoxazol-3-ylamid,
[6] N-Benzo[d]isoxazol-3-yl-2,2-dimethyl-propionamid,
[7] N-(4-Methoxy-benzo[d]isoxazol-3-yl)-2,2-diphenyl-acetamid,
[8] Cyclopropancarbonsäure-(5-bromo-benzo[d]isoxazol-3-yl)-amid,
[9] 3,5-Dichloro-N-(6-fluoro-benzo[d]isoxazol-3-yl)-benzamid,
[10] N-(4-Amino-benzo[d]isoxazol-3-yl)-4-nitro-benzamid,
[11] Naphthalin-1-carbonsäure-(4-amino-benzo[d]isoxazol-3-yl)-amid,
[12] Benzo[b]thiophen-2-carbonsäure-(4-fluoro-benzo[d]isoxazol-3-yl)-amid,
[13] N-Benzo[d]isoxazol-3-yl-2-trifluoromethyl-benzamid,
[14] N-Benzo[d]isoxazol-3-yl-3,4-dichloro-benzamid,
[15] N-Benzo[d]isoxazol-3-yl-2,3-difluoro-benzamid,
[16] N-(4-Dimethylamino-benzo[d]isoxazol-3-yl)-3-fluoro-benzamid,
[17] Thiophen-2-carbonsäure-(6-fluoro-benzo[d]isoxazol-3-yl)-amid,
[18] N-(6-Fluoro-benzo[d]isoxazol-3-yl)-2-methyl-butyramid,
[19] N-(6-Chloro-benzo[d]isoxazol-3-yl)-2-fluoro-3-trifluoromethyl-benzamid,
[20] N-(6-Chloro-benzo[d]isoxazol-3-yl)-3-methoxy-benzamid,
[21] N-(6-Bromo-benzo[d]isoxazol-3-yl)-4-tert-butyl-benzamid,
[23] N-(6-Bromo-benzo[d]isoxazol-3-yl)-2-trifluoromethyl-benzamid,
[24] Adamantan-2-carbonsäure-(6-bromo-benzo[d]isoxazol-3-yl)-amid,
[25] N-(6-Bromo-benzo[d]isoxazol-3-yl)-2-methyl-butyramid,
[26] N-(5-Fluoro-benzo[d]isoxazol-3-yl)-2-trifluoromethyl-benzamid,
[27] 3,4-Dichloro-N-(5-fluoro-benzo[d]isoxazol-3-yl)-benzamid,
[29] N-(5-Bromo-benzo[d]isoxazol-3-yl)-2-fluoro-5-trifluoromethyl-benzamid,
[30] N-(5-Bromo-benzo[d]isoxazol-3-yl)-2,4-difluoro-benzamid,
[31] N-(5-Methyl-benzo[d]isoxazol-3-yl)-2-trifluoromethyl-benzamid,
[32] 3-Fluoro-N-(5-methyl-benzo[d]isoxazol-3-yl)-benzamid,
[33] N-(4-Methoxy-benzo[d]isoxazol-3-yl)-3,5-bis-trifluoromethyl-benzamid,
[34] 3,5-Difluoro-N-(4-methoxy-benzo[d]isoxazol-3-yl)-benzamid,
[35] N-(4-Dimethylamino-benzo[d]isoxazol-3-yl)-3,5-bis-trifluoromethyl-benzamid,
[36] 2-Bromo-N-[4-(2,2,2-trifluoro-ethoxy)-benzo[d]isoxazol-3-yl]-benzamid,
[37] 3-Chloro-N-[4-(2,2,2-trifluoro-ethoxy)-benzo[d]isoxazol-3-yl]-benzamid,
[38] 4-tert-Butyl-N-[4-(2,2,2-trifluoro-ethoxy)-benzo[d]isoxazol-3-yl]-benzamid,
[39] 2-Methoxy-N-[4-(2,2,2-trifluoro-ethoxy)-benzo[d]isoxazol-3-yl]-benzamid,
[40] N-(6-Chloro-benzo[d]isoxazol-3-yl)-4-iodo-benzamid,
[41] Naphthalen-2-carbonsäure-(6-chloro-benzo[d]isoxazol-3-yl)-amid,
[42] N-(6-Chloro-benzo[d]isoxazol-3-yl)-3,4-difluoro-benzamid,
[43] N-(6-Bromo-benzo[d]isoxazol-3-yl)-4-fluoro-2-trifluoromethyl-benzamid,
[44] 2,4-Dichloro-N-(6-bromo-benzo[d]isoxazol-3-yl)-5-fluoro-benzamid,
[45] N-(6-Bromo-benzo[d]isoxazol-3-yl)-3-fluoro-4-trifluoromethyl-benzamid,
[46] N-(6-Bromo-benzo[d]isoxazol-3-yl)-3-fluoro-5-trifluoromethyl-benzamid,
[47] N-(6-Bromo-benzo[d]isoxazol-3-yl)-2,3,4-trifluoro-benzamid,
[48] N-(6-Bromo-benzo[d]isoxazol-3-yl)-4-propyl-benzamid,
[49] N-(6-Bromo-benzo[d]isoxazol-3-yl)-3,4-difluoro-benzamid,
[50] Furan-2-carbonsäure-(5-bromo-benzo[d]isoxazol-3-yl)-amid,
[51] 4-Fluoro-N-(7-fluoro-benzo[d]isoxazol-3-yl)-2-trifluoromethyl-benzamid,
[52] 2,4-Dichloro-5-fluoro-N-(7-fluoro-benzo[d]isoxazol-3-yl)-benzamid,
[53] 3-Fluoro-N-(7-fluoro-benzo[d]isoxazol-3-yl)-4-trifluoromethyl-benzamid,
[54] 2,3,4-Trifluoro-N-(7-fluoro-benzo[d]isoxazol-3-yl)-benzamid,
[55] N-(7-Fluoro-benzo[d]isoxazol-3-yl)-4-iodo-benzamid,
[56] 2-Chloro-N-(7-fluoro-benzo[d]isoxazol-3-yl)-nicotinamid,
[57] Naphthalen-2-carbonsäure-(7-fluoro-benzo[d]isoxazol-3-yl)-amid,
[58] N-(4-Fluoro-benzo[d]isoxazol-3-yl)-74-propyl-benzamid,
[59] 3,4-Difluoro-N-(7-fluoro-benzo[d]isoxazol-3-yl)-benzamid,
[60] 2-Fluoro-N-(7-fluoro-benzo[d]isoxazol-3-yl)-3-trifluoromethyl-benzamid,
[61] N-(7-Fluoro-benzo[d]isoxazol-3-yl)-3-methoxy-benzamid,
[62] N-(7-Fluoro-benzo[d]isoxazol-3-yl)-2,2-diphenyl-acetamid,
[63] Furan-2-carbonsäure-(7-fluoro-benzo[d]isoxazol-3-yl)-amid,
[64] 2,4-Dichloro-N-(4-dimethylamino-benzo[d]isoxazol-3-yl)-5-fluoro-benzamid,
[65] N-(4-Dimethylamino-benzo[d]isoxazol-3-yl)-2,3,4-trifluoro-benzamid,
[66] 2-Chloro-N-(4-dimethylamino-benzo[d]isoxazol-3-yl)-nicotinamid,
[67] Naphthalen-2-carbonsäure-(4-dimethylamino-benzo[d]isoxazol-3-yl)-amid,
[68] N-(4-Dimethylamino-benzo[d]isoxazol-3-yl)-4-propyl-benzamid,
[69] N-(4-Dimethylamino-benzo[d]isoxazol-3-yl)-3,4-difluoro-benzamid,
[70] N-(4-Dimethylamino-benzo[d]isoxazol-3-yl)-2-fluoro-3-trifluoromethyl-benzamid,
[71] N-(4-Dimethylamino-benzo[d]isoxazol-3-yl)-3-methoxy-benzamid,
[72] N-(4-Dimethylamino-benzo[d]isoxazol-3-yl)-2,2-dimethyl-propionamid,
[73] Cyclohexancarbonsäure-(4-dimethyl amino-benzo[d]isoxazol-3-yl)-amid,
[74] N-(4-Dimethylamino-benzo[d]isoxazol-3-yl)-2,2-diphenyl-acetamid,
[75] Furan-2-carbonsäure-(4-dimethylamino-benzo[d]isoxazol-3-yl)-amid,
[76] N-(4-Dimethylamino-benzo[d]isoxazol-3-yl)-2,2,3,3,4,4,4-heptafluoro-butyramid,
[77] 4-Fluoro-N-[4-(2,2,2-trifluoro-ethoxy)-benzo[d]isoxazol-3-yl]-2-trifluoromethyl-benzamid,
[78] 2,4-Dichloro-5-fluoro-N-[4-(2,2,2-trifluoro-ethoxy)-benzo[d]isoxazol-3-yl]-benzamid,
[79] 3-Fluoro-N-[4-(2,2,2-trifluoro-ethoxy)-benzo[d]isoxazol-3-yl]-4-trifluoromethyl-benzamid,
[80] 2,3,4-Trifluoro-N-[4-(2,2,2-trifluoro-ethoxy)-benzo[d]isoxazol-3-yl]-benzamid,
[81] Naphthalen-2-carbonsäure-[4-(2,2,2-trifluoro-ethoxy)-benzo[d]isoxazol-3-yl]-amid,
[82] 3,4-Difluoro-N-[4-(2,2,2-trifluoro-ethoxy)-benzo[d]isoxazol-3-yl]-benzamid,
[83] 2-Fluoro-N-[4-(2,2,2-trifluoro-ethoxy)=benzo[d]isoxazol-3-yl]-3-trifluoromethyl-benzamid,
[85] 2-Ethyl-hexansäure-benzo[d]isoxazol-3-ylamid,
[86] N-Benzo[d]isoxazol-3-yl-2-methyl-butyramid,
[88] N-Benzo[d]isoxazol-3-yl-3-fluoro-4-trifluoromethyl-benzamid,
[89] N-Benzo[d]isoxazol-3-yl-2,3,6-trifluoro-benzamid,
[90] N-Benzo[d]isoxazol-3-yl-nicotinamid,
[91] 5-Methyl-isoxazol-3-carbonsäure-benzo[d]isoxazol-3-ylamid,
[92] Benzo[b]thiophen-3-carbonsäure-benzo[d]isoxazol-3-ylamid,
[94] 4-tert-Butyl-N-(7-fluoro-benzo[d]isoxazol-3-yl)-benzamid,
[96] 4-Fluoro-N-(7-fluoro-benzo[d]isoxazol-3-yl)-benzamid,
[100] 3,5-Difluoro-N-(6-fluoro-benzo[d]isoxazol-3-yl)-benzamid,
[101] Naphthalen-1-carbonsäure-(6-fluoro-benzo[d]isoxazol-3-yl)-amid,
[102] Adamantan-1-carbonsäureg-(6-fluoro-benzo[d]isoxazol-3-yl)-amid,
[104] 4-tert-Butyl-N-(5-fluoro-benzo[d]isoxazol-3-yl)-benzamid,
[105] 2,4-Difluoro-N-(5-fluoro-benzo[d]isoxazol-3-yl)-benzamid,
[106] Naphthalen-2-carbonsäure-(5-fluoro-benzo[d]isoxazol-3-yl)-amid,
[107] N-(5-Flüoro-benzo[d]isoxazol-3-yl)-4-propyl-benzamid,
[109] 4-tert-Butyl-N-(6-chloro-benzo[d]isoxazol-3-yl)-benzamid,
[110] N-(6-Chloro-benzo[d]isoxazol-3-yl)-4-fluoro-2-trifluoromethyl-benzamid,
[111]2,4-Dichloro-N-(6-chloro-benzo[d]isoxazol-3-yl)-5-fluoro-benzamid,
[112] 2-Ethyl-hexansäure-(6-chloro-benzo[d]isoxazol-3-yl)-amid,
[113] N-(6-Chloro-benzo[d]isoxazol-3-yl)-2-methyl-butyramid,
[115] N-(6-Bromo-benzo[d]isoxazol-3-yl)-3,4-dichloro-benzamid,
[116] Thiophen-2-carbonsäure-(6-bromo-benzo[d]isoxazol-3-yl)-amid,
[117] N-(6-Bromo-benzo[d]isoxazol-3-yl)-3,3-dimethyl-butyramid,
[119] 4-Bromo-N-(5-methyl-benzo[d]isoxazol-3-yl)-benzamid,
[121] 4-Fluoro-N-(5-methyl-benzo[d]isoxazol-3-yl)-benzamid,
[123] 3-Methyl-N-(5-methyl-benzo[d]isoxazol-3-yl)-benzamid,
[124] 4-tert-Butyl-N-(5-methyl-benzo[d]isoxazol-3-yl)-benzamid,
[126] 2,3-Difluoro-N-(5-methyl-benzo[d]isoxazol-3-yl)-benzamid,
[127] 2,4-Difluoro-N-(5-methyl-benzo[d]isoxazol-3-yl)-benzamid,
[128] 2-Chloro-N-(5-methyl-benzo[d]isoxazol-3-yl)-nicotinamid,
[129] Cyclopropancarbonsäure-(5-fluoro-benzo[d]isoxazol-3-yl)-amid,
[130] N-(5-Fluoro-benzo[d]isoxazol-3-yl)-3,3-dimethyl-butyramid,
[131] 2,4-Difluoro-N-(5-fluoro-benzo[d]isoxazol-3-yl)-benzamid,
[132] 2,5-Dimethyl-furan-3-carbonsäure-(5-fluoro-benzo[d]isoxazol-3-yl)-amid,
[133] N-(5-Bromo-benzo[d]isoxazol-3-yl)-2,3-difluoro-benzamid,
[134] 2,5-Dimethyl-furan-3-carbonsäure-(5-bromo-benzo[d]isoxazol-3-yl)-amid,
[135] N-(5-Bromo-benzo[d]isoxazol-3-yl)-2-methyl-butyramid,
[137] N-(5-Bromo-benzo[d]isoxazol-3-yl)-3,4-dimethoxy-benzamid,
[138] N-(5-Bromo-benzo[d]isoxazol-3-yl)-4-methyl-benzamid,
[141] N-(5-Bromo-benzo[d]isoxazol-3-yl)-fluoro-2-trifluoromethyl-benzamid,
[142] N-(5-Bromo-benzo[d]isoxazol-3-yl)-3-methoxy-benzamid,
[143] 3-Methyl-N-(5-methyl-benzo[d]isoxazol-3-yl)-benzamid,
[144] 4-Cyano-N-(5-methyl-benzo[d]isoxazol-3-yl)-benzamid,
[145] N-(5-Methyl-benzo[d]isoxazol-3-yl)-2-phenyl-butyramid,
[146] 2-Methyl-pentansäure-(5-methyl-benzo[d]isoxazol-3-yl)-amid,
[147] 4-Chloro-N-(4-fluoro-benzo[d]isoxazol-3-yl)-benzamid,
[150] Adamantan-1-carbonsäure-(4-fluoro-benzo[d]isoxazol-3-yl)-amid,
[151] Adamantan-1-carbonsäure-(4-chloro-benzo[d]isoxazol-3-yl)-amid,
[153] N-(4-Chloro-benzo[d]isoxazol-3-yl)-3-methyl-benzamid,
[154] N-(4-Chloro-benzo[d]isoxazol-3-yl)-2-methyl-butyramid,
[155] N-(4-Chloro-benzo[d]isoxazol-3-yl)-3,3-dimethyl-butyramid,
[156] N-(4-Methoxy-benzo[d]isoxazol-3-yl)-2-methyl-benzamid,
[157] 2-Chloro-N-(4-methoxy-benzo[d]isoxazol-3-yl)-benzamid,
[158] N-(4-Methoxy-benzo[d]isoxazol-3-yl)-2-trifluoromethyl-benzamid,
[159] 2-Ethyl-hexansäure-(4-methoxy-benzo[d]isoxazol-3-yl)-amid,
[160] N-(4-Methoxy-benzo[d]isoxazol-3-yl)-2-methyl-butyramid
[161] 2-Methyl-pentansäure-(4-methoxy-benzo[d]isoxazol-3-yl)-amid,
[162] Cyclopropancarbonsäure-[4-(2,2,2-trifluoro-ethoxy)-benzo[d]isoxazol-3-yl]-amid,
[163] 2-Methyl-N-[4-(2,2,2-trifluoro-ethoxy)-benzo[d]isoxazol-3-yl]-butyramid,
[164] 3,3-Dimethyl-N-[4-(2,2,2-trifluoro-ethoxy)-benzo[d]isoxazol-3-yl]-butyramid,
[165] Cyclohexancarbonsäure-[4-(2,2,2-trifluoro-ethoxy)-benzo[d]isoxazol-3-yl]-amid,
[166] 2,5-Dimethyl-furan-3-carbonsäure-[4-(2,2,2-trifluoro-ethoxy)-benzo[d]isoxazol-3-yl]-amid,
[167] N-[4-(4-Methyl-benzyloxy)-benzo[d]isoxazol-3-yl]-3-nitro-benzamid,
[168] N-[4-(4-Methyl-benzyloxy)-benzo[d]isoxazol-3-yl]-4-propyl-benzamid,
[169] N-(4-Dimethylamino-benzo[d]isoxazol-3-yl)-3-fluoro-5-trifluoromethyl-benzamid,
[170] 3,4-Dichloro-N-(4-dimethylamino-benzo[d]isoxazol-3-yl)-benzamid,
[171] N-(4-Dimethylamino-benzo[d]isoxazol-3-yl)-3-nitro-benzamid,
[172] Adamantan-1-carbonsäure-(4-dimethylamino-benzo[d]isoxazol-3-yl)-amid,
[173] Benzo[b]thiophen-2-carbonsäure-(4-dimethylamino-benzo[d]isoxazol-3-yl)-amid,
[174] N-(4-Dimethylamino-benzo[d]isoxazol-3-yl)-3,3-dimethyl-butyramid, [176] N-(6-Amino-4,5,7-trifluoro-benzo[d]isoxazol-3-yl)-2,6-difluoro-benzamid
und
[177] N-(6-Amino-4,5,7-trifluoro-benzo[d]isoxazol-3-yl)-2,4-difluoro-benzamid.
jeweils ggf. in Form eines ihrer reinen Stereoisomeren, insbesondere Enantiomeren oder Diastereomeren oder Rotameren, ihrer Racemate oder in Form einer Mischung von Stereoisomeren, insbesondere der Enantiomeren und/oder Diastereomeren und/oder Rotameren, in einem beliebigen Mischungsverhältnis, oder jeweils in Form entsprechender Salze, oder jeweils in Form entsprechender Solvate.

7. Verfahren zur Herstellung einer Verbindung gemäß Anspruch 6, **dadurch gekennzeichnet, dass** wenigstens eine Verbindung der allgemeinen Formel IIa, 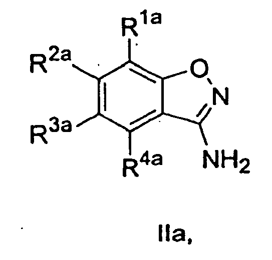 worin R^{1a} bis R^{4a} die Bedeutung wie durch die Verbindungen gemäß Anspruch 6 definiert haben,
in einem Reaktionsmedium ggf. in Gegenwart wenigstens einer Base, mit wenigstens einer Verbindung der allgemeinen Formel R^{5a}-C(=O)-X, worin R^{5a} die Bedeutung wie durch die Verbindungen gemäß Anspruch 6 definiert hat und X für eine Abgangsgruppe, vorzugsweise einen Halogen-Rest steht, besonders bevorzugt für ein Chloratom steht,
oder in einem Reaktionsmedium, in Gegenwart wenigstens eines Kupplungsreagenzes, ggf. in Gegenwart wenigstens einer Base, mit wenigstens einer Verbindung der allgemeinen Formel R^{5a}-C(=O)-OH, worin R^{5a} die Bedeutung wie durch die Verbindungen gemäß Anspruch 6 definiert hat, zu einer Verbindung gemäß Anspruch 6 umgesetzt wird und diese ggf. isoliert und/oder gereinigt wird.

8. Arzneimittel enthaltend wenigstens eine Verbindung gemäß Anspruch 6 sowie ggf. einen oder mehrere physiologisch verträgliche Hilfsstoffe.

9. Arzneimittel gemäß Anspruch 8 zur Prophylaxe und/oder Behandlung von Schmerz, vorzugsweise Schmerz ausgewählt aus der Gruppe bestehend aus akutem Schmerz, chronischem Schmerz, visceralem Schmerz und neuropathischem Schmerz.

10. Arzneimittel gemäß Anspruch 8 zur Prophylaxe und/oder Behandlung von einer oder mehrerer Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie;Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Narkolepsie; Diarrhoe; Gastritis; Magengeschwür, Pruritus; Angstzuständen; Panikattacken; Schizophrenie; cerebralen lschämien; Muskelspasmen; Krämpfen; Magen-Ösaphagus-Reflux-Syndrom; Alkohol-und/oder Drogen-, vorzugsweise Nikotin- oder Kokain-, und/oder Medikamentenmißbrauch: Alkohol- und/oder Drogen-, vorzugsweise Nikotin-oder Kokain-, und/oder Medikamentenabhängigkeit, vorzugsweise zur Prophylaxe und/oder Verminderung von Entzugserscheinungen bei Alkohol-und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Regulation des kardiovaskulären Systems; zur Lokalanästhesie; zur Vigilanzsteigerung; zur Libidosteigerung; zur Diurese und/oder zur Antinatriurese.

11. Verwendung wenigstens einer Verbindung gemäß Anspruch 6 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, visceralem Schmerz und neuropathischem Schmerz.

12. Verwendung wenigstens einer Verbindung gemäß Anspruch 6 zur Herstellung eines
Arzneimittels zur Prophylaxe und/oder Behandlung von einer oder mehrerer Erkrankungen ausgewählt aus der Gruppe bestehend aus Störungen der Nahrungsmittelaufnahme, vorzugsweise ausgewählt aus der Gruppe bestehend aus Bulimie, Anorexie, Fettleibigkeit und Kachexie; Migräne; chronische paroxysmale Hemikranie; Depressionen; Harninkontinenz; Husten; Asthma; Glaukom; Tinitus; Entzündungen; neurodegenerativen Erkrankungen, vorzugsweise ausgewählt aus der Gruppe bestehend aus Morbus Parkinson, Morbus Huntington, Morbus Alzheimer und multipler Sklerose; kognitiven Dysfunktionen, vorzugsweise Gedächtnisstörungen; kognitiven Mangelzuständen (attention deficit syndrom, ADS); Epilepsie; Narkolepsie; Diarrhoe; Gastritis; Magengeschwür, Pruritus; Angstzuständen; Panikattacken; Schizophrenie; cerebralen Ischämien; Muskelspasmen; Krämpfen; Magen-Ösaphagus-Reflux-Syndrom; Alkohol- und/oder Drogen-, vorzugsweise Nikotin- oder Kokain-, und/oder Medikamentenmißbrauch; Alkohol- und/oder Drogen-, vorzugsweise Nikotin- oder Kokain-, und/oder Medikamentenabhängigkeit, vorzugsweise zur Prophylaxe und/oder Verminderung von Entzugserscheinungen bei Alkohol- und/oder Drogen-, vorzugsweise Nikotin- oder Kokain-, und/oder Medikamentenabhängigkeit; zur Prophylaxe und/oder Verminderung einer Toleranzentwicklung gegenüber Medikamenten und/oder Drogen, insbesondere Medikamenten auf Basis von Opioiden; zur Regulation der Nahrungsaufnahme; zur Modulation der Bewegungsaktivität, zur Regulation des kardiovaskulären Systems; zur Lokalanästhesie; zur Vigilanzsteigerung; zur Libidosteigerung; zur Diurese und/oder zur Antinatriurese.

## Claims

1. Medicament comprising at least one compound selected from the group consisting of
[1] thiophene-2-benzo[d]isoxazol-3-ylcarboxamide,
[3] naphthalene-2-benzo[d]isoxazol-3-ylcarboxamide,
[4] adamantane-2-benzo[d]isoxazol-3-ylcarboxamide,
[5] cyclohexane-benzo[d]isoxazol-3-ylcarboxamide,
[6] N-benzo[d]isoxazol-3-yl-2,2-dimethylpropionamide,
[7] N-(4-methoxybenzo[d]isoxazol-3-yl)-2,2-diphenylacetamide,
[8] cyclopropane(5-bromobenzo[d]isoxazol-3-yl)carboxamide,
[91] 3,5-dichloro-N-(6-fluorobenzo[d]isoxazol-3-yl)benzamide,
[10] N-(4-aminobenzo[d]isoxazol-3-yl)-4-nitrobenzamide,
[11] naphthalene-1-(4-aminobenzo[d]isoxazol-3-yl)carboxamide,
[12] benzo[b]thiophene-2-(4-fluorobenzo[d]isoxazol-3-yl)carboxamide,
[13] N-benzo[d]isoxazol-3-yl-2-trifluoromethylbenzamide,
[14] N-benzo[d]isoxazol-3-yl-3,4-dichlorobenzamide
[15] N-benzo[d]isoxazol-3-yl-2, 3-difluorobenzamide,
[16] N-(4-dimethylaminobenzo[d]isoxazol-3-yl)-3-fluorobenzamide,
[17] thiophene-2-(6-fluorobenzo[d]isoxazol-3-yl)carboxamide,
[18] N-(6-fluorobenzo[d]isoxazol-3-yl)-2-methylbutyramide,
[19] N-(6-chlorobenzo[d]isoxazol-3-yl)-2-fluoro-3-trifluoromethylbenzamide,
[20] N-(6-chlorobenzo[d]isoxazol-3-yl)-3-methoxybenzamide,
[21] N-(6-bromobenzo[d]isoxazol-3-yl)-4-tert-butylbenzamide,
[22] N-(6-bromobenzo[d]isoxazol-3-yl)-2-methoxybenzamide,
[23] N-(6-bromobenzo[d]isoxazol-3-yl)-2-trifluoromethylbenzamide,
[24] adamantane-2-(6-hromobenzo[d]isoxazol-3-yl)carboxamide,
[25] N-(6-bromobenzo[d]isoxazol-3-yl)-2-methylbutyramide,
[26] N-(5-fluorobenzo[d]isoxazol-3-yl)-2-trifluoromethylbenzamide,
[27] 3,4-dichloro-N- (5-fluorobenzo[d]isoxazol-3-yl)benzamide,
[28] N-(5-fluorobenzo[d]isoxazol-3-yl)-2-methylbenzamide,
[29] N-(5-bromobenzo[d]isoxazol-3-yl)-2-fluoro-5-trifluoromethylbenzamide,
[30] N-(5-bromobenzo[d]isoxazol-3-yl)-2,4-difluorobenzamide,
[31] N-(5-methylbenzo[d]isoxazol-3-yl)-2-trifluoromethylbenzamide,
[32] 3-fluoro-N-(5-methylbenzo[d]isoxazol-3-yl)benzamide,
[33] N-(4-methoxybenzo[d]isoxazol-3-yl)-3,5-bis-trifluoromethylbenzamide,
[34] 3,5-difluoro-N-(4-methoxybenzo[d]isoxazol-3-yl)benzamide,
[35] N-(4-dimethylaminobenzo[d]isoxazol-3-yl)-3,5-bis-trifluoromethylbenzamide,
[36] 2-bromo-N-[4-(2,2,2-trifluoroethoxy)benzo[d]isoxazol-3-yl]benzamide,
[37] 3-chloro-N-[4-(2,2,2-trifluoroethoxy)benzo[d]isoxazol-3-yl]benzamide,
[38] 4-tert-butyl-N-[4-(2,2,2-trifluoroethoxy)benzo[d]isoxazol-3-yl]benzamide,
[39] 2-methoxy-N-[4-(2,2,2-trifluoroethoxy)benzo[d]isoxazol-3-yl]benzamide,
[40] N-(5-chlorobenzo[d]isoxazol-3-yl)-4-iodobenzamide,
[41] naphthalene-2-(6-chlorobenzo[d]isoxazol-3-yl)carboxamide,
[42] N-(6-chlorobenzo[d]isoxazol-3-yl)-3,4-difluorobenzamide,
[43] N-(6-bromobenzo[d]isoxazol-3-yl)-4-fluoro-2-trifluoromethylbenzamide,
[44] 2,4-dichloro-N-(6-bromobenzo[d]isoxazol-3-yl)-5-fluorobenzamide,
[45] N-(6-bromobenzo[d]isoxazol-3-yl)-3-fluoro-4-trifluoromethylbenzamide,
[45] N-(5-bromobenzo[d]isoxazol-3-yl)-3-fluoro-5-trifluoromethylbenzamide,
[47] N-(6-bromobenzo[d]isoxazol-3-yl)-2,3,4-trifluorobenzamide,
[48] N-(5-bromobenzo[d]isoxazol-3-yl)-4-propylbenzamide,
[49] N-(6-bromobenzo[d]isoxazol-3-yl)-3,4-difluorobenzamide,
[50] furan-2-(5-bromobenzo[d]isoxazol-3-yl)carboxamide.
[51] 4-fluoro-N-(7-fluorobenzo[d]isoxazol-3-yl)-2-trifluoromethylbenzamide,
[52] 2,4-dichloro-5-fluoro-N-(7-fluorobenzo[d]isoxazol-3-yl)benzamide,
[53] 3-fluoro-N-(7-fluorobenzo[d]isoxazol-3-yl)-4-trifluoromethylbenzamide,
[54] 2,3,4-trifluoro-N-(7-fluorobenzo[d]ispxazol-3-yl)benzamide,
[55] N-(7-fluorobenzo[d]isoxazol-3-yl)-4-iodobenzamide,
[56] 2-chloro-N-(7-fluorobenzo[d]isoxazol-3-yl)nicotinamide,
[57] naphthalene-2-(7-fluorobenzo[d]isoxazol-3-yl)carboxamide,
[58] N-(7-fluorobenzo[d]isoxazol-3-yl)-4-propylbenzamide,
[59] 3,4-difluoro-N-(7-fluorobenzo[d]isoxazol-3-yl)benzamide,
[60] 2-fluoro-N-(7-fluorobenzo[d]isoxazol-3-yl)-3-trifluoromethylbenzamide,
[61] N-(7-fluorobenzo[d]isoxazol-3-yl)-3-methoxybenzamide,
[62] N-(7-fluorobenzo[d]isoxazol-3-yl)-2,2-diphenylacetamide,
[63] furan-2-(7-fluorobenzo[d]isoxazol-3-yl)carboxamide,
[64] 2,4-dichloro-N-(4-dimethylaminobenzo[d]isoxazol-3-y1)-5-fluorobenzamide,
[65] N-(4-dimethylaminobenzo[d]isoxazol-3-yl)-2,3,4-trifluorobenzamide,
[66] 2-chloro-N-(4-dimethylaminobenzo[d]isoxazol-3-yl)nicotinamide,
[67] naphthalene-2-(4-dimethylaminobenzo[d]isoxazol-3-y1)carboxamide,
[68] N-(4-dimethylaminobenzo[d]isoxazol-3-yl)-4-propylbenzamide,
[69] N-(4-dimethylaminobenzo[d]isoxazol-3-yl)-3,4-difluorobenzamide,
[70] N-(4-dimethylaminobenzo[d]isoxazol-3-yl)-2-fluoro-3-trifluoromethylbenzamide,
[71] N-(4-dimethylaminobenzo[d]isoxazol-3-yl)-3-methoxybenzamide,
[72] N-[4-dimethylaminobenzo[d]isoxazol-3-yl)-2,2-dimethylpropionamide,
[73] cyclohexane-(4-dimethylaminobenzo [d] isoxazol-3-yl)carboxamide,
[74] N-(4-dimethylaminobenzo [d] isoxazol-3-yl)-2,2-diphenylacetamide,
[75] furan-2-(4-dimethylaminobenzo [d] isoxazol-3-yl) carboxamide,
[76] N-(4-dimethylaminobenzo [d] isoxazol-3-yl)-2,2,3,3,4,4,4-heptafluorobutyramide,
[77] 4-fluoro-N-[4-(2,2,2-trifluoroethoxy)benzo[d]isoxazol-3-yl]-2-trifluoromethylbenzamide,
[78] 2,4-dichloro-5-fluoro-N-[4-(2,2,2-trifluoroethoxy)benzo[d]isoxazol-3-yl]benzamide,
[79] 3-fluoro-N-[4-(2,2,2-trifluoroethoxy)benzo[d]isoxazol-3-yl]-4-trifluoromethylbenzamide,
[80] 2,3,4-trifluoro-N-[4-(2,2,2-trifluoroethoxy)benzo[d]isoxazol-3-yl]benzamide,
[81] naphthalene-2-[4-(2,2,2-trifluoroethoxy)benzo[d]isoxazol-3-yl]carboxamide,
[82] 3,4-difluoro-N-[4-(2,2,2-trifluoroethoxy)benzo[d]isoxazol-3-yl]benzamide,
[83] 2-fluoro-N-[4-(2,2,2-trifluoroethoxy)benzo[d]isoxazol-3-yl]-3-trifluoromethylbenzamide,
[85] N-benzo[d]isoxazol-3-yl-2-ethylhexanamide,
[86] N-benzo[d]isoxazol-3-yl-2-methylbutyramide,
[87] N-benzo[d]isoxazol-3-yl-2,6-difluorobenzamide,
[88] N-benzo[d]isoxazol-3-yl-3-fluoro-4-trifluoromethylbenzamide,
[89] N-benzo[d]isoxazol-3-yl-2,3,6-trifluorobenzamide,
[90] N-benzo[d]isoxazol-3-ylnicotinamide,
[91] 5-methylisoxazole-3-benzo[d]isoxazol-3-ylcarboxamide,
[92] benzo[b]thiophene-3-benzo[d]isoxazol-3-ylcarboxamide,
[93] 2-chloro-N-(7-fluorobenzo[d]isoxazol-3-yl)benzamide,
[94] 4-tert-butyl-N-(7-fluorobenzo[d]isoxazol-3-yl) benzamide,
[95] N-(7-fluorobenzo[d]isoxazol-3-yl)benzamide,
[96] 4-fluoro-N-(7-fluorobenzo[d]isoxazol-3-yl)benzamide,
[97] 2-fluoro-N-(7-fluorobenzo[d] isoxazol-3-yl)benzamide,
[98] N-(7-fluorobenzo[d]isoxazol-3-yl)-2-methoxybenzamide,
[99] N-(7-fluorobenzo[d]isoxazol-3-yl)-2-methylbenzamide,
[100] 3,5-difluoro-N-(6-fluorobenzo[d]isoxazol-3-yl) benzamide,
[101] naphthalene-1-(6-fluorobenzo[d]isoxazol-3-yl)carboxamide,
[102] adamantane-1-(6-fluorobenzo[d]isoxazol-3-yl)carboxamide,
[103] 2-bromo-N-(5-fluorobenzo[d]isoxazol-3-yl)benzamide,
[104] 4-tert-butyl-N-(5-fluorobenzo[d]isoxazol-3-yl)benzamide,
[105] 2,4-difluoro-N-[5-fluorobenzo[d]isoxazol-3-yl)benzamide,
[106] naphthalene-2-(5-fluorobenzo[d]isoxazol-3-yl)carboxamide,
[107] N-(5-fluorobenzo[d]isoxazol-3-yl)-4-propylbenzamide,
[108] 2-chloro-N-(6-chlorobenzo[d]isoxazol-3-yl)benzamide,
[109] 4-tert-butyl-N-(6-chlorobenzo[d]isoxazol-3-yl)benzamide,
[110] N-(6-chlorobenzo[d]isoxazol-3-yl)-4-fluoro-2-trifluoromethylbenzamide,
[111] 2,4-dichloro-N-(6-chlorobenzo[d]isoxazol-3-yl)-5-fluorobenzamide,
[112] N-(6-chlorobenzo[d]isoxazol-3-yl)-2-ethylhexanamide,
[113] N-(6-chlorobenzo[d]isoxazol-3-yl)-2-methylbutyramide,
[114] N-(6-bromobenzo[d]isoxazol-3-yl)-2-chlorobenzamide,
[115] N-(6-bromobenzo[d] isoxazol-3-yl)-3,4-dichlorobenzamide,
[116] thiophene-2-(6-bromobenzo[d]isoxazol-3-yl)carboxamide,
[117] N-(6-bromobenzo[d]isoxazol-3-yl)-3,3-dimethylbutyramide,
[118] N-(5-methylbenzo[d]isoxazol-3-yl) benzamide,
[119] 4-bromo-N-(5-methylbenzo[d]isoxazol-3-yl)benzamide,
[120] 2-chloro-N-(5-methylbenzo[d]isoxazol-3-yl)benzamide,
[121] 4-fluoro-N-(5-methylbenzo[d]isoxazol-3-yl) benzamide,
[122] 2-fluoro-N-(5-methylbenzo[d]isoxazol-3-yl) benzamide,
[123] 3-methyl-N-(5-methylbenzo[d]isoxazol-3-yl)benzamide,
[124] 4-tert-butyl-N-(5-methylbenzo[d]isoxazol-3-yl)benzamide,
[125] 2-methyl-N-(5-methylbenzo[d]isoxazol-3-yl)benzamide,
[126] 2,3-difluoro-N-(5-methylbenzo[d]isoxazol-3-yl) benzamide,
[127] 2,4-difluoro-N-(5-methylbenzo[d]isoxazol-3-yl) benzamide,
[128] 2-chloro-N-(5-methylbenzo[d]isoxazol-3-yl) nicotinamide,
[129] cyclopropane-(5-fluorobenzo[d]isoxazol-3-yl) carboxamide,
[130] N-(5-fluorobenzo[d]isoxazol-3-yl)-3,3-dimethylbutyramide,
[131] 2,4-difluoro-N-(5-fluorobenzo[d]isoxazol-3-yl)benzamide,
[132] 2,5-dimethylfuran-3-(5-fluorobenzo[d]isoxazol-3-yl)carboxamide,
[133] N-(5-bromobenzo[d]isoxazol-3-yl)-2,3-difluorobenzamide,
[134] 2,5-dimethylfuran-3-(5-bromobenzo[d]isoxazol-3-yl)carboxamide,
[135] N-(5-bromobenzo[d]isoxazol-3-yl)-2-methylbutyramide,
[136] N-(5-bromobenzo[d]isoxazol-3-yl)-2,6-difluoxobenzamide,
[137] N-(5-bromobenzo[d]isoxazol-3-yl)-3,4-dimethoxybenzamide,
[138] N-(5-bromobenzo[d]isoxazol-3-yl)-4-methylbenzamide,
[139] N-(5-bromobenzo[d]isoxazol-3-yl)-2,6-dimethoxybenzamide,
[140] 2-bromo-N-(5-bromobenzo[d]isoxazol-3-yl) benzamide,
[141] N-(5-bromobenzo[d]isoxazol-3-yl)-5-fluoro-2-trifluoromethylbenzamide,
[142] N-(5-bromobenzo[d]isoxazol-3-yl)-3-methoxybenzamide,
[143] 3-methyl-N-(5-methylbenzo[d]isoxazol-3-yl)benzamide,
[144] 4-cyano-N-(5-methylbenzo[d]isoxazol-3-yl)benzamide,
[145] N-(5-methylbenzo[d]isoxazol-3-yl)-2-phenylbutyramide,
[146] N-(5-methylbenzo[d]isoxazol-3-yl)-2-methylpentanamide,
[147] N-(4-fluorobenzo[d]isoxazol-3-yl)benzamide,
[148] 4-chloro-N-(4-fluorobenzo[d]isoxazol-3-yl)benzamide,
[149] 2-fluoro-N-(4-fluorobenzo[d]isoxazol-3-yl)benzamide,
[150] adamantane-1-(4-fluorobenzo[d]isoxazol-3-yl)carboxamide,
[151] adamantane-1-(4-chlorobenzo[d]isoxazol-3-yl)carboxamide,
[152] N-(4-chlorobenzo[d]isoxazol-3-yl)benzamide,
[153] N-(4-chlorobenzo[d]isoxazol-3-yl)-3-methylbenzamide,
[154] N-(4-chlorobenzo[d]isoxazol-3-yl)-2-methylbutyramide,
[155] N-(4-chlorobenzo[d]isoxazol-3-yl)-3,3-dimethylbutyramide,
[156] N-(4-methoxybenzo[d]isoxazol-3-yl)-2-methylbenzamide,
[157] 2-chloro-N-(4-methoxybenzo[d]isoxazol-3-yl)benzamide,
[158] N-(4-methoxybenzo[d]isoxazol-3-yl)-2-trifluoromethylbenzamide,
[159] N-(4-methoxybenzo[d]isoxazol-3-yl)-2-ethylhexanamide,
[160] N-(4-methoxybenzo[d]isoxazol-3-yl)-2-methylbutyramide,
[161] N-(4-methoxybenzo[d]isoxazol-3-yl)-2-methylpentanamide,
[162] cyclopropane-[4-(2,2,2-trifluoroethoxy)benzo[d]isoxazol-3-yl]carboxamide,
[163] 2-methyl-N-[4-(2,2,2-trifluoroethoxy)benzo[d]isoxazol-3-yl] butyramide,
[164] 3,3-dimethyl-N-[4-(2,2,2-trifluoroethoxy)benzo[d]isoxazol-3-yl]butyramide,
[165] cyclohexane-[4-(2,2,2-trifluoroethoxy)benzo[d]isoxazol-3-yl]carboxamide,
[166] 2,5-dimethylfuran-3-[4-(2,2,2-trifluoroethoxy)benzo[d]isoxazol-3-yl]carboxamide,
[167] N-[4-(4-methylbenzyloxy)benzo[d]isoxazol-3-yl]-3-nitrobenzamide,
[168] N-[4-(4-methylbenzyloxy)benzo[d]isoxazol-3-yl]-4-propylbenzamide,
[169] N-(4-dimethylaminobenzo[d]isoxazol-3-yl)-3-fluoro-5-trifluoromethylbenzamide,
[170] 3,4-dichloro-N-(4-dimethylaminobenzo[d]isoxazol-3-yl)benzamide,
[171] N-(4-dimethylaminobenzo[d]isoxazol-3-yl)-3-nitrobenzamide,
[172] adamantane-1-(4-dimethylaminobenzo[d]isoxazol-3-yl)carboxamide,
[173] benzo[b]thiophene-2-(4-dimethylaminobenzo[d]isoxazol-3-yl)carboxamide,
[174] N-(4-dimethylaminobenzo[d]isoxazol-3-yl)-3,3-dimethylbutyramide,
[176] N-(6-amino-4,5,7-trifluorobenzo[d]isoxazol-3-yl)-2,6-difluorobenzamide and
[177] N-(6-amino-4,5,7-trifluorobenzo[d]isoxazol-3-yl)-2,4-difluorobenzamide,
in each case optionally in the form of one of their pure stereoisomers, in particular enantiomers or diastereomers or rotamers, their racemates or in the form of a mixture of stereoisomers, in particular of enantiomers and/or diastereomers and/or rotamers, in any mixing ratio, or in each case in the form of corresponding salts, or in each case in the form of corresponding solvates;
and where appropriate one or more physiologically tolerated excipients.

2. Medicament according to Claim 1 for the prophylaxis and/or treatment of pain, preferably of pain selected from the group consisting of acute pain, chronic pain, visceral pain and neuropathic pain.

3. Medicament according to Claim 1 for the prophylaxis and/or treatment of one or more disorders selected from the group consisting of disorders of food intake, preferably selected from the group consisting of bulimia, anorexia, obesity and cachexia; migraines; chronic paroxysmal hemicrania; depression; urinary incontinence; cough, asthma; glaucoma; tinitus; inflammations; neurodegenerative disorders, preferably selected from the group consisting of Parkinson's disease, Huntington's disease, Alzheimer's disease and multiple sclerosis; cognitive dysfunctions, preferably memory impairments; cognitive deficiencies (attention deficit syndrome, ADS); epilepsy; narcolepsy; diarrhea; gastritis, gastric ulcer; pruritus; anxiety states; panic attacks; schizophrenia; cerebral ischemia; muscle spasms; cramps; gastroesaphageal reflux syndrome; alcohol and/or drug abuse, preferably nicotine or cocaine, and/or medicament abuse; alcohol and/or drug dependency, preferably nicotine or cocaine, and/or medicament dependency, preferably for the prophylaxis and/or reduction of withdrawal manifestations associated with alcohol and/or drug dependency, preferably nicotine or cocaine, and/or medicament dependency; for the prophylaxis and/or reduction of a development of tolerance to medicaments and/or drugs, especially medicaments based on opioids;
for regulating food intake; for modulating motor activity, for regulating the cardiovascular system; for local anesthesia; for increasing vigilance; for increasing libido; for diuresis and/or for antinatriuresis.

4. Use of at least one compound according to Claim 1 for manufacturing a medicament for the prophylaxis and/or treatment of pain, preferably of pain selected from the group consisting of acute pain, chronic pain, visceral pain and neuropathic pain.

5. Use of at least one compound according to Claim 1 for manufacturing a medicament for the prophylaxis and/or treatment of one or more disorders selected from the group consisting of disorders of food intake, preferably selected from the group consisting of bulimia, anorexia, obesity and cachexia; migraines; chronic paroxysmal hemicrania; depression; urinary incontinence; cough, asthma; glaucoma; tinitus; inflammations; neurodegenerative disorders, preferably selected from the group consisting of Parkinson's disease, Huntington's disease, Alzheimer's disease and multiple sclerosis; cognitive dysfunctions, preferably memory impairments; cognitive deficiencies (attention deficit syndrome, WADS) ; epilepsy; narcolepsy; diarrhea; gastritis, gastric ulcer; pruritus; anxiety states; panic attacks; schizophrenia; cerebral ischemia; muscle spasms; cramps; gastroesaphageal reflux syndrome; alcohol and/or drug abuse, preferably nicotine or cocaine, and/or medicament abuse; alcohol and/or drug dependency, preferably nicotine or cocaine, and/or medicament dependency, preferably for the prophylaxis and/or reduction of withdrawal manifestations associated with alcohol and/or drug dependency, preferably nicotine or cocaine, and/or medicament dependency; for the prophylaxis and/or reduction of a development of tolerance to medicaments and/or drugs, especially medicaments based on opioids; for regulating food intake; for modulating motor activity, for regulating the cardiovascular system; for local anesthesia; for increasing vigilance; for increasing libido; for diuresis and/or for antinatriuresis.

6. Compound selected from the group consisting of
[1] thiophene-2-benzo[d]isoxazol-3-ylcarboxamide,
[3] naphthalene-2-benzo[d]isoxazol-3-ylcarboxamide,
[4] adamantane-2-benzo[d]isoxazol-3-ylcarboxamide,
[5] cyclohexane-benzo[d]isoxazol-3-ylcarboxamide,
[6] N-benzo[d]isoxazol-3-yl-2,2-dimethylpropionamide,
[7] N-(4-methoxybenzo[d]isoxazol-3-yl)-2,2-diphenylacetamide,
[8] cyclopropane(5-bromobenzo[d]isoxazol-3-yl)carboxamide,
[9] 3,5-dichloro-N-(6-fluorobenzo[d]isoxazol-3-yl)benzamide,
[10] N-(4-aminobenzo[d]isoxazol-3-yl)-4-nitrobenzamide,
[11] naphthalene-1-(4-aminobenzo[d]isoxazol-3-yl)carboxamide,
[12] benzo[b]thiophene-2-(4-fluorobenzo[d]isoxazol-3-yl)carboxamide,
[13] N-benzo[d]isoxazol-3-yl-2-trifluoromethylbenzamide,
[14] N-benzo[d]isoxazol-3-yl-3,4-dichlorobenzamide
[15] N-benzo[d]isoxazol-3-yl-2,3-difluorobenzamide,
[16] N-(4-dimethylaminobenzo[d]isoxazol-3-yl)-3-fluorobenzamide,
[17] thiophene-2-(6-fluorobenzo[d]isoxazol-3-yl)carboxamide,
[18] N-(6-fluorobenzo[d]isoxazol-3-yl)-2-methylbutyramide,
[19] N-(6-chlorobenzo[d]isoxazol-3-yl)-2-fluoro-3-trifluoxomethylbenzamide,
[20] N-(6-chlorobenzo[d]isoxazol-3-yl)-3-methoxybenzamide,
[21] N-(6-bromobenzo[d]isoxazol-3-yl)-4-tert-butylbenzamide,
[23] N-(6-bromobenzo[d]isoxazol-3-yl)-2-trifluromethylbenzamide,
[24] adamantane-2-(6-bromobenzo[d]isoxazol-3-yl)carboxamide,
[25] N-(6-bromobenzo[d]isoxazol-3-yl)-2-methylbutyramide,
[26] N-(5-fluorobenzo[d]isoxazol-3-yl)-2-trifluoromethylbenzamide,
[27] 3,4-dichloro-N-(5-fluorobenzo[d]isoxazol-3-yl)benzamide,
[29] N-(5-bromobenzo[d]isoxazol-3-yl)-2-fluoro-5-trifluoromethylbenzamide,
[30] N-(5-bromobenzo[d]isoxazol-3-yl)-2,4-difluorobenzamide,
[31] N-(5-methylbenzo[d]isoxazol-3-yl)-2-trifluoromethylbenzamide,
[32] 3-fluoro-N-(5-methylbenzo[d]isoxazol-3-yl)benzamide,
[33] N-(4-methoxybenzo[d]isoxazol-3-yl)-3,5-bis-trifluoromethylbenzamide,
[34] 3,5-difluoro-N-(4-methoxybenzo[d]isoxazol-3-yl)benzamide,
[35] N-(4-dimethylaminobenzo[d]isoxazol-3-yl)-3,5-bis-trifluoromethylbenzamide,
[36] 2-bromo-N-[4-(2,2,2-trifluoroethoxy)benzo[d]isoxazol-3-yl]benzamide,
[37] 3-chloro-N-[4-(2,2,2-trifluoroethoxy)benzo[d]isoxazol-3-yl]benzamide,
[38] 4-tert-butyl-N-[4-(2,2,2-trifluoroethoxy)benzo[d]isoxazol-3-yl]benzamide,
[39] 2-methoxy-N-[4-(2,2,2-trifluoroethoxy)benzo[d]isoxazol-3-yl]benzamide,
[40] N-(6-chlorobenzo[d]isoxazol-3-yl)-4-iodobenzamide,
[41] naphthalene-2-(6-chlorobenzo[d]isoxazol-3-yl)carboxamide,
[42] N-(6-chlorobenzo[d]isoxazol-3-yl)-3,4-difluorobenzamide,
[43] N-(6-bromobenzo[d]isoxazol-3-yl)-4-fluoro-2-trifluoromethylbenzamide,
[44] 2,4-dichloro-N-(6-bromobenzo[d]isoxazol-3-yl)-5-fluorobenzamide,
[45] N-(6-bromobenzo[d]isoxazol-3-yl)-3-fluoro-4-trifluoromethylbenzamide,
[46] N-(6-bromobenzo[d]isoxazol-3-yl)-3-fluoro-5-trifluoromethylbenzamide,
[47] N-(5-bromobenzo[d]isoxazol-3-yl)-2,3,4-trifluorobenzamide,
[48] N-(6-bromobenzo[d]isoxazol-3-yl)-4-propylbenzamide,
[49] N-(6-bromobenzo[d]isoxazol-3-yl)-3,4-difluorobenzamide,
[50] furan-2-(5-bromobenzo[d]isoxazol-3-yl)carboxamide,
[51] 4-fluoro-N-(7-fluorobenzo[d]isoxazol-3-yl)-2-trifluoromethylbenzamide,
[52] 2,4-dichloro-5-fluoro-N-(7-fluorobenzo[d]isoxazol-3-yl)benzamide,
[53] 3-fluoro-N-(7-fluorobenzo[d]isoxazol-3-yl)-4-trifluoromethylbenzamide,
[54] 2,3,4-trifluoro-N-(7-fluorobenzo[d]isoxazol-3-yl)benzamide,
[55] N-(7-fluorobenzo[d]isoxazol-3-yl)-4-iodobenzamide,
[56] 2-chloro-N-(7-fluorobenzo[d]isoxazol-3-yl)nicotinamide,
[57] naphthalene-2-(7-fluorobenzo[d]isoxazol-3-yl)carboxamide,
[58] N-(4-fluorobenzo[d]isoxazol-3-yl)-74-propylbenzamide,
[59] 3,4-difluoro-N-(7-fluorobenzo[d]isoxazol-3-yl)benzamide,
[60] 2-fluoro-N-(7-fluorobenzo[d]isoxazol-3-yl)-3-trifluoromethylbenzamide,
[61] N-(7-fluorobenzo[d]isoxazol-3-yl)-3-methoxybenzamide,
[62] N-(7-fluorobenzo[d]isoxazol-3-yl)-2,2-diphenylacetamide,
[63] furan-2-(7-fluorobenzo[d]isoxazol-3-yl)carboxamide,
164] 2,4-dichloro-N-(4-dimethylaminobenzo[d]isoxazol-3-yl)-5-fluorobenzamide,
[65] N-(4-dimethylaminobenzo[d]isoxazol-3-yl)-2,3,4-trifluorobenzamide,
[66] 2-chloro-N-(4-dimethylaminobenzo[d]isoxazol-3-yl)nicotinamide,
[67] naphthalene-2-(4-dimethylaminobenzo[d]isoxazol-3-yl)carboxamide,
[68] N-(4-dimethylaminobenzo[d]isoxazol-3-yl)-4-propylbenzamide,
[69] N-(4-dimethylaminobenzo[d]isoxazol-3-yl)-3,4-difluorobenzamide,
[70] N-(4-dimethylaminobenzo[d]isoxazol-3-yl)-2-fluoro-3-trifluoromethylbenzamide,
[71] N-(4-dimethylaminobenzo[d]isoxazol-3-yl)-3-methoxybenzamide,
[72] N-(4-dimethylaminobenzo[d]isoxazol-3-yl)-2,2-dimethylpropionamide,
[73] cyclohexane-(4-dimethylaminobenzo[d]isoxazol-3-yl)carboxamide,
[74] N-(4-dimethylaminobenzo[d]isoxazol-3-yl)-2,2-diphenylacetamide,
[75] furan-2-(4-dimethylaminobenzo[d]isoxazol-3-yl)carboxamide,
[76] N-(4-dimethylaminobenzo[d]isoxazol-3-yl)-2,2,3,3,4,4,4-heptafluorobutyramide,
[77] 4-fluoro-N-[4-(2,2,2-trifluoroethoxy)benzo[d]isoxazol-3-yl]-2-trifluoromethylbenzamide,
[78] 2,4-dichloro-5-fluoro-N-[4-(2,2,2-trifluoroethoxy)benzo[d]isoxazol-3-yl]benzamide,
[79] 3-fluoro-N-[4-(2,2,2-trifluoroethoxy)benzo[d]isoxazol-3-yl]-4-trifluoromethylbenzamide,
[80] 2,3,4-trifluoro-N-[4-(2,2,2-trifluoroethoxy)benzo[d]isoxazol-3-yl]benzamide,
[81] naphthalene-2-[4-(2,2,2-trifluoroethoxy)benzo[d]isoxazol-3-yl]carboxamide,
[82] 3,4-difluoro-N-[4-(2,2,2-trifluoroethoxy)benzo[d]isoxazol-3-yl]benzamide,
[83] 2-fluoro-N-[4-(2,2,2-trifluoroethoxy)benzo[d]isoxazol-3-yl]-3-trifluoromethylbenzamide,
[85] N-benzo[d]isoxazol-3-yl-2-ethylhexanamide,
[86] N-benzo[d]isoxazol-3-yl-2-methylbutyramide,
[88] N-benzo[d]isoxazol-3-yl-3-fluoro-4-trifluoromethylbenzamide,
[89] N-benzo[d]isoxazol-3-yl-2,3,6-trifluorobenzamide,
[90] N-benzo[d]isoxazol-3-ylnicotinamide,
[91] 5-methylisoxazol-3-benzo[d]isoxazol-3-ylcarboxamide,
[92] benzo[b]thiophene-3-benzo[d]isoxazol-3-ylcarboxamide,
[94] 4-tert-butyl-N-(7-fluorobenzo[d]isoxazol-3-yl)benzamide,
[96] 4-fluoro-N-(7-fluorobenzo[d]isoxazol-3-yl)benzamide,
[100] 3,5-difluoro-N-(6-fluorobenzo[d]isoxazol-3-yl)benzamide,
[101] naphthalene-1-(6-fluorobenzo[d]isoxazol-3-yl)carboxamide,
[102] adamantane-1-(6-fluorobenzo[d]isoxazol-3-yl)carboxamide,
[104] 4-tert-butyl-N-(5-fluorobenzo[d]isoxazol-3-yl)benzamide,
[105] 2,4-difluoro-N-(5-fluorobenzo[d]isoxazol-3-yl)benzamide,
[106] naphthalene-2-(5-fluorobenzo[d]isoxazol-3-yl)carboxamide,
[107] N-(5-fluorobenzo[d]isoxazol-3-yl)-4-propylbenzamide,
[109] 4-tert-butyl-N-(6-chlorobenzo[d]isoxazol-3-yl)benzamide,
[110] N-(6-chlorobenzo[d]isoxazol-3-yl)-4-fluoro-2-trifluoromethylbenzamide,
[111] 2,4-dichloro-N-(6-chlorobenzo[d]isoxazol-3-yl)-5-fluorobenzamide,
[112] N-(6-chlorobenzo[d]isoxazol-3-yl)-2-ethylhexanamide,
[113] N-(6-chlorobenzo[d]isoxazol-3-yl)-2-methylbutyramide,
[115] N-(6-bromobenzo[d]isoxazol-3-yl)-3,4-dichlorobenzamide,
[116] thiophene-2-(6-bromobenzo[d]isoxazol-3-yl)carboxamide,
[117] N-(6-bromobenzo[d]isoxazol-3-yl)-3,3-dimethylbutyramide,
[119] 4-bromo-N-(5-methylbenzo[d]isoxazol-3-yl)benzamide,
[121] 4-fluoro-N-(5-methylbenzo[d]isoxazol-3-yl)benzamide,
[123] 3-methyl-N-(5-methylbenzo[d]isoxazol-3-yl)benzamide,
[124] 4-tert-butyl-N-(5-methylbenzo[d]isoxazol-3-yl)benzamide,
[126] 2,3-difluoro-N-(5-methylbenzo[d]isoxazol-3-yl)benzamide,
[127] 2,4-difluoro-N-(5-methylbenzo[d]isoxazol-3-yl)benzamide,
[128] 2-chloro-N-(5-methylbenzo[d]isoxazol-3-yl)nicotinamide,
[129] cyclopropane-(5-fluorobenzo[d]isoxazol-3-yl)carboxamide,
[130] N-(5-fluorobenzo[d]isoxazol-3-yl)-3,3-dimethylbutyramide,
[131] 2,4-difluoro-N-(5-fluorobenzo[d]isoxazol-3-yl)benzamide,
[132] 2,5-dimethylfuran-3-(5-fluorobenzo[d]isoxazol-3-yl)carboxamide,
[133] N-(5-bromobenzo[d]isoxazol-3-yl)-2,3-difluorobenzamide,
[134] 2,5-dimethylfuran-3-(5-bromobenzo[d]isoxazol-3-yl)carboxamide,
[135] N-(5-bromobenzo[d]isoxazol-3-yl)-2-methylbutyramide,
[137] N-(5-bromobenzo[d]isoxazol-3-yl)-3,4-dimethoxybenzamide,
[138] N-(5-bromobenzo[d]isoxazol-3-yl)-4-methylbenzamide,
[141] N-(5-bromobenzo[d]isoxazol-3-yl)-5-fluoro-2-trifluoromethylbenzamide,
[142] N-(5-bromobenzo[d]isoxazol-3-yl)-3-methoxybenzamide,
[143] 3-methyl-N-(5-methylbenzo[d]isoxazol-3-yl)benzamide,
[144] 4-cyano-N-(5-methylbenzo[d]isoxazol-3-yl)benzamide,
[145] N-(5-methylbenzo[d]isoxazol-3-yl)-2-phenylbutyramide,
[146] N-(5-methylbenzo[d]isoxazol-3-yl)-2-methylpentanamide,
[147] 4-chloro-N-(4-fluorobenzo[d]isoxazol-3-yl)benzamide,
[150] adamantane-1-(4-fluorobenzo[d]isoxazol-3-yl)carboxamide,
[151] adamantane-1-(4-chlorobenzo[d]isoxazol-3-yl)carboxamide,
[153] N-(4-chlorobenzo[d]isoxazol-3-yl)-3-methylbenzamide,
[154] N-(4-chlorobenzo[d]isoxazol-3-yl)-2-methylbutyramide,
[155] N-(4-chlorobenzo[d]isoxazol-3-yl)-3,3-dimethylbutyramide,
[156] N-(4-methoxybenzo[d]isoxazol-3-yl)-2-methylbenzamide,
[157] 2-chloro-N-(4-methoxybenzo[d]isoxazol-3-yl)benzamide,
[158] N-(4-methoxybenzo[d]isoxazol-3-yl)-2-trifluoromethylbenzamide,
[159] N-(4-methoxybenzo[d]isoxazol-3-yl)-2-ethylhexanamide,
[160] N-(4-methoxybenzo[d]isoxazol-3-yl)-2-methylbutyramide,
[161] N-(4-methoxybenzo[d]isoxazol-3-yl)-2-methylpentanamide,
[162] cyclopropane-[4-(2,2,2-trifluoroethoxy)benzo[d]isoxazol-3-yl]carboxamide,
[163] 2-methyl-N-[4-(2,2,2-trifluoroethoxy)benzo[d]isoxazol-3-yl]butyramide,
[164] 3,3-dimethyl-N-[4-(2,2,2-trifluoroethoxy)benzo[d]isoxazyl-3-y]butyramide,
[165] cyclohexane-[4-(2,2,2-trifluoroethoxy)benzo[d]isoxazol-3-yl]carboxamide,
[166] 2,5-dimethylfuran-3-[4-(2,2,2-trifluoroethoxy)benzo[d]isoxazol-3-yl]carboxamide,
[167] N-[4-(4-methylbenzyloxy)benzo[d]isoxazol-3-yl]-3-nitrobenzamide,
[168] N-[4-(4-methylbenzyloxy)benzo[d]isoxazol-3-yl]-4-propylbenzamide,
[169] N-(4-dimethylaminobenzo[d]isoxazol-3-yl)-3-fluoro-5-trifluoromethylbenzamide,
[170] 3,4-dichloro-N-(4-dimethylaminobenzo[d]isoxazol-3-yl)benzamide,
[171] N-(4-dimethylaminobenzo[d]isoxazol-3-yl)-3-nitrobenzamide,
[172] adamantane-1-(4-dimethylaminobenzo[d]isoxazol-3-yl)carboxamide,
[173] benzo[b]thiophene-2-(4-dimethylaminobenzo[d]isoxazol-3-yl)carboxamide,
[174] N-(4-dimethylaminobenzo[d]isoxazol-3-yl)-3,3-dimethylbutyramide,
[176] N-(6-amino-4,5,7-trifluorobenzo[d]isoxazol-3-yl)-2,6-difluorobenzamide and
[177] N-(6-amino-4,5,7-trifluorobenzo[d]isoxazol-3-yl)-2,4-difluorobenzamide,
in each case optionally in the form of its pure stereoisomers, in particular enantiomers or diastereomers or rotamers, its racemates or in the form of a mixture of stereoisomers, in particular of enantiomers and/or diastereomers and/or rotamers, in any mixing ratio, or in each case in the form of corresponding salts, or in each case in the form of corresponding solvates.

7. Process for preparing a compound according to Claim 6, **characterized in that** at least one compound of the general formula IIa 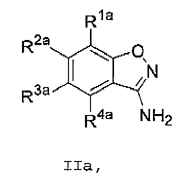 in which R^{1a} to R^{4a} have the meaning as defined by the compounds according to Claim 6, is reacted
in a reaction medium, where appropriate in the presence of at least one base, with at least one compound of the general formula R^{5a}-C (=O)-X in which R^{5a} has the meaning as defined by the compounds according to Claim 6, and X is a leaving group, preferably a halogen radical, particularly preferably a chlorine atom,
or in a reaction medium in the presence of at least one coupling reagent, where appropriate in the presence of at least one base, with at least one compound of the general formula R^{5a}-C(=O)-OH in which R^{5a} has the meaning as defined by the compounds according to Claim 6, to give a compound according to Claim 6, and the latter is isolated and/or purified where appropriate.

8. Medicament comprising at least one compound according to Claim 6 and where appropriate one or more physiologically tolerated excipients.

9. Medicament according to Claim 8 for the prophylaxis and/or treatment of pain, preferably of pain selected from the group consisting of acute pain, chronic pain, visceral pain and neuropathic pain.

10. Medicament according to Claim 8 for the prophylaxis and/or treatment of one or more disorders selected from the group consisting of disorders of food intake, preferably selected from the group consisting of bulimia, anorexia, obesity and cachexia; migraines; chronic paroxysmal hemicrania; depression; urinary incontinence; cough, asthma; glaucoma; tinitus; inflammations; neurodegenerative disorders, preferably selected from the group consisting of Parkinson's disease, Huntington's disease, Alzheimer's disease and multiple sclerosis; cognitive dysfunctions, preferably memory impairments; cognitive deficiencies (attention deficit syndrome, ADS); epilepsy; narcolepsy; diarrhea; gastritis, gastric ulcer; pruritus; anxiety states; panic attacks; schizophrenia; cerebral ischemia; muscle spasms; cramps; gastroesaphageal reflux syndrome; alcohol and/or drug abuse, preferably nicotine or cocaine, and/or medicament abuse; alcohol and/or drug dependency, preferably nicotine or cocaine, and/or medicament dependency, preferably for the prophylaxis and/or reduction of withdrawal manifestations associated with alcohol and/or drug dependency, preferably nicotine or cocaine, and/or medicament dependency; for the prophylaxis and/or reduction of a development of tolerance to medicaments and/or drugs, especially medicaments based on opioids; for regulating food intake; for modulating motor activity, for regulating the cardiovascular system; for local anesthesia; for increasing vigilance; for increasing libido; for diuresis and/or for antinatriuresis.

11. Use of at least one compound according to Claim 6 for manufacturing a medicament for the prophylaxis and/or treatment of pain, preferably of pain selected from the group consisting of acute pain, chronic pain, visceral pain and neuropathic pain.

12. Use of at least one compound according to Claim 6 for manufacturing a medicament for the prophylaxis and/or treatment of one or more disorders selected from the group consisting of disorders of food intake, preferably selected from the group consisting of bulimia, anorexia, obesity and cachexia; migraines; chronic paroxysmal hemicrania; depression; urinary incontinence; cough; asthma; glaucoma; tinitus; inflammations; neurodegenerative disorders, preferably selected from the group consisting of Parkinson's disease, Huntington's disease, Alzheimer's disease and multiple sclerosis; cognitive dysfunctions,
preferably memory impairments; cognitive deficiencies (attention deficit syndrome, ADS); epilepsy; narcolepsy; diarrhea; gastritis, gastric ulcer; pruritus; anxiety states; panic attacks; schizophrenia; cerebral ischemia; muscle spasms; cramps; gastroesaphageal reflux syndrome; alcohol and/or drug abuse, preferably nicotine or cocaine, and/or medicament abuse; alcohol and/or drug dependency, preferably nicotine or cocaine, and/or medicament dependency, preferably for the prophylaxis and/or reduction of withdrawal manifestations associated with alcohol and/or drug dependency, preferably nicotine or cocaine, and/or medicament dependency; for the prophylaxis and/or reduction of a development of tolerance to medicaments and/or drugs, especially medicaments based on opioids; for regulating food intake; for modulating motor activity, for rregulating the cardiovascular system; for local anesthesia; for increasing vigilance; for increasing libido; for diuresis and/or for antinatriuresis.

## Revendications

1. Médicament contenant au moins un composé choisi dans le groupe constitué de :
[1] acide thiophène-2-carboxylique-benzo[d]isoxazole-3-ylamide,
[3] acide naphtalène-2-carboxylique-benzo[d]isoxazole-3-ylamide,
[4] acide adamantane-2-carboxylique-benzo[d]isoxazole-3-ylamide,
[5] acide cyclohexanecarboxylique-benzo[d]isoxazole-3-ylamide,
[6] N-benzo[d]isoxazole-3-yl-2,2-diméthyl-propionamide,
[7] N-(4-méthoxy-benzo[d]isoxazole-3-yl)-2,2-diphényl-acétamide,
[8] acide cyclopropanecarboxylique-(5-bromo-benzo[d]isoxazole-3-yl)-amide,
[9] 3,5-dichloro-N-(6-fluoro-benzo[d]isoxazole-3-yl)-benzamide,
[10] N-(4-amino-benzo[d]isoxazole-3-yl)-4-nitrobenzamide,
[11] acide naphtalène-1-carboxylique-(4-amino-benzo[d]isoxazole-3-yl)-amide,
[12] acide benzo[b]thiophène-2-carboxylique-(4-fluoro-benzo[d]isoxazole-3-yl)-amide,
[13] N-benzo[d]isoxazole-3-yl-2-trifluorométhyl-benzamide,
[14] N-benzo[d]isoxazole-3-yl-3,4-dichloro-benzamide,
[15] N-benzo[d]isoxazole-3-yl-3,4-difluoro-benzamide,
[16] N-(4-diméthylamino-benzo[d]isoxazole-3-yl)-3-fluorobenzamide,
[17] acide thiophène-2-carboxylique-(6-fluoro-benzo[d]isoxazole-3-yl)-amide,
[18] N-(6-fluoro-benzo[d]isoxazole-3-yl)-2-méthyl-butyramide,
[19] N-(6-chloro-benzo[d]isoxazole-3-yl)-2-fluoro-3-trifluorométhyl-benzamide,
[20] N-(6-chloro-benzo[d]isoxazole-3-yl)-3-méthoxy-benzamide,
[21] N-(6-bromo-benzo[d]isoxazole-3-yl)-4-tert-butyl-benzamide,
[22] N-(6-bromo-benzo[d]isoxazole-3-yl)-2-méthoxy-benzamide,
[23] N-(6-bromo-benzo[d]isoxazole-3-yl)-2-trifluorométhyl-benzamide,
[24] acide adamantane-2-carboxylique-(6-bromo-benzo[d]isoxazole-3-yl)-amide,
[25] N-(6-bromo-benzo[d]isoxazole-3-yl)-2-méthyl-butyramide,
[26] N-(5-fluoro-benzo[d]isoxazole-3-yl)-2-trifluorométhyl-benzamide,
[27] 3,4-dichloro-N-(5-fluoro-benzo[d]isoxazole-3-yl)-benzamide,
[28] N-(5-fluoro-benzo[d]isoxazole-3-yl)-2-méthyl-benzamide,
[29] N-(5-bromo-benzo[d]isoxazole-3-yl)-2-fluoro-5-trifluorométhyl-benzamide,
[30] N-(5-bromo-benzo[d]isoxazole-3-yl)-2,4-difluoro-benzamide,
[31] N-(5-méthyl-benzo[d]isoxazole-3-yl)-2-trifluorométhyl-benzamide,
[32] 3-fluoro-N-(5-méthyl-benzo[d]isoxazole-3-yl)-benzamide,
[33] N-(4-méthoxy-benzo[d]isoxazole-3-yl)-3,5-bis-trifluorométhyl-benzamide,
[34] 3,5-difluoro-N-(4-méthoxy-benzo[d]isoxazole-3-yl)-benzamide,
[35] N-(4-diméthylamino-benzo[d]isoxazole-3-yl)-3,5-bis-trifluoxométhyl-benzamide,
[36] 2-bromo-N-[4-(2,2,2-trifluoro-éthoxy)-benzo[d]isoxazole-3-yl]-benzamide,
[37] 3-chloro-N-[4-(2,2,2-trifluoro-éthoxy)-benzo[d]isoxazole-3-yl]-benzamide,
[38] 4-tert-butyl-N-[4-(2,2,2-trifluoro-éthoxy)-benzo[d]isoxazole-3-yl]-benzamide,
[39] 2-méthoxy-N-[4-(2,2,2-trifluoro-éthoxy)-benzo[d]isoxazole-3-yl]-benzamide,
[40] N-(6-chloro-benzo[d]isoxazole-3-yl)-4-iodo-benzamide,
[41] acide naphtalène-2-carboxylique-(6-chloro-benzo[d]isoxazole-3-yl)-amide,
[42] N-(6-chloro-benzo[d]isoxazole-3-yl)-3,4-difluoro-benzamide,
[43] N-(6-bromo-benzo[d]isoxazole-3-yl)-4-fluoro-2-trifluorométhyl-benzamide,
[44] 2,4-dichloro-N-(5-bromo-benzo[d]isoxazole-3-yl)-5-fluoro-benzamide,
[45] N-(6-bromo-benzo[d]isoxazole-3-yl)-3-fluoro-4-trifluorométhyl-benzamide,
[46] N-(6-bromo-benzo[d]isoxazole-3-yl)-3-fluoro-5-trifluorométhyl-benzamide,
[47] N-(6-bromo-benzo[d]isoxazole-3-yl)-2,3,4-trifluoro-benzamide,
[48] N-(6-bromo-benzo[d]isoxazole-3-yl)-4-propyl-benzamide,
[49] N-(6-bromo-benzo[d]isoxazole-3-yl)-3,4-difluoro-benzamide,
[50] acide furane-2-carboxylique-(5-bromo-benzo[d]isoxazole-3-yl)-amide,
[51] 4-fluoro-N-(7-fluoro-benzo[d]isoxazole-3-yl)-2-trifluorométhyl-benzamide,
[52] 2,4-dichloro-5-fluoro-N-(7-fluoro-benzo[d]isoxazole-3-yl)-benzamide,
[53] 3-fluoro-N-(7-fluoro-benzo[d]isoxazole-3-yl)-4-trifluorométhyl-benzamide,
[54] 2,3,4-trifluoro-N-(7-fluoro-benzo[d]isoxazole-3-yl)-benzamide,
[55] N-(7-fluoro-benzo[d]isoxazole-3-yl)-4-iodo-benzamide,
[56] 2-chloro-N-(7-fluoro-benzo[d]isoxazole-3-yl)-nicotinamide,
[57] acide naphtalène-2-carboxylique-(7-fluoro-benzo[d]isoxazole-3-yl)-amide,
[58] N-(7-fluoro-benzo[d]isoxazole-3-yl)-4-propyl-benzamide,
[59] 3,4-difluoro-N-(7-fluoro-benzo[d]isoxazole-3-yl)-benzamide,
[60] 2-fluoro-N-(7-fluoro-benzo[d]isoxazole-3-yl)-3-trifluorométhyl-benzamide,
[61] N-(7-fluoro-benzo[d]isoxazole-3-yl)-3-méthoxy-benzamide,
[62] N-(7-fluoro-benzo[d]isoxazole-3-yl)-2,2-diphényl-acétamide,
[63] acide furane-2-carboxylique-(7-fluoro-benzo[d]isoxazole-3-yl)-amide,
[64] 2,4-dichloro-N-(4-diméthylamino-benzo[d]isoxazole-3-yl)-5-fluoro-benzamide,
[65] N-(4-diméthylamino-benzo[d]isoxazole-3-yl)-2,3,4-trifluoro-benzamide,
[66] 2-chloro-N-(4-diméthylamino-benzo[d]isoxazole-3-yl)-nicotinamide,
[67] acide naphtalène-2-carboxylique-(4-diméthylamino-benzo[d]isoxazole-3-yl)-amide,
[68] N-(4-diméthylamino-benzo[d]isoxazole-3-yl)-4-propyl-benzamide,
[69] N-(4-diméthylamino-benzo[d]isoxazole-3-yl) - 3,4-difluoro-benzamide,
[70] N-(4-diméthylamino-benzo[d]isoxazole-3-yl)-2-fluoro-3-trifluorométhyl-benzamide,
[71] N-(4-diméthylamino-benzo[d]isoxazole-3-yl)-3-méthoxy-benzamide,
[72] N-(4-diméthylamino-benzo[d]isoxazole-3-yl)-2,2-diméthyl-propionamide,
[73] acide cyclohexanecarboxylique-(4-diméthylamino-benzo[d]isoxazole-3-yl)-amide,
[74] N-(4-diméthylamino-benzo[d]isoxazole-3-yl)-2,2-diphényl-acétamide,
[75] acide furane-2-carboxylique-(4-diméthylamino-benzo[d]isoxazole-3-yl)-amide,
[76] N-(4-diméthylamino-benzo[d]isoxazole-3-yl)-2,2,3,3,4,4,-heptafluoro-butyramide,
[77] 4-fluoro-N-[4-(2,2,2-trifluoro-éthoxy)-benzo[d]isoxazole-3-yl]-2-trifluorométhyl-benzamide,
[78] 2,4-dichloro-5-fluoro-N-[4-(2,2,2-trifluoro-éthoxy)-benzo[d]isoxazole-3-yl]-benzamide,
[79] 3-fluoro-N-[4-(2,2,2-trifluoro-éthoxy)-benzo[d]isoxazole-3-yl]-4-trifluorométhyl-benzamide,
[80] 2,3,4-trifluoro-N-[4-(2,2,2-trifluoro-éthoxy)-benzo[d]isoxazole-3-yl]-benzamide,
[81] acide naphtalène-2-carboxylique-[4-(2,2,2-trifluoro-éthoxy)-benzo[d]isoxazole-3-yl]-amide,
[82] 3,4-difluoro-N-[4-(2,2,2-trifluoro-éthoxy)-benzo[d]isoxazole-3-yl]-benzamide,
[83] 2-fluoro-N-[4-(2,2,2-trifluoro-éthoxy)-benzo[d]isoxazole-3-yl]-3-trifluorométhyl-benzamide, [85] acide 2-éthyl-hexane-benzo[d]isoxazole-3-ylamide,
[86] N-benzo[d]isoxazole-3-yl-2-méthyl-butyramide,
[87] N-benzo[d]isoxazole-3-yl-2,6-difluoro-benzamide,
[88] N-benzo[d]isoxazole-3-yl-3-fluoro-4-trifluorométhyl-benzamide,
[89] N-benzo[d]isoxazole-3-yl-2,3,6-trifluoro-benzamide,
[90] N-benzo[d]isoxazole-3-yl-nicotinamide,
[91] acide 5-méthyl-isoxazol-3-carboxylique-benzo[d]isoxazole-3-ylamide,
[92] acide benzo[b]thiophène-3-carboxylique-benzo[d]isoxazole-3-ylamide,
[93] 2-chloro-N-(7-fluoro-benzo[d]isoxazole-3-yl)-benzamide,
[94] 4-tert-butyl-N-(7-fluoro-benzo[d]isoxazole-3-yl)-benzamide,
[95] N-(7-fluoro-benzo[d]isoxazole-3-yl)-benzamide,
[96] 4-fluoro-N-(7-fluoro-benzo[d]isoxazole-3-yl)-benzamide,
[97] 2-fluoro-N-(7-fluoro-benzo[d]isoxazole-3-yl)-benzamide,
[98] N-(7-fluoro-benzo[d]isoxazole-3-yl)-2-méthoxy-benzamide,
[99] N-(7-fluoro-benzo[d]isoxazole-3-yl)-2-méthyl-benzamide,
[100] 3,5-difluoro-N-(6-fluoro-benzo[d]isoxazole-3-yl)-benzamide,
[101] acide naphtalène-1-carboxylique-(6-fluoro-benzo[d]isoxazole-3-yl)-amide,
[102] acide adamantane-1-carboxylique-(6-fluoro-benzo[d]isoxazole-3-yl)-amide,
[103] 2-bromo-N-(5-fluoro-benzo[d]isoxazole-3-yl)-benzamide,
[104] 4-tert-butyl-N-(5-fluoro-benzo[d]isoxazole-3-yl)-benzamide,
[105] 2,4-difluoro-N-(5-fluoro-benzo[d]isoxazole-3-yl)-benzamide,
[106] acide naphtalène-2-carboxylique-(5-fluoro-benzo[d]isoxazole-3-yl)-amide,
[107] N-(5-fluoro-benzo[d]isoxazole-3-yl)-4-propyl-benzamide,
[108] 2-chloro-N-(6-chloro-benzo[d]isoxazole-3-yl)-benzamide,
[109] 4-tert-butyl-N-(6-chloro-benzo[d]isoxazole-3-yl)-benzamide,
[110] N-(6-chloro-benzo[d]isoxazole-3-yl)-4-fluoro-2-trifluorométhyl-benzamide,
[111] 2,4-dichloro-N-(6-chloro-benzo[d]isoxazole-3-yl)-5-fluoro-benzamide,
[112] acide 2-éthyl-hexane-(6-chloro-benzo[d]isoxazole-3-yl)-amide,
[113] N-(6-chloro-benzo[d]isoxazole-3-yl)-2-méthyl-butyramide,
[114] N-(6-bromo-benzo[d]isoxazole-3-yl)-2-chloro-benzamide,
[115] N-(6-bromo-benzo[d]isoxazole-3-yl)-3,4-dichloro-benzamide,
[116] acide thiophène-2-carboxylique-(6-bromo-benzo[d]isoxazole-3-yl)-amide,
[117] N-(6-bromo-benzo[d]isoxazole-3-yl)-3,3-diméthyl-butyramide,
[118] N-(5-méthyl-benzo[d]isoxazole-3-yl)-benzamide,
[119] 4-bromo-N-(5-méthyl-benzo[d]isoxazole-3-yl)-benzamide,
[120] 2-chloro-N-(5-méthyl-benzo[d]isoxazole-3-yl)-benzamide,
[121] 4-fluoro-N-(5-méthyl-benzo[d]isozole-3-yl)-benzamide,
[122] 2-fluoro-N-(5-méthyl-benzo[d]isoxazole-3-yl)-benzamide,
[123] 3-méthyl-N-(5-méthyl-benzo[d]isoxazole-3-yl)-benzamide,
[124] 4-tert-butyl-N-(5-méthyl-benzo[d]isoxazole-3-yl)-benzamide,
[125] 2-méthyl-N-(5-méthyl-benzo[d]isoxazole-3-yl)-benzamide,
[126] 2,3-difluoro-N-(5-mèthyl-benzo[d] isoxazole-3-yl)-benzamide,
[127] 2,4-difluoro-N-(5-méthyl-benzo[d]isoxazole-3-yl)-benzamide,
[128] 2-chloro-N-(5-méthyl-benzo[d]isoxazole-3-yl)-nicotinamide,
[129] acide cyclopropanecarboxylique-(5-fluoro-benzo[d]isoxazole-3-yl)-amide,
[130] N-(5-fluoro-benzo[d]isoxazole-3-yl)-3,3-diméthyl-butyramide,
[131] 2,4-difluoro-N-(5-fluoro-benzo[d]isoxazole-3-yl)-benzamide,
[132] acide 2,5-diméthyl-furane-3-carboxylique-(5-fluoro-benzo[d]isoxazole-3-yl)-amide,
[133] N-(5-bromo-benzo[d]isoxazole-3-yl)-2,3-difluoro-benzamide,
[134] acide 2,5-diméthyl-furane-3-carboxylique-(5-bromo-benzo[d]isoxazole-3-yl)-amide,
[135] N-(5-bromo-benzo[d]isoxazole-3-yl)-2-méthyl-butyramide,
[136] N-(5-bromo-benzo[d]isoxazole-3-yl)-2,6-difluoro-benzamide,
[137] N-(5-bromo-benzo[d]isoxazole-3-yl)-3,4-diméthoxy-benzamide,
[138] N-(5-bromo-benzo[d]isoxazole-3-yl)-4-méthyl-benzamide,
[139] N-(5-bromo-benzo[d]isoxazole-3-yl)-2,6-diméthoxy-benzamide,
[140] 2-bromo-N-(5-bromo-benzo[d]isoxazole-3-yl)-benzamide,
[141] N-(5-bromo-benzo[d]isoxazole-3-yl)-5-fluoro-2-trifluorométhyl-benzamide,
[142] N-(5-bromo-benzo[d]isoxazole-3-yl)-3-méthoxy-benzamide,
[143] 3-méthyl-N-(5-méthyl-benzo[d]isoxazole-3-yl)-benzamide,
[144] 4-cyano-N-(5-méthyl-benzo[d]isoxazole-3-yl)-benzamide,
[145] N-(5-méthyl-benzo[d]isaxazole-3-yl)-2-phényl-butyramide,
[146] acide 2-méthylpentane-(5-méthyl-benzo[d]isoxazole-3-yl)-amide,
[147] N-(4-fluoro-benzo[d]isoxazole-3-yl)-benzamide,
[148] 4-chloro-N-(4-fluoro-benzo[d]isoxazole-3-yl)-benzamide,
[199] 2-fluoro-N-(4-fluoro-benzo[d]isoxazole-3-y1)-benzamide,
[150] acide adamantane-1-carboxylique-(4-fluoro-benzo[d]isoxazole-3-yl)-amide,
[151] acide adamantane-1-carboxylique-(4-chloro-benzo[d]isoxazole-3-yl)-amide,
[152] N-(4-chloro-benzo[d]isoxazole-3-yl)-benzamide,
[153] N-(4-chloro-benzo[d]isoxazole-3-yl)-3-méthyl-benzamide,
[154] N-(4-chloro-benzo[d]isoxazole-3-yl)-2-méthyl-butyramide,
[155] N-(4-chloro-benzo[d]isoxazole-3-yl)-3,3-diméthyl-butyramide,
[156] N-(4-méthoxy-benzo[d]isoxazole-3-yl)-2-méthyl-benzamide,
[157] 2-chloro-N-(4-méthoxy-benzo[d]-isoxazole-3-yl)-benzamide,
[158] N-(4-métoxy-benzo[d]isoxazole-3-yl)-2-trifluorométhyl-benzamide,
[159] acide 2-éthylhexane-(4-métoxy-benzo[d]isoxazole-3-yl)-amide,
[160] N-(4-méthoxy-benzo[d]isoxazole-3-yl)-2-méthyl-butyramide,
[161] acide 2-méthylpentane-(4-métoxy-benzo[d]isoxazole-3-yl)-amide,
[162] acide cyclopropanecarboxylique-[4-(2,2,2-trifluoro-ethoxy)-benzo[d]isoxazole-3-yl]-amide,
[163] 2-méthyl-N-[4-(2,2,2-trifluoro-éthoxy)-benzo[d]isoxazole-3-yl]-butyramide,
[164] 3,3-diméthyl-N-[4-(2,2,2-trifluoro-éthoxy)-benzo[d]isoxazole-3-yl]-butyramide,
[165] acide cyclohexanecarboxylique-[4-(2,2,2-trifluoro-éthoxy)-benzo[d]isoxazole-3-yl]-amide,
[166] acide 2,5-diméthyl-furane-3-carboxylique-[4-(2,2,2-trifluoro-éthoxy)-benzo[d]isoxazole-3-yl]-amide,
[167] N-[4-(4-méthyl-benzyloxy)-benzo[d]isoxazole-3-yl]-3-nitro-benzamide,
[163] N-[4-(4-méthyl-benzyloxy)-benzo[d]isoxazole-3-yl]-4-propyl-benzamide,
[169] N-(4-diméthylamino-benzo[d]isoxazole-3-yl)-3-fluoro-5-trifluorométhyl-benzamide,
[170] 3,4-dichloro-N-(4-diméthylamino-benzo[d]isoxazole-3-yl)-benzamide,
[171] N-(4-diméthylamino-benzo[d]isoxazole-3-yl)-3-nitro-benzamide,
[172] acide adamantane-1-carboxylique-(4-diméthylamino-benzo[d]isoxazole-3-yl)-amide,
[173] acide benzo[b]thiophène-2-carboxylique-(4-diméthylamino-benzo[d]isoxazole-3-yl)-amide,
[174] N-(4-diméthylamino-benzo[d]isoxazole-3-yl)-3,3-diméthyl-butyramide,
[176] N-(6-amino-4,5,7-trifluoro-benzo[d]isoxazole-3-yl)-2,6-difluoro-benzamide
et
[177] N-(5-amino-4,5,7-trifluoro-benzo[d]isoxazole-3-yl)-2,4-difluoro-benzamide,
chaque fois éventuellement sous la forme d'un de leurs stéréo-isomères purs, notamment énantiomères ou diastéréo- isomères ou rotamères, de leurs racémates ou sous la forme d'un mélange de stéréo-isomères, notamment des énantiomères et/ou diastéréo-isoméres et/ou rotamères, dans des proportions quelconques dans le mélange, ou à chaque fois sous la forme de sels correspondants, ou à chaque fois sous la forme de solvates correspondants ;
ainsi que, éventuellement, un ou plusieurs adjuvants physiologiquement compatibles.

2. Médicament selon la revendication 1 pour la prophylaxie et/ou pour le traitement de la douleur, de préférence d'une douleur prise dans le groupe constitué de la douleur aiguë, de la douleur chronique, de la douleur viscérale et de la douleur neuropathique.

3. Médicament selon la revendication 1 pour la prophylaxie et/ou le traitement d'une ou plusieurs maladies prises dans le groupe constitué des troubles de l'alimentation, de préférence dans le groupe constitué de la boulimie, de l'anorexie, de l'obésité et de la cachexie ; de la migraine ; de l'hêmicrânie paroxystique chronique ; des dépressions ; de l'incontinence urinaire ; de la toux ; de l'asthme ; du glaucome ; des acouphènes ; des inflammations ; des maladies neurodégénératives, de préférence dans le groupe constitué de la maladie de Parkinson, de la maladie de Huntington, de la maladie d'Alzheimer et de la sclérose en plaques ; des dysfonctionnements cognitifs, de préférence des troubles de la mémoire ; troubles de l'attention (syndrome du déficit de l'attention, TDA) ; épilepsie ; narcolepsie ; diarrhée ; gastrite ; ulcère de l'estomac ; prurit ; anxiété ; panique ; schizophrénie ; ischémie cérébrale ; spasmes musculaires ; crampes ; syndrome de reflux gastro-oesophagien ; abus d'alcools et/ou de drogues, de préférence de nicotine ou de cocaïne, et/ou de médicaments ; dépendance à l'alcool et/ou à la drogue, de préférence à la nicotine ou à la cocaïne, et/ou à des médicaments, de préférence pour la prophylaxie et/ou la réduction de l'état de manque en cas de dépendance à l'alcool et/ou à la drogue, de préférence à la nicotine ou à la cocaïne, et/ou à des médicament ; pour la prophylaxie et/ou la réduction d'un développement de tolérance par rapport à des médicaments et/ou à des drogues, notamment des médicaments à base d'opiacés ; pour la régulation de l'alimentation ; pour la modulation de l'activité physique, pour la régulation du système cardiovasculaire ; pour l'anesthésie locale ; pour l'augmentation de la vigilance ; pour l'augmentation de la libido ; pour à diurèse et/ou pour l'anti-natriurèse,

4. Utilisation d'au moins un composé selon la revendication 1 pour la fabrication d'un médicament pour la prophylaxie et/ou le traitement de la douleur, de préférence d'une douleur prise dans le groupe constitué de la douleur aiguë, de la douleur chronique, de la douleur viscérale et de la douleur neuropathique.

5. Utilisation d'au moins un composé selon la revendication 1 pour la fabrication d'un médicament pour la prophylaxie et/ou le traitement d'une ou plusieurs maladies prises dans le groupe constitué des troubles de l'alimentation, de préférence dans le groupe constitué de la boulimie, de l'anorexie, de l'obésité et de la cachexie ; de la migraine ; de l'hémicrânie paroxystique chronique ; des dépressions ; de l'incontinence urinaire ; de la toux ; de l'asthme ; du glaucome ; des acouphènes ; des inflammations ; des maladies neurodégénératives, de préférence dans le groupe constitué de la maladie de Parkinson, de la maladie de Huntington, de la maladie d'Alzheimer et de la sclérose en plaques ; des dysfonctionnements cognitifs, de préférence des troubles de la mémoire ; troubles de l'attention (syndrome du déficit de l'attention, TDA) ; épilepsie ; narcolepsie ; diarrhée ; gastrite ; ulcère de l'estomac ; prurit ; anxiété ; panique ; schizophrénie ; ischémie cérébrale ; spasmes musculaires ; crampes ; syndrome de reflux gastro-oesophagien ; abus d'alcools et/ou de drogues, de préférence de nicotine ou de cocaïne, et/ou de médicaments ; dépendance à l'alcool et/ou à la drogue, de préférence à la nicotine ou à la cocaïne, et/ou à des médicaments, de préférence pour la prophylaxie et/ou la réduction de l'état de manque en cas de dépendance à l'alcool et/ou à la drogue, de préférence à la nicotine ou à la cocaïne, et/ou à des médicaments ; pour la prophylaxie et/ou la réduction d'un développement de tolérance par rapport à des médicaments et/ou à des drogues, notamment des médicaments à base d'opiacés ; pour la régulation de l'alimentation ; pour la modulation de l'activité physique, pour la régulation du système cardiovasculaire ; pour l'anesthésie locale ; pour l'augmentation de la vigilance ; pour l'augmentation de la libido ; pour la diurèse et/ou pour l'anti-natriurèse.

6. Composé, choisi dans le groupe constitué de :
[1] acide thiophène-2-carboxylique-benzo[d]isoxazole-3-ylamide,
[3] acide naphtalène-2-carboxylique-benzo[d]isoxazole-3-ylamide,
[4] acide adamantane-2-carboxylique-benzo[d]isoxazole-3-ylamide,
[5] acide cyclohexanecarboxylique-benzo[d]isoxazole-3-ylamide,
[6] N-benzo[d]isoxazole-3-yl-2,2-diméthyl-propionamide,
[7] N-(4-méthoxy-benzo[d]isoxazole-3-yl)-2,2-diphényl-acétamide,
[8] acide cyclopropanecarboxylique-(5-bromo-benzo[d]isoxazole-3-yl)-amide,
[9] 3,5-dichloro-N-(6-fluoro-benzo[d]isoxazole-3-yl)-benzamide,
[10] N-(4-amino-benzo[d]isoxazole-3-yl)-4-nitrobenzamide,
[11] acide naphtalène-1-carboxylique-(4-amino-benzo[d]isoxazole-3-yl)-amide,
[12] acide benzo[b]thiophène-2-carboxylique-(4-fluoro-benzo[d]isoxazole-3-yl)-amide,
[13] N-benzo[d]isoxazole-3-yl-2-trifluorométhyl-benzamide,
[14] N-benzo[d]isoxazole-3-yl-3,4-dichloro-benzamide,
[15] N-benzo[d]isoxazole-3-yl-3,4-difluoro-benzamide,
[16] N-(4-diméthylamino-benzo[d]isoxazole-3-yl)-3-fluorobenzamide,
[17] acide thiophène-2-carboxylique-(6-fluoro-benzo[d]isoxazole-3-yl)-amide,
[18] N-(6-fluoro-benzo[d]isoxazole-3-yl)-2-méthyl-butyramide,
[19] N-(6-chloro-benzo[d]isoxazole-3-yl)-2-fluoro-3-trifluorométhyl-benzamide,
[20] N-(6-chloro-benzo[d]isoxazole-3-yl)-3-méthoxy-benzamide,
[21] N-(6-bromo-benzo[d]isoxazole-3-yl)-4-tert-butyl-benzamide,
[23] N-(6-bromo-benzo[d]isoxazole-3-yl)-2-trifluorométhyl-henzamide,
[24] acide adamantane-2-carboxylique-(6-bromo-benzo[d]isoxazole-3-yl)-amide,
[25] N-(6-bromo-benzo[d]isoxazole-3-yl)-2-méthyl-butyramide,
[26] N-(5-fluoro-benzo[d]isoxazole-3-yl)-2-trifluorométhyl-benzamide,
[27] 3,4-dichloro-N-(5-fluoro-benzo[d]isoxazole-3-yl)-benzamide,
[29] N-(5-bromo-benzo[d]isoxazole-3-yl)-2-fluoro-5-trifluorométhyl-benzamide,
[30] N-(5-bromo-benzo[d]isoxazole-3-yl)-2,4-difluoro-benzamide,
[31] N-(5-méthyl-benzo[d]isoxazole-3-yl)-2-trifluorométhyl-benzamide,
[32] 3-fluoro-N-(5-méthyl-benzo[d]isoxazole-3-yl)-benzamide,
[33] N-(4-méthoxy-benzo[d]isoxazole-3-yl)-3,5-bis-trifluorométhyl-benzamide,
[34] 3,5-difluoro-N-(4-méthoxy-benzo[d]isoxazole-3-yl)-benzamide,
[35] N-(4-diméthylamino-benzo[d]isoxazole-3-yl)-3,5-bis-trifluorométhyl-benzamide,
[36] 2-bromo-N-[4-(2,2,2-trifluoro-éthoxy)-benzo[d]isoxazole-3-yl]-benzamide,
[37] 3-chloro-N-[4-(2,2,2-trifluoro-éthoxy)-benzo[d]isoxazole-3-yl]-benzamide,
[38] 4-tert-butyl-N-[4-(2,2,2-trifluoro-éthoxy)-benzo[d]isoxazole-3-yl]-benzamide,
[39] 2-méthoxy-N-[4-(2,2,2-trifluoro-éthoxy)-benzo[d]isoxazole-3-yl]-benzamide,
[40] N-(6-chloro-benzo[d]isoxazole-3-yl)-4-iodo-benzamide,
[41] acide naphtalène-2-carboxylique-(6-chloro-benzo[d]isoxazole-3-yl)-amide,
[42] N-(6-chloro-benzo[d]isoxazole-3-yl)-3,4-difluoro-benzamide,
[43] N-(6-bromo-benzo[d]isoxazole-3-yl)-4-fluoro-2-trifluorométhyl-benzamide,
[44] 2,4-dichloro-N-(5-bromo-benzo[d]isoxazole-3-yl)-5-fluoro-benzamide,
[45] N-(5-bromo-benzo[d]isoxazole-3-yl)-3-fluoro-4-trifluorométhyl-benzamide,
[46] N-(6-bromo-benzo[d]isoxazole-3-yl)-3-fluoro-5-trifluorométhyl-benzamide,
[47] N-(6-bromo-benzo[d]isoxazole-3-yl)-2,3,4-trifluoro-benzamide,
[48] N-(6-bromo-benzo[d]isoxazole-3-yl)-4-propyl-benzamide,
[49] N-(6-bromo-benzo[d]isoxazole-3-yl)-3,4-difluoro-benzamide,
[50] acide furane-2-carboxylique-(5-bromo-benzo[d]isoxazole-3-yl)-amide,
[51] 4-fluoro-N-(7-fluoro-benzo[d]isoxazole-3-yl)-2-trifluorométhyl-benzamide,
[52] 2,4-dichloro-5-fluoro-N-(7-fluoro-benzo[d]isoxazole-3-yl)-benzamide,
[53] 3-fluoro-N-(7-fluoro-benzo[d]isoxazole-3-yl)-4-trifluorométhyl-benzamide,
[54] 2,3,4-trifluoro-N-(7-fluoro-benzo[d]isoxazole-3-yl)-benzamide,
[55] N-(7-fluoro-benzo[d]isoxazole-3-yl)-4-iodo-benzamide,
[56] 2-chloro-N-(7-fluoro-benzo[d]isoxazole-3-yl)-nicotinamide,
[57] acide naphtalène-2-carboxylique-(7-fluoro-benzo[d]isoxazole-3-yl)-amide,
[58] N-(7-fluoro-benzo[d]isoxazole-3-yl)-4-propyl-benzamide,
[59] 3,4-difluoro-N-(7-fluoro-benzo[d]isoxazole-3-yl)-benzamide,
[60] 2-fluoro-N-(7-fluoro-benzo[d]isoxazole-3-yl)-3-trifluorométhyl-benzamide,
[61] N-(7-fluoro-benzo[d]isoxazole-3-yl)-3-méthoxy-benzamide,
[62] N-(7-fluoro-benzo[d]isoxazole-3-yl)-2,2-diphényl-acétamide,
[63] acide furane-2-carboxylique-(7-fluoro-benzo[d]isoxazole-3-yl)-amide,
[64] 2,4-dichloro-N-(4-diméthylamino-benzo[d]isoxazole-3-yl)-5-fluoro-benzamide,
[65] N-(4-diméthylamino-benzo[d]isoxazole-3-yl)-2,3,4-trifluoro-benzamide,
[66] 2-chloro-N-(4-diméthylamino-benzo[d]isoxazole-3-yl)-nicotinamide,
[67] acide naphtaléne-2-carboxylique-(4-diméthylamino-benzo[d]isoxazole-3-yl)-amide,
[68] N-(4-diméthylamino-benzo[d]isoxazole-3-yl)-4-propyl-benzamide,
[69] N-(4-diméthylamino-benzo[d]isoxazole-3-yl)-3,4-difluoro-benzamide,
[70] N-(4-diméthylamino-benzo[d]isoxazole-3-yl)-2-fluoro-3-trifluorométhyl-benzamide,
[71] N-(4-diméthylamino-benzo[d]isoxazole-3-yl)-3-methoxy-benzamide,
[72] N-(4-diméthylamino-benzo[d]isoxazole-3-yl)-2,2-diméthyl-propionamide,
[73] acide cyclohexanacarboxylique-(4-diméthylamino-benzo[d]isozazole-3-yl)-amide,
[74] N-(4-diméthylamino-benzo[d]isoxazole-3-yl)-2,2-diphényl-acétamide,
[75] acide furane-2-carboxylique-(4-diméthylamino-benzo[d]isoxazole-3-yl)-amide,
[76] N-(4-diméthylamino-benzo[d]isoxazole-3-yl)-2,2,3,3,4,4,4-heptafluoro-butyramide,
[77] 4-fluoro-N-[4-(2,2,2-trifluoro-éthoxy)-benzo[d]isoxazole-3-yl]-2-trifluorométhyl-benzamide,
[78] 2,4-dichloro-5-fluoro-N-[4-(2,2,2-trifluoro-éthoxy)-benzo[d]isoxazole-3-yl]-benzamide,
[79] 3-fluoro-N-[4-(2,2,2-trifluoro-éthoxy)-benzo[d]isoxazole-3-yl]-4-trifluorométhyl-benzamide,
[80] 2,3,4-trifluoro-N-[4-(2,2,2-trifluoro-éthoxy)-benzo[d]isoxazole-3-yl]-benzamide,
[81] acide naphtaléne-2-carboxylique-[4-(2,2,2-trifluoro-éthoxy)-benzo[d]isoxazole-3-yl]-amide,
[82] 3,4-difluoro-N-[4-(2,2,2-trifluoro-éthoxy)-benzo[d]isoxazole-3-yl]-benzamide,
[83] 2-fluoro-N-[4-(2,2,2-trifluoro-éthoxy)-benzo[d]isoxazole-3-yl]-3-trifluorométhyl-benzamide, [85] acide 2-éthyl-hexane-benzo[d]isoxazole-3-ylamide,
[86] N-benzo[d]isoxazole-3-yl-2-méthyl-butyramide,
[88] N-benzo[d]isoxazole-3-yl-3-fluoro-4-trifluorométhyl-benzamide,
[89] N-benzo[d]isoxazole-3-yl-2,3,6-trifluoro-benzamide,
[90] N-benzo[d]isoxazole-3-yl-nicotinamide,
[91] acide 5-méthyl-isoxazol-3-carboxylique-benzo[d]isoxazole-3-ylamide,
[92] acide benzo[b]thiophène-3-carboxylique-benzo[d]isoxazole-3-ylamide,
[94] 4-tert-butyl-N-(7-fluoro-benzo[d]isoxazole-3-yl)-benzamide,
[96] 4-fluoro-N-(7-fluoro-benzo[d]isoxazole-3-yl)-benzamide,
[100] 3,5-difluoro-N-(6-fluoro-benzo[d]isoxazole-3-yl)-benzamide,
[101] acide naphtalène-1-carboxylique-(6-fluoro-benzo[d]isoxazole-3-yl)-amide,
[102] acide adamantane-1-carboxylique-(6-fluoro-benzo[d]isoxazole-3-yl)-amide,
[104] 4-tert-butyl-N-(5-fluoro-benzo[d]isoxazole-3-yl)-benzamide,
[105] 2,4-difluoro-N-(5-fluoro-benzo[d]isoxazole-3-yl)-benzamide,
[106] acide naphtalène-2-carboxylique-(5-fluoro-benzo[d]isoxazole-3-yl)-amide,
[107] N-(5-fluoro-benzo[d]isoxazole-3-yl)-4-propyl-benzamide,
[109] 4-tert-butyl-N-(6-chloro-benzo[d]isoxazole-3-yl)-benzamide,
[110] N-(6-chloro-benzo[d]isoxazole-3-yl)-4-fluoro-2-trifluorométhyl-benzamide,
[111] 2,4-dichloro-N-(6-chloro-benzo[d]isoxazole-3-yl)-5-fluoro-benzamide,
[112] acide 2-éthyl-hexane-(6-chloro-benzo[d]isoxazole-3-yl)-amide,
[113] N-(6-chloro-benzo[d]isoxazole-3-yl)-2-méthyl-butyramide,
[115] N-(6-bromo-benzo[d]isoxazole-3-yl)-3,4-dichloro-benzamide,
[116] acide thiophène-2-carboxylique-(6-bromo-benzo[d]isoxazole-3-yl)-amide,
[117] N-(6-bromo-benzo[d]isoxazole-3-yl)-3,3-diméthyl-butyramide,
[119] 4-bromo-N-(5-méthyl-benzo[d]isoxazole-3-yl)-benzamide,
[121] 4-fluoro-N-(5-méthyl-benzo[d]isoxazole-3-yl)-benzamide,
[123] 3-méthyl-N-(5-méthyl-benzo[d]isoxazole-3-yl)-benzamide,
[124] 4-tert-butyl-N-(5-méthyl-benzo[d]isoxazole-3-yl)-benzamide,
[126] 2,3-difluoro-N-(5-méthyl-benzo[d]isoxazole-3-yl)-benzamide,
[127] 2,4-difluoro-N-(5-méthyl-benzo[d]isoxazole-3-yl)-benzamide,
[128] 2-chloro-N-(5-méthyl-benzo[d]isoxazole-3-yl)-nicotinamide,
[129] acide cyclopropanecarboxylique-(5-fluoro-benzo[d]isoxazole-3-yl)-amide,
[130] N,(5-fluoro-benzo[d]isoxazole-3-yl)-3,3-diméthyl-butyramide,
[131] 2,4-difluoro-N-(5-fluoro-benzo[d]isoxazole-3-yl)-benzamide,
[132] acide 2,5-diméthyl-furane-3-carboxylique-(5-fluoro-benza[d]isoxazole-3-yl)-amide,
[133] N-(5-bromo-benzo[d]isoxazole-3-yl)-2,3-difluoro-benzamide,
[134] acide 2,5-diméthyl-furane-3-carboxylique-(5-bromo-benzo[d]isoxazole-3-yl)-amide,
[135] N-(5-bromo-benzo[d]isoxazole-3-yl)-2-méthyl-butyramide,
[137] N-(5-bromo-benzo[d]isoxazole-3-yl)-3,4-diméthoxy-benzamide,
[138] N-(5-bromo-benzo[d]isoxazole-3-yl)-4-méthyl-benzamide,
[141] N-(5-bromo-benzo[d]isoxazole-3-yl)-5-fluoro-2-trifluorométhyl-benzamide,
[142] N-(5-bromo-benzo[d]isoxazole-3-yl)-3-méthoxy-benzamide,
[143] 3-méthyl-N-(5-méthyl-benzo[d]isoxazole-3-yl)-benzamide,
[144] 4-cyano-N-(5-méthyl-benza[d]isaxazole-3-yl)-benzamide,
[145] N-(5-méthyl-benzo[d]isxazole-3-yl)-2-phényl-buryramide,
[146] acide 2-méthylpentane-(5-méthyl-benzo[d]isoxazole-3-yl)-amide,
[147] 4-chloro-N-(4-fluoro-benzo[d]isoxazole-3-yl)-benzamide,
[150] acide adamantane-1-carboxylique-(4-fluoro-benzo[d]isoxazole-3-yl)-amide,
[151] acide adamantane-1-carboxylique-(4-chloro-benzo[d]isoxazole-3-yl)-amide,
[153] N-(4-chloro-benzo[d]isoxazole-3-yl)-3-méthyl-benzamide,
[154] N-(4-chloro-benzo[d]isoxazole-3-yl)-2-méthyl-butyramide,
[155] N-(4-chloro-benzo[d]isoxazole-3-yl)-3,3-diméthyl-butyramide,
[156] N-(4-méthoxy-benzo[d]isoxazole-3-yl)-2-méthyl-benzamide,
[157] 2-chloro-N-(4-méthoxy-benzo[d]isoxazole-3-yl) -benzamide,
[158] N-(4-métoxy-benzo[d]isoxazole-3-yl)-2-trifluorométhyl-benzamide,
[159] acide 2-éthylhexane-(4-métoxy-benzo[d]isoxazole-3-yl)-amide,
[160] N-(4-méthoxy-benzo[d]isoxazole-3-yl)-2-méthyl-butyramide,
[161] acide 2-méthylpentane-(4-métoxy-benzo[d]isoxazole-3-yl)-amide,
[162] acide cyclopropanecarboxylique-[4-(2,2,2-trifluoro-ethoxy)-benzo[d]isoxazole-3-yl]-amide,
[163] 2-méthyl-N-[4-(2,2,2-trifluoro-éthoxy)-benzo[d]isoxazole-3-yl]-butyramide,
[164] 3,3-diméthyl-N-[4-(2,2,2-trifluoro-éthoxy)-benzo[d]isoxazole-3-yl]-butyramide,
[165] acide cyclohexanecarboxylique-[4-(2,2,2-trifluoro-éthoxy)-benzo[d]isoxazole-3-yl]-amide,
[166] acide 2,5-diméthyl-furane-3-carboxylique-[4-(2,2,2-trifluoro-éthoxy)-benzo[d]isoxazole-3-yl]-amide,
[167] N-[4-(4-méthyl-benzyloxy)-benzo[d]isoxazole-3-yl]-3-nitro-benzamide,
[168] N-[4-(4-méthyl-benzyloxy)-benzo[d]isoxazole-3-yl]-4-propyl-bensamide,
[169] N-(4-diméthylamino-benzo[d]isoxazole-3-yl)-3-fluoro-5-trifluorométhyl-benzamide,
[170] 3,4-dichloro-N-(4-diméthylamino-benzo[d]isoxazole-3-yl)-benzamide,
[171] N-(4-diméthylamino-benzo[d]isoxazole-3-yl)-3-nitro-benzamide,
[172] acide adamantane-1-carboxylique-(4-diméthylamino-benzo[d]isoxazole-3-yl)-amide,
[173] acide benzo[b]thiophène-2-carboxylique-(4-diméthylamino-benzo[d]isoxazole-3-yl)-amide,
[174] N-(4-diméthylamino-benzo[d]isoxazole-3-yl)-3,3-diméthyl-butyramide,
[176] N-(5-amino-4,5,7-trifluoro-benzo[d]isoxazole-3-yl)-2,6-difluoro-benzamide
et
[177] N-(6-amino-4,5,7-trifluoro-benzo[d]isoxazole-3-yl)-2,4-difluoro-benzamide,
à chaque fois éventuellement sous la forme d'un de leurs stéréo-isomères purs, notamment énantiomères ou diastéréo-isomères ou rotamères, de leurs racémates ou sous la forme d'un mélange de stéréo-isomères, notamment des énantiomères et/ou diastéréo-isomères et/ou rotamères, dans des proportions quelconques dans le mélange, ou à chaque fois sous la forme de sels correspondants, ou à chaque fois sous la forme de solvates correspondants.

7. Procédé de fabrication d'un composé selon la revendication 6, **caractérisé en ce qu'**au moins un composé de la formule générale IIa, 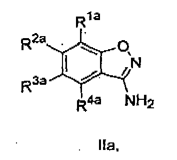 R^{1a} à R^{4a} ayant la signification telle que définie par les composés selon la revendication 6,
est transformé,
dans un milieu réactionnel éventuellement en présence d'au moins une base, avec au moins un composé de la formule générale R^{5a}-C (=O) -X, R^{5a} ayant la signification telle que définie par les composés selon la revendication 6 et X représentant un groupe de départ, de préférence un radical halogène, et encore de préférence un atome de chlore,
ou dans un milieu réactionnel, en présence d'au moins un réactif d'accouplement, le cas échéant en présence d'au moins une base, avec au moins un composé de la formule générale R^{5a}-C(=O)-OH, R^{5a} ayant la signification telle que définie par les composés selon la revendication 6,
en un composé selon la revendication 6 et celui-ci est le cas échéant isolé et/ou purifié.

8. Médicament contenant au moins un composé selon la revendication 6 ainsi que, éventuellement, un ou plusieurs adjuvants physiologiquement compatibles.

9. Médicament selon la revendication 8 pour la prophylaxie et/ou le traitement de la douleur, de préférence d'une douleur prise dans le groupe constitué de la douleur aiguë, de la douleur chronique, de la douleur viscérale et de la douleur neuropathique.

10. Médicament selon la revendication 8 pour la prophylaxie et/ou le traitement d'une ou plusieurs maladies prises dans le groupe constitué des troubles de l'alimentation, de préférence dans le groupe constitué de la boulimie, de l'anorexie, de l'obésité et de la cachexie ; de la migraine ; de l'hémicrânie paroxystique chronique ; des dépressions ; de l'incontinence urinaire ; de la toux ; de l'asthme ; du glaucome ; des acouphènes ; des inflammations ; des maladies neurodégénératives, de préférence dans le groupe constitué de la maladie de Parkinson, de la maladie de Huntington, de la maladie d'Alzheimer et de la sclérose en plaques ; des dysfonctionnements cognitifs, de préférence des troubles de la mémoire ; troubles de l'attention (syndrome du déficit de l'attention, TDA) ; épilepsie; narcolepsie ; diarrhée ; gastrite ; ulcère de l'estomac ; prurit ; anxiété ; panique ; schizophrénie ; ischémie cérébrale ; spasmes musculaires ; crampes ; syndrome de reflux gastro-oesophagien ; abus d'alcools et/ou de drogues, de préférence de nicotine ou de cocaïne, et/ou de médicaments ; dépendance à l'alcool et/ou à la drogue, de préférence à la nicotine ou à la cocaïne, et/ou à des médicaments, de préférence pour la prophylaxie et/ou la réduction de l'état de manque en cas de dépendance à l'alcool et/ou à la drogue, de préférence à la nicotine ou à la cocaïne, et/ou à des médicaments ; pour la prophylaxie et/ou la réduction d'un développement de tolérance par rapport à des médicaments et/ou à des drogues, notamment des médicaments à base d'opiacés ; pour la régulation de l'alimentation ; pour la modulation de l'activité physique, pour la régulation du système cardiovasculaire ; pour l'anesthésie locale ; pour l'augmentation de la vigilance ; pour l'augmentation de la libido ; pour la diurèse et/ou pour l'anti-natriurèse.

11. Utilisation d'au moins un composé selon la revendication 6 pour la fabrication d'un médicament pour la prophylaxie et/ou le traitement de la douleur, de préférence d'une douleur prise dans le groupe constitué de la douleur aiguë, de la douleur chronique, de la douleur viscérale et de la douleur neuropathique.

12. Utilisation d'au moins un composé selon la revendication 6 pour la fabrication d'un médicament pour la prophylaxie et/ou le traitement d'une ou plusieurs maladies prises dans le groupe constitué des troubles de l'alimentation, de préférence dans le groupe constitué de la boulimie, de l'anorexie, de l'obésité et de la cachexie ; de la migraine ; de l'hémicrânie paroxystique chronique ; des dépressions ; de l'incontinence urinaire ; de la toux ; de l'asthme ; du glaucome ; des acouphènes ; des inflammations ; des maladies neurodégénératives, de préférence dans le groupe constitué de la maladie de Parkinson, de la maladie de Huntington, de la maladie d'Alzheimer et de la sclérose en plaques ; des dysfonctionnements cognitifs, de préférence des troubles de la mémoire ; troubles de l'attention (syndrome du déficit de l'attention, TDA) ; épilepsie ; narcolepsie ; diarrhée; gastrite ; ulcère de l'estomac ; prurit ; anxiété ; panique ; schizophrénie ; ischémie cérébrale; spasmes musculaires ; crampes ; syndrome de reflux gastro-oesophagien ; abus d'alcools et/ou de drogues, de préférence de nicotine ou de cocaïne, et/ou de médicaments ; dépendance à l'alcool et/ou à la drogue, de préférence à la nicotine ou à la cocaïne, et/ou à des médicaments, de préférence pour la prophylaxie et/ou la réduction de l'état de manque en cas de dépendance à l'alcool et/ou à la drogue, de préférence à la nicotine ou à la cocaïne, et/ou à des médicaments ; pour la prophylaxie et/ou la réduction d'un développement de tolérance par rapport à des médicaments et/ou à des drogues, notamment des médicaments à base d'opiacés ; pour la régulation de l'alimentation ; pour la modulation de l'activité physique, pour la régulation du système cardiovasculaire ; pour l'anesthésie locale ; pour l'augmentation de la vigilance ; pour l'augmentation de la libido ; pour la diurèse et/ou pour l'anti-natriurèse.
